# EUROPEAN PATENT APPLICATION

(11) **EP 3 211 706 A1**
(43) Date of publication of application: **30.08.2017**
(21) Application number: 15852080.9
(22) Date of filing: 22.10.2015
(51) Int. Cl.: H01M 10/052, H01M 4/62, H01M 10/0567, H01M 10/058

(54) **LITHIUM SECONDARY BATTERY**

(30) Priority: 22.10.2014 JP 2014215007; 18.11.2014 JP 2014234052; 18.11.2014 JP 2014234053; 18.11.2014 JP 2014234054; 18.11.2014 JP 2014234055
(71) Applicant: Mitsui Chemicals, Inc., Minato-ku Tokyo 105-7122 (JP)
(72) Inventor: ZHANG, Han, Sodegaura-shi Chiba 299-0265 (JP); KOISO, Ayumi, Sodegaura-shi Chiba 299-0265 (JP); SHIGEMATSU, Akihito, Sodegaura-shi Chiba 299-0265 (JP); IIMURO, Yu, Sodegaura-shi Chiba 299-0265 (JP); NAGAKAWA, Keita, Sodegaura-shi Chiba 299-0265 (JP); CHIDA, Mitsuaki, Sodegaura-shi Chiba 299-0265 (JP); HAYASHI, Takaomi, Sodegaura-shi Chiba 299-0265 (JP); ONISHI, Hitoshi, Sodegaura-shi Chiba 299-0265 (JP); MIYASATO, Masataka, Sodegaura-shi Chiba 299-0265 (JP); FUJIYAMA, Satoko, Sodegaura-shi Chiba 299-0265 (JP)
(74) Representative: Hoffmann Eitle
(86) International application number: PCT/JP2015/079884
(87) International publication number: WO 2016/063964

(57) **Abstract**

A lithium secondary battery including: a positive electrode which contains a positive electrode active material capable of absorbing and desorbing lithium; a negative electrode which contains a negative electrode active material capable of absorbing and desorbing lithium; and a non-aqueous electrolytic solution, wherein at least one of the positive electrode or the negative electrode contains a polymer that is a reaction product of a compound (A) and a compound (B), and the non-aqueous electrolytic solution contains an additive (X). The additive (X) is at least one compound selected from the group consisting of: a carbonate compound having a carbon-carbon unsaturated bond, a carbonate compound having a halogen atom, an alkali metal salt, a sulfonic acid ester compound, a sulfuric acid ester compound, a nitrile compound, a dioxane compound, and a substituted aromatic hydrocarbon compound. The compound (A) is at least one compound selected from the group consisting of an amine compound, an amide compound, an imide compound, a maleimide compound and an imine compound. The compound (B) is a compound having two or more carbonyl groups in one molecule and being different from the compound (A).

## Description

### TECHNICAL FIELD

The present invention relates to a lithium secondary battery which can be charged and discharged and is utilized, for example, as a power source of a portable electronic device, as an on-vehicle battery, or for electric power storage.

### BACKGROUND ART

In recent years, lithium secondary batteries have been widely used as power sources for electronic devices such as cellular phones and laptop computers as well as for electric cars and electric power storage. Especially recently, there is a rapidly increasing demand for a high-capacity and high-power battery having a high energy density which can be mounted on hybrid vehicles and electric vehicles.

Such lithium secondary batteries are mainly constituted by a positive electrode which contains a material capable of absorbing and desorbing lithium, a negative electrode which contains a material capable of absorbing and desorbing lithium, and a non-aqueous electrolytic solution which contains a lithium salt and a non-aqueous solvent.

Examples of a positive electrode active material that can be used in the positive electrode include lithium metal oxides, such as LiCoO₂ LiMnO₂, LiNiO₂ and LiFePO₄.

As the non-aqueous electrolytic solution, a solution prepared by mixing a mixed solvent (non-aqueous solvent) of carbonates, such as ethylene carbonate, propylene carbonate, dimethyl carbonate and ethylmethyl carbonate, with a Li electrolyte such as LiPF₆, LiBF₄, LiN(SO₂CF₃)₂ or LiN(SO₂CF₂CF₃)₂, is used.

Meanwhile, as negative electrode active materials that can be used in the negative electrode, metal lithium, metal compounds capable of absorbing and desorbing lithium (e.g., elemental metals, oxides, and alloys with lithium) and carbon materials are known and, particularly, lithium secondary batteries in which a coke, artificial graphite or natural graphite capable of absorbing and desorbing lithium is employed have been put into practical use.

As an attempt to improve the performance of lithium secondary batteries, it has been proposed to incorporate a variety of additives in their non-aqueous electrolytic solutions.

For instance, non-aqueous electrolytic solutions containing a cyclic carbonate such as vinylene carbonate (VC), vinylethylene carbonate (VEC) or fluorinated ethylene carbonate (FEC) as an additive are known (see, for example, the below-described Patent Documents 1 and 2).

In addition, non-aqueous electrolytic solutions containing a lithium salt-type compound such as lithium difluorophosphate, lithium difluorooxalato borate or lithium difluorobis(oxalato)phosphate as an additive are known (see, for example, the below-described Patent Documents 3 and 4).

Moreover, non-aqueous electrolytic solutions containing a cyclic sulfate as an additive are also known (see, for example, the below-described Patent Document 5).

As batteries in which the reaction between a positive electrode and a non-aqueous electrolyte can be inhibited and which exhibit excellent cycle characteristics under a low-temperature environment, non-aqueous electrolyte secondary batteries which contain a chelate compound having a specific structure and a nitrile compound in a non-aqueous electrolytic solution are known (see, for example, the below-described Patent Document 6).

In addition, as non-aqueous electrolytic solutions for lithium secondary batteries which can improve the battery properties such as cycle characteristics, capacity and storage characteristics, those non-aqueous electrolytic solutions for lithium secondary batteries, in which an electrolyte is dissolved in a non-aqueous solvent and which further contain a nitrile compound and a S=O group-containing compound, are known (see, for example, the below-described Patent Document 7).

Moreover, as non-aqueous electrolytic solutions which can provide a lithium secondary battery comprising a current-breaking sealing body in a battery container without adversely affecting the battery properties such as low-temperature characteristics and storage characteristics while securing the battery safety, those non-aqueous electrolytic solutions which are mainly composed of a non-aqueous solvent dissolving a lithium salt as an electrolyte and contain an alkylbenzene derivative or cycloalkylbenzene derivative that has a tertiary carbon adjacent to a phenyl group are known (see, for example, the below-described Patent Document 8).

Meanwhile, the safety is a major problem in high-capacity and high-power lithium secondary batteries having a high energy density.

As a method for improving the safety, there has been disclosed a technology of coating a positive electrode active material with a nitrogen-containing polymer (see, for example, the below-described Patent Document 9). This nitrogen-containing polymer is considered to inhibit thermal runaway by undergoing a cross-linking reaction when the temperature of a lithium secondary battery is increased due to an abnormality in the lithium secondary battery.
Patent Document 1: Japanese Patent No. 3573521
Patent Document 2: Japanese Patent No. 4489207
Patent Document 3: Japanese Patent No. 3439085
Patent Document 4: Japanese Patent No. 3722685
Patent Document 5: Japanese Patent No. 3978881
Patent Document 6: Japanese Patent No. 5289091
Patent Document 7: Japanese Patent Application Laid-Open (JP-A) No. 2004-179146
Patent Document 8: Japanese Patent No. 3113652
Patent Document 9: JP-A No. 2010-157512

### SUMMARY OF INVENTION

### Technical Problem

Nevertheless, there is a demand for a lithium secondary battery in which the discharge capacity retention ratio after repeated charging and discharging is improved and an increase in the battery resistance is suppressed.

Therefore, an object of one embodiment of the invention is to provide a lithium secondary battery in which the discharge capacity retention ratio after repeated charging and discharging is improved and an increase in the battery resistance is suppressed.

### Solution to Problem

Concrete means for solving the above-described problems are as follows.
<1> A lithium secondary battery comprising:
   a positive electrode which contains a positive electrode active material capable of absorbing and desorbing lithium;
   a negative electrode which contains a negative electrode active material capable of absorbing and desorbing lithium; and
   a non-aqueous electrolytic solution,
   wherein:
      at least one of the positive electrode or the negative electrode contains a polymer that is a reaction product of at least one compound (A) and a compound (B), the at least one compound (A) being selected from the group consisting of an amine compound, an amide compound, an imide compound, a maleimide compound and an imine compound, and the compound (B) having two or more carbonyl groups in one molecule and being different from the compound (A), and
      the non-aqueous electrolytic solution contains an additive (X), which is at least one compound selected from the group consisting of:
         a carbonate compound having a carbon-carbon unsaturated bond,
         a carbonate compound having a halogen atom and not having a carbon-carbon unsaturated bond,
         an alkali metal salt,
         a sulfonic acid ester compound,
         a sulfuric acid ester compound,
         a nitrile compound,
         a dioxane compound, and
         an aromatic hydrocarbon compound substituted with at least one substituent selected from the group consisting of a halogen atom, an alkyl group, a halogenated alkyl group, an alkoxy group, a halogenated alkoxy group, an aryl group and a halogenated aryl group.
<2> The lithium secondary battery according to <1>, wherein the polymer is a reaction product of the maleimide compound and the compound (B).
<3> The lithium secondary battery according to <1> or <2>, wherein the compound (B) is at least one compound selected from the group consisting of barbituric acid and derivatives thereof.
<4> The lithium secondary battery according to any one of <1> to <3>, wherein the polymer comprises a reactive double bond.
<5> The lithium secondary battery according to any one of <1> to <4>, wherein the maleimide compound is at least one compound selected from the group consisting of compounds each represented by any one of Formulae (1) to (4): 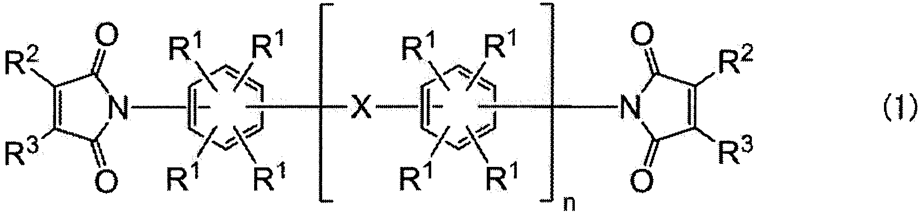 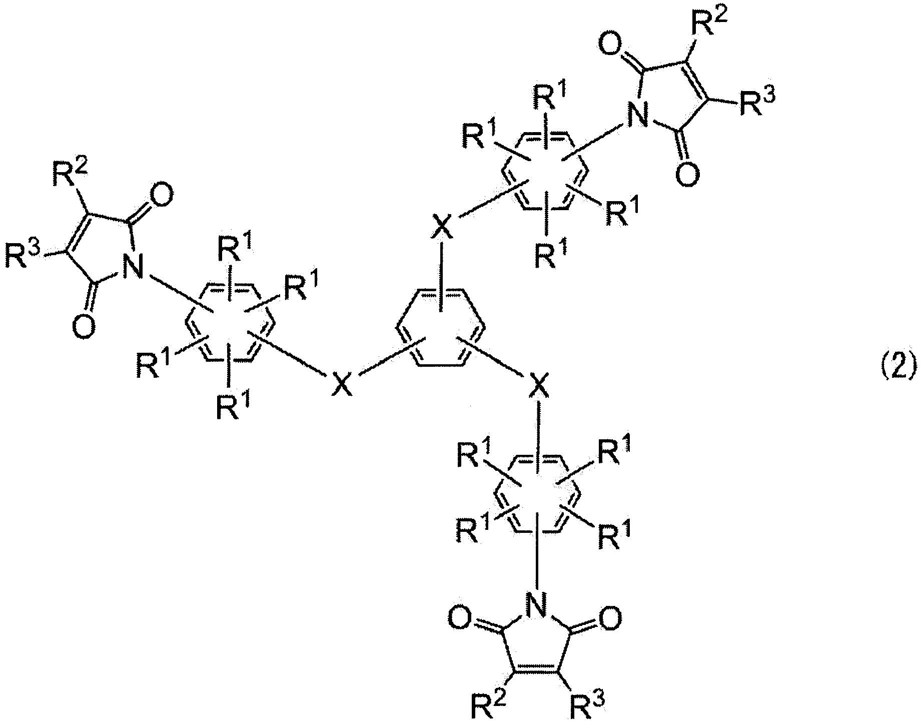 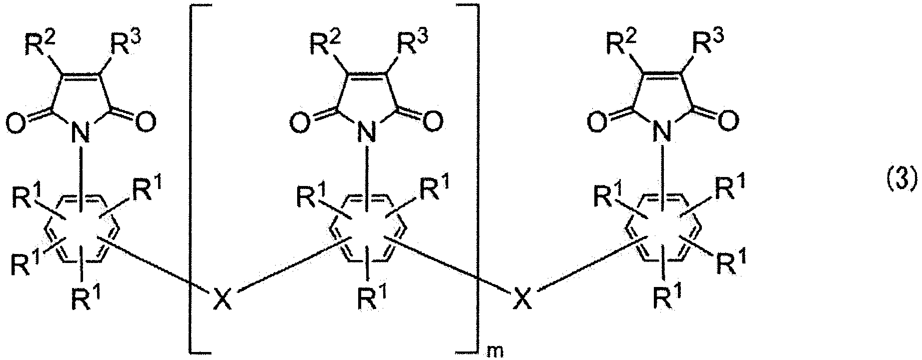 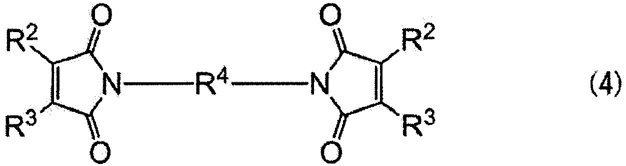 wherein, in Formula (1), n is an integer of 0 or larger;
   in Formula (3), m represents a real number from 1 to 1,000;
   in Formulae (1) to (3), X represents -O-, -SO₂- -S-, -CO-, -CH₂-, -C(CH₃)₂-, -C(CF₃)₂-, -CR=CR-, or a single bond, wherein R is a hydrogen atom or an alkyl group, and when there are plural Xs in one molecule, the plural Xs may be the same as, or different from, each other;
   in Formulae (1) to (3), R¹ represents a hydrogen atom, a halogen atom or a hydrocarbon group, plural R¹s existing in one molecule may be the same as, or different from, each other, and each of R² and R³ independently represents a hydrogen atom, a halogen atom, or an alkyl group having from 1 to 3 carbon atoms; and
   in Formula (4), R⁴ represents an alkylene group having from 1 to 10 carbon atoms which optionally has a side chain, -NR³-, -C(O)CH₂-, -CH₂OCH₂- -C(O)-, -O-, -O-O-, -S-, -S-S-, -S(O)-, -CH₂S(O)CH₂- or -SO₂-, and each of R² and R³ independently represents a hydrogen atom, a halogen atom, or an alkyl group having from 1 to 3 carbon atoms.
<6> The lithium secondary battery according to <3>, wherein the at least one compound selected from the group consisting of barbituric acid and derivatives thereof is a compound represented by Formula (5): 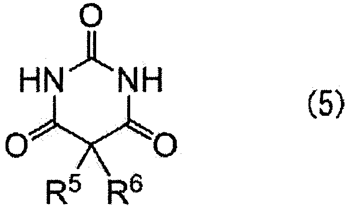 wherein each of R⁵ and R⁶ independently represents a hydrogen atom, a methyl group, an ethyl group, a phenyl group, an isopropyl group, an isobutyl group, an isopentyl group, or a 2-pentyl group.
<7> The lithium secondary battery according to any one of <1> to <6>, wherein at least one of the positive electrode or the negative electrode comprises a composite layer containing the polymer, and a content of the polymer in the composite layer is from 0.01% by mass to 5% by mass.
<8> The lithium secondary battery according to any one of <1> to <7>, wherein the carbonate compound having a carbon-carbon unsaturated bond is at least one selected from the group consisting of chain carbonate compounds each represented by Formula (X1), cyclic carbonate compounds each represented by Formula (X2), cyclic carbonate compounds each represented by Formula (X3) and cyclic carbonate compounds each represented by Formula (X4): 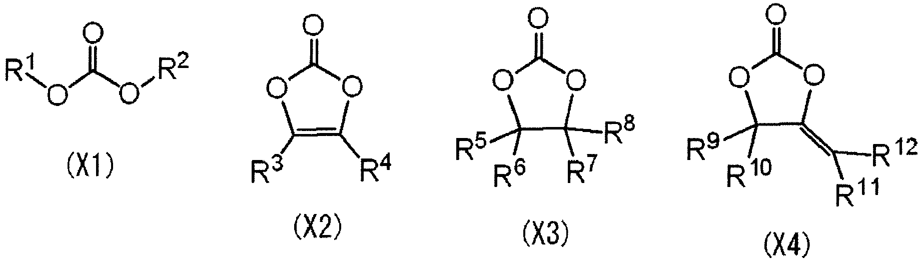 wherein, in Formula (X1), each of R¹ and R² independently represents a group having from 1 to 12 carbon atoms which optionally has a carbon-carbon unsaturated bond, an ether bond or a carbon-halogen bond, and at least one of R¹ or R² has a carbon-carbon unsaturated bond;
   in Formula (X2), each of R³ and R⁴ independently represents a hydrogen atom, or a group having from 1 to 12 carbon atoms which optionally has a carbon-carbon unsaturated bond, an ether bond or a carbon-halogen bond;
   in Formula (X3), each of R⁵ to R⁸ independently represents a hydrogen atom, or a group having from 1 to 12 carbon atoms which optionally has a carbon-carbon unsaturated bond, an ether bond or a carbon-halogen bond, at least one of R⁵ to R⁸ has a carbon-carbon unsaturated bond, and either R⁵ or R⁶, and either R⁷ or R⁸, are optionally combined to form, in combination with carbon atoms to which they are respectively bonded, a benzene ring structure or a cyclohexyl ring structure; and
   in Formula (X4), each of R⁹ to R¹² independently represents a hydrogen atom, or a group having from 1 to 12 carbon atoms which optionally has a carbon-carbon unsaturated bond, an ether bond or a carbon-halogen bond.
<9> The lithium secondary battery according to any one of <1> to <8>, wherein the alkali metal salt is at least one selected from the group consisting of a monofluorophosphate salt, a difluorophosphate salt, an oxalato salt, a sulfonate salt, a carboxylate salt, an imide salt and a methide salt.
<10> The lithium secondary battery according to <9>, wherein the alkali metal salt is at least one selected from the group consisting of a monofluorophosphate salt, a difluorophosphate salt, an oxalato salt and a fluorosulfonate salt.
<11> The lithium secondary battery according to any one of <1> to <10>, wherein the sulfonic acid ester compound is at least one compound selected from the group consisting of chain sulfonic acid ester compounds each represented by Formula (X6), cyclic sulfonic acid ester compounds each represented by Formula (X7), cyclic sulfonic acid ester compounds each represented by Formula (X8) and disulfonic acid ester compounds each represented by Formula (X9): 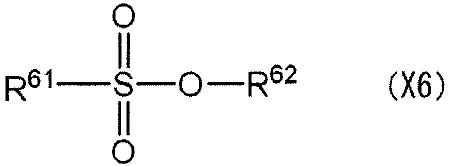 wherein each of R⁶¹ and R⁶² independently represents a linear or branched aliphatic hydrocarbon group having from 1 to 12 carbon atoms, an aryl group having from 6 to 12 carbon atoms, or a heterocyclic group having from 6 to 12 carbon atoms, and each of the groups is optionally substituted with a halogen atom; 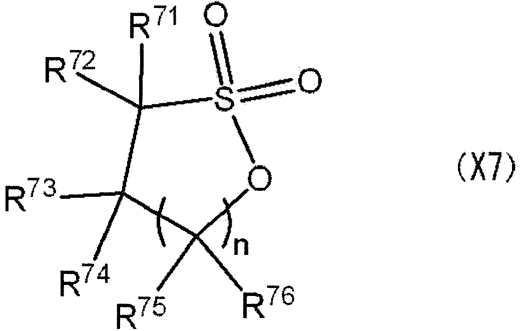 wherein each of R⁷¹ to R⁷⁶ independently represents a hydrogen atom, a halogen atom, or an alkyl group having from 1 to 6 carbon atoms; and n is an integer from 0 to 3; 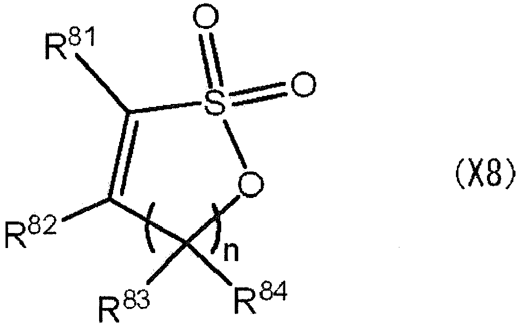 wherein each of R⁸¹ to R⁸⁴ independently represents a hydrogen atom, a halogen atom, or an alkyl group having from 1 to 6 carbon atoms; and n is an integer from 0 to 3; 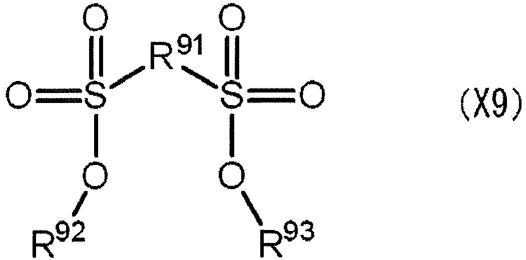
   wherein R⁹¹ represents an aliphatic hydrocarbon group having from 1 to 10 carbon atoms, or a halogenated alkylene group having from 1 to 3 carbon atoms; and
   R⁹² and R⁹³ each independently represent an alkyl group having from 1 to 6 carbon atoms or an aryl group, or
   R⁹² and R⁹³ are combined to represent an alkylene group having from 1 to 10 carbon atoms, or a 1,2-phenylene group which is optionally substituted with a halogen atom, an alkyl group having from 1 to 12 carbon atoms or a cyano group.
<12> The lithium secondary battery according to any one of <1> to <11>, wherein the sulfuric acid ester compound is at least one compound selected from the group consisting of chain sulfuric acid ester compounds each represented by Formula (X10) and cyclic sulfuric acid ester compounds each represented by Formula (X11): 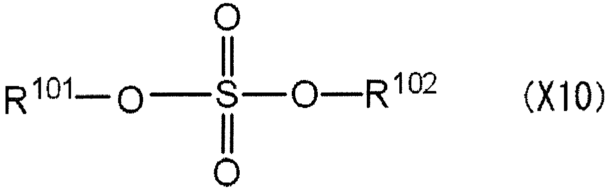 wherein each of R¹⁰¹ and R¹⁰² independently represents a linear or branched aliphatic hydrocarbon group having from 1 to 12 carbon atoms, an aryl group having from 6 to 12 carbon atoms, or a heterocyclic group having from 6 to 12 carbon atoms, and each of the groups is optionally substituted with a halogen atom; 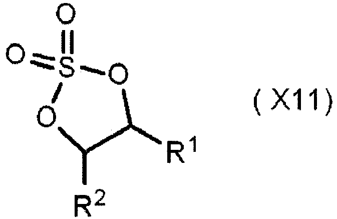 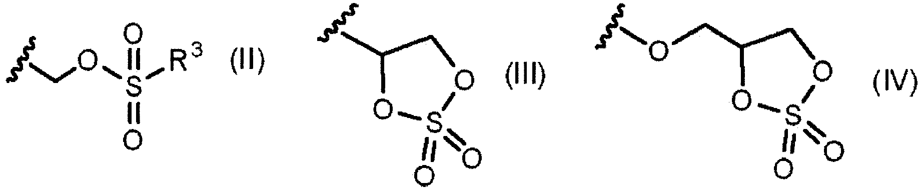 wherein, in Formula (X11), each of R¹ and R² independently represents a hydrogen atom, an alkyl group having from 1 to 6 carbon atoms, a phenyl group, a group represented by Formula (II) or a group represented by Formula (III), or R¹ and R² are combined to represent, in combination with carbon atoms to which R¹ and R² are respectively bonded, a group forming a benzene ring or a cyclohexyl ring;
   in Formula (II), R³ represents a halogen atom, an alkyl group having from 1 to 6 carbon atoms, a halogenated alkyl group having from 1 to 6 carbon atoms, an alkoxy group having from 1 to 6 carbon atoms, or a group represented by Formula (IV), and wavy lines in Formulae (II), (III) and (IV) each represent a bonding position; and
   when the cyclic sulfuric acid ester compound represented by Formula (X11) contains two groups each represented by Formula (II), the two groups each represented by Formula (II) may be the same as, or different from, each other.
<13> The lithium secondary battery according to any one of <1> to <12>, wherein the nitrile compound is a nitrile compound represented by Formula (X12): 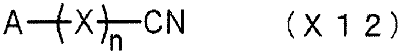 wherein, in Formula (X12):
   A represents a hydrogen atom or a nitrile group;
   X represents -CH₂-, -CFH-, -CF₂-, -CHR¹¹-, -CFR¹²-, -CR¹³R¹⁴- -C(=O)-, -O-, -S-, -NH-, or -NR¹⁵-;
   each of R¹¹ to R¹⁵ independently represents a nitrile group or a hydrocarbon group having from 1 to 5 carbon atoms, which optionally has a substituent;
   n represents an integer greater than or equal to 1; and
   when n is an integer greater than or equal to 2, plural Xs may be the same as, or different from, each other.
<14> The lithium secondary battery according to any one of <1> to <13>, wherein the aromatic hydrocarbon compound is an aromatic hydrocarbon compound which is substituted with at least one substituent selected from the group consisting of a fluorine atom, a chlorine atom, an alkyl group having from 1 to 6 carbon atoms, a halogenated alkyl group having from 1 to 6 carbon atoms, an alkoxy group having from 1 to 6 carbon atoms, a halogenated alkoxy group having from 1 to 6 carbon atoms, an aryl group having from 6 to 12 carbon atoms and a halogenated aryl group having from 6 to 12 carbon atoms.
<15> The lithium secondary battery according to any one of <1> to <14>, wherein a ratio of a battery resistance R1 at 150°C with respect to a battery resistance R0 at 30°C (R1/R0) is 3.8 or higher.
<16> A lithium secondary battery comprising:
   a positive electrode which contains a positive electrode active material capable of absorbing and desorbing lithium;
   a negative electrode which contains a negative electrode active material capable of absorbing and desorbing lithium; and
   a non-aqueous electrolytic solution,
   wherein
   a ratio of a battery resistance R1 at 150°C with respect to a battery resistance R0 at 30°C (R1/R0) is 3.8 or higher, and
   the non-aqueous electrolytic solution contains an additive (X) which is at least one compound selected from the group consisting of:
      a carbonate compound having a carbon-carbon unsaturated bond;
      a carbonate compound having a halogen atom and not having a carbon-carbon unsaturated bond;
      an alkali metal salt;
      a sulfonic acid ester compound;
      a sulfuric acid ester compound;
      a nitrile compound;
      a dioxane compound; and
      an aromatic hydrocarbon compound substituted with at least one substituent selected from the group consisting of a halogen atom, an alkyl group, a halogenated alkyl group, an alkoxy group, a halogenated alkoxy group, an aryl group and a halogenated aryl group.
<17> A lithium secondary battery obtained by charging and discharging the lithium secondary battery according to any one of claims 1 to 16.

### Advantageous Effects of Invention

According to one embodiment of the invention, there is provided a lithium secondary battery in which the discharge capacity retention ratio after repeated charging and discharging is improved and an increase in the battery resistance is suppressed.

### BRIEF DESCRIPTION OF DRAWINGS

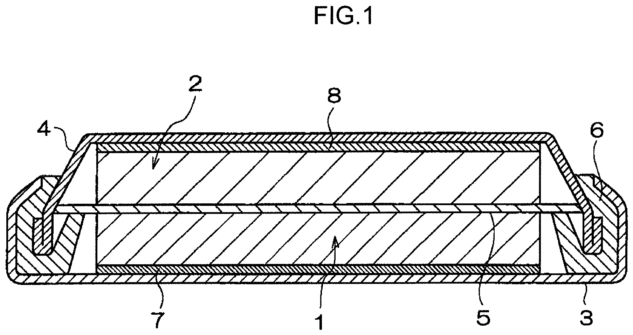

FIG. 1 is a schematic cross-sectional view of a coin-type battery, which shows one example of the lithium secondary battery according to one embodiment of the invention.

### DESCRIPTION OF EMBODIMENTS

The first to the ninth embodiments of the invention will now be described.

At least two of the first to the ninth embodiments may share a conceptually redundant part.

### [First Embodiment]

The lithium secondary battery according to the first embodiment is a lithium secondary battery comprising:
a positive electrode which contains a positive electrode active material capable of absorbing and desorbing lithium;
a negative electrode which contains a negative electrode active material capable of absorbing and desorbing lithium; and
a non-aqueous electrolytic solution,
wherein:
   at least one of the positive electrode or the negative electrode contains a polymer that is a reaction product of at least one compound (A) and a compound (B) (hereinafter, also referred to as "specific polymer"), the at least one compound (A) being selected from the group consisting of an amine compound, an amide compound, an imide compound, a maleimide compound and an imine compound, and the compound (B) having two or more carbonyl groups in one molecule and being different from the compound (A), and
   the non-aqueous electrolytic solution contains an additive (X).

The additive (X) is at least one compound selected from the group consisting of:
a carbonate compound having a carbon-carbon unsaturated bond,
a carbonate compound having a halogen atom and not having a carbon-carbon unsaturated bond,
an alkali metal salt,
a sulfonic acid ester compound,
a sulfuric acid ester compound,
a nitrile compound,
a dioxane compound, and
an aromatic hydrocarbon compound substituted with at least one substituent selected from the group consisting of a halogen atom, an alkyl group, a halogenated alkyl group, an alkoxy group, a halogenated alkoxy group, an aryl group and a halogenated aryl group.

According to the first embodiment, the discharge capacity retention ratio after repeated charging and discharging is improved by a combination of the feature that at least one of the positive electrode or the negative electrode contains the specific polymer and the feature that the non-aqueous electrolytic solution contains the additive (X).

Further, according to the first embodiment, an increase in the battery resistance (particularly, an increase in the battery resistance caused by repeated charging and discharging) is also suppressed by the combination.

In the present specification, the concept of "repeated charging and discharging" also encompasses the concept of "trickle charging" described in Examples below.

The term "trickle charging" used herein refers to continuous charging of a lithium secondary battery with a microcurrent for compensation of self-discharge of the lithium secondary battery.

The reasons why the above-described combination improves the discharge capacity retention ratio after repeated charging and discharging and suppresses an increase in the battery resistance are not necessarily clear; however, they are speculated as follows.

That is, in the early stage of charging and discharging, the additive (X) undergoes a reaction on the surface of at least one of the positive electrode active material or the negative electrode active material (hereinafter, also referred to as "active material") to form a protective film covering the surface of the active material. This protective film inhibits deterioration of the active material caused by repeated charging and discharging, thereby contributing to maintenance of a favorable capacity retention ratio and inhibition of an increase in the electrical resistance. However, with the additive (X) alone, since the surface of the active material has a portion that is not covered with the protective film (for example, the boundary region of the protective film), deterioration of the active material may progress in this portion.

Meanwhile, by repeatedly performing charging and discharging, the specific polymer contained in at least one of the positive electrode or the negative electrode gradually reacts with the active material, although the extent thereof is very limited.

In the first embodiment where a combination of the additive (X) and the specific polymer is incorporated, first, in the early stage of charging and discharging, the additive (X) forms a protective film covering the surface of the active material. Then, during repeated charging and discharging, the reaction between the specific polymer and the active material proceeds, whereby the portion of the active material surface that is not covered by the protective film (for example, the boundary region of the protective film) is reinforced.

As described above, in the first embodiment, it is believed that the additive (X) and the specific polymer work together to improve the discharge capacity retention ratio after repeated charging and discharging and to suppress an increase in the battery resistance.

The reaction between the specific polymer and the active material is believed to proceed more readily when the specific polymer comprises a reactive double bond.

The preferred scope of the specific polymer will be described later.

In the lithium secondary battery according to the first embodiment, it is preferred that the ratio of the battery resistance R1 at 150°C with respect to the battery resistance R0 at 30°C (R1/R0) is 3.8 or higher.

When the ratio (R1/R0) is 3.8 or higher, the discharge capacity retention ratio after repeated charging and discharging is more effectively improved, and an increase in the battery resistance (particularly, an increase in the battery resistance caused by repeated charging and discharging) is further suppressed.

The reason for this is not necessarily clear; however, it is speculated as follows.

The ratio (R1/R0) of 3.8 or higher, that is, an increase in the resistance of a lithium secondary battery associated with an increase in the temperature of the lithium secondary battery, means that the reaction between the specific polymer and the active material effectively proceeds during repeated charging and discharging.

Therefore, it is believed that, when the ratio (R1/R0) is 3.8 or higher, the above-described effects of the combination of the additive (X) and the specific polymer are more effectively exerted, as a result of which the discharge capacity retention ratio after repeated charging and discharging is more effectively improved and an increase in the battery resistance (particularly, an increase in the battery resistance caused by repeated charging and discharging) is further suppressed.

The ratio (R1/R0) is 3.8 or higher, and it is preferably 3.9 or higher, more preferably 4.0 or higher.

The upper limit of the ratio (R1/R0) is not particularly restricted; however, it is preferably 1,000, more preferably 100.

The positive electrode and the negative electrode in the lithium secondary battery according to the first embodiment (these electrodes may be hereinafter collectively referred to as "electrodes for lithium secondary battery") will now be described, followed by description of the non-aqueous electrolytic solution.

### <<Electrodes for Lithium Secondary Battery (Positive Electrode and Negative Electrode)>>

The lithium secondary battery according to the first embodiment comprises electrodes for lithium secondary battery (a positive electrode and a negative electrode).

### <Positive Electrode Active Material>

The positive electrode contains a positive electrode active material capable of absorbing and desorbing lithium.

The positive electrode active material is not particularly restricted as long as it is a material capable of absorbing and desorbing lithium, and any positive electrode active material that is usually used in a lithium ion secondary battery can be used.

Specific examples of the positive electrode active material include lithium-manganese composite oxides (e.g., LiMn₂O₄), lithium-nickel composite oxides (e.g., LiNiO₂), lithium-cobalt composite oxides (e.g., LiCoO₂), lithium-iron composite oxides (e.g., LiFeO₂), lithium-nickel-manganese composite oxides (e.g., LiNi_{0.5}Mn_{0.5}O₂), lithium-nickel-cobalt composite oxides (e.g., LiNi_{0.8}Co_{0.2}O₂), lithium-nickel-cobalt-manganese composite oxides, lithium-transition metal phosphate compounds (e.g., LiFePO₄), lithium-transition metal sulfate compounds (e.g., LiₓFe₂(SO₄)₃), solid solution compounds (Li₂MO₃-LiMO₂ (wherein, M represents Ni, Co, or Mn)), vanadium oxide compounds, silicate compounds, and sulfur compounds.

The positive electrode may contain only one positive electrode active material, or a combination of two or more positive electrode active materials.

When the positive electrode comprises a positive electrode active material-containing composite layer, the content ratio of the positive electrode active material in the composite layer is, for example, not less than 10% by mass, preferably not less than 30% by mass, more preferably not less than 50% by mass, with respect to the total amount of the composite layer. On another front, the content ratio of the positive electrode active material is, for example, 99.9% by mass or less, preferably 99% by mass or less, with respect to the total amount of the composite layer.

The composite layer will be described later.

### <Negative Electrode Active Material>

The negative electrode contains a negative electrode active material capable of absorbing and desorbing lithium.

As the negative electrode active material, at least one selected from the group consisting of metal lithium, lithium-containing alloys, metals and alloys that can be alloyed with lithium, oxides capable of doping and dedoping lithium ions, transition metal nitrides capable of doping and dedoping lithium ions, and carbon materials capable of doping and dedoping lithium ions (these materials may be used singly, or in combination of two or more thereof as a mixture) can be used.

Examples of the metals and alloys that can be alloyed with lithium (or lithium ion) include silicon, silicon alloys, tin, tin alloys, and lithium titanate.

The negative electrode active material is preferably a carbon material capable of doping and dedoping lithium ions.

Examples of such a carbon material include carbon black, activated charcoal, graphite materials (artificial graphites, natural graphites), and an amorphous carbon materials.

The form of the carbon material may be any of a fibrous form, a spherical form, a potato form and a flake form.

Specific examples of the amorphous carbon materials include hard carbon, cokes, mesocarbon microbeads (MCMB) calcined at 1,500°C or lower, and mesophase pitch carbon fibers (MCF).

Examples of the graphite materials include natural graphites and artificial graphites. As artificial graphite, graphitized MCMB, graphitized MCF and the like can be used.

As the graphite materials, those which contain boron can also be used. Further, as the graphite materials, graphite materials coated with a metal such as gold, platinum, silver, copper or tin; graphite materials coated with amorphous carbon; and mixtures of amorphous carbon and graphite can be used as well.

These carbon materials may be used singly, or in combination of two or more thereof.

The above-described carbon material is particularly preferably a carbon material whose interplanar spacing d(002) of the (002) plane, which is measured by an X-ray analysis, is 0.340 nm or smaller.

As the carbon material, a graphite having a true density of not less than 1.70 g/cm³ or a highly crystalline carbon material having a property comparable thereto is also preferred.

By using such a carbon material as described above, the energy density of the battery can be further increased.

### <Specific Polymer>

In the first embodiment, at least one of the positive electrode or the negative electrode contains a polymer ("specific polymer") that is a reaction product of at least one compound (A), which is selected from the group consisting of an amine compound, an amide compound, an imide compound, a maleimide compound and an imine compound, and a compound (B) which has two or more carbonyl groups in one molecule and is different from the compound (A).

The specific polymer may be contained only in the positive electrode or the negative electrode, or in both of the positive electrode and the negative electrode.

It is preferred that the specific polymer is contained at least in the positive electrode.

In the production of the specific polymer, the compounds (A) and (B) may each be used singly, or in combination of two or more thereof.

As the compound (A), at least one compound selected from maleimide compounds is preferred.

The compound (A) is preferably at least one compound selected from the group consisting of compounds each represented by any one of Formulae (1) to (4).

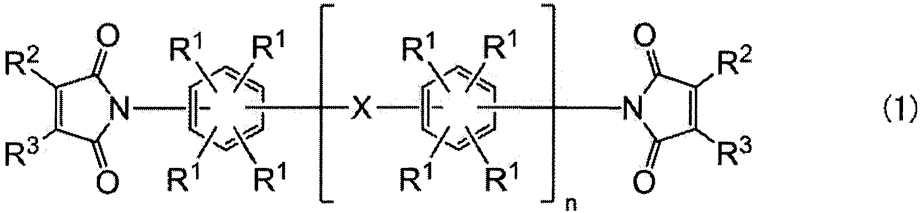

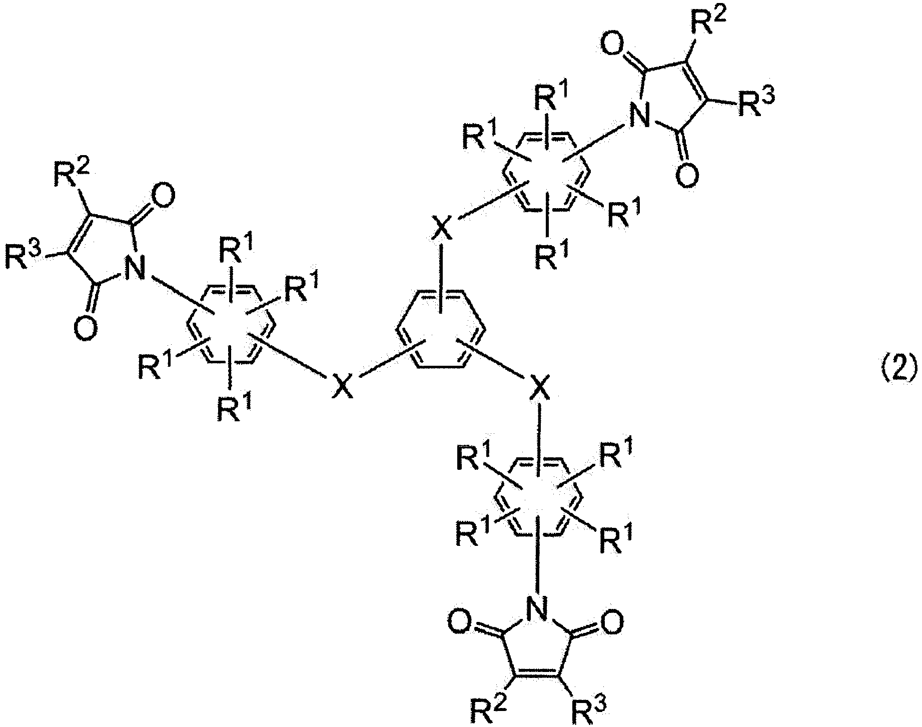

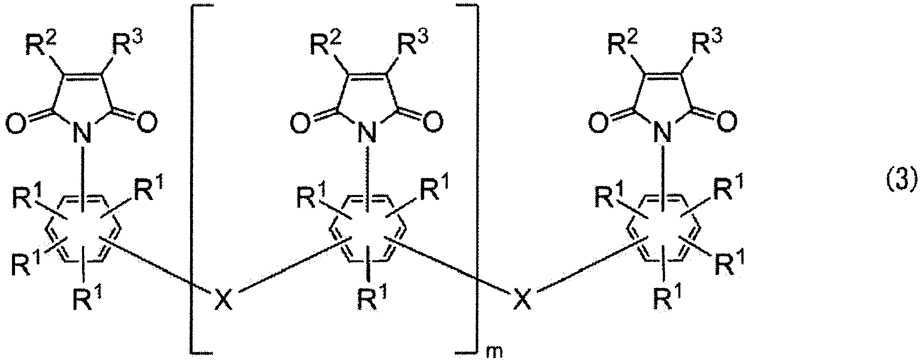

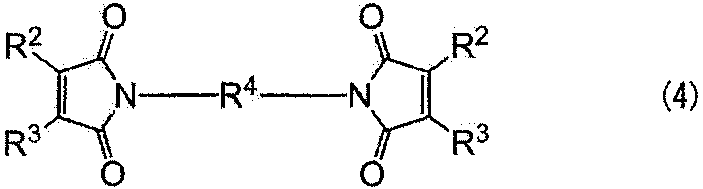

In Formula (1), n is an integer of 0 or larger. In Formula (1), n is preferably from 1 to 10.

In Formula (3), m represents a real number from 1 to 1,000.

When the compound represented by Formula (3) is used as a maleimide compound, a plurality of compounds having different ms in Formula (3) may be used.

In Formulae (1) to (3), X represents -O-, -SO₂- -S-, -CO-, -CH₂-, -C(CH₃)₂-, -C(CF₃)₂-, -CR=CR- (wherein, R is a hydrogen atom or an alkyl group), or a single bond. In Formulae (1) to (3), when there are plural Xs in one molecule, the plural Xs may be the same or different from each other.

In Formulae (1) to (3), R¹ represents a hydrogen atom, a halogen atom, or a hydrocarbon group. In Formulae (1) to (3), plural R¹s existing in one molecule may be the same or different from each other.

In Formulae (1) to (3), R² and R³ each independently represent a hydrogen atom, a halogen atom, or an alkyl group having from 1 to 3 carbon atoms.

In Formula (4), R⁴ represents an alkylene group having from 1 to 10 carbon atoms which optionally has a side chain, -NR³-, -C(O)CH₂-, -CH₂OCH₂-, -C(O)-, -O-, -O-O-, -S-, -S-S-, -S(O)-, -CH₂S(O)CH₂-, or -SO₂-.

In Formula (4), R² and R³ each independently represent a hydrogen atom, a halogen atom, or an alkyl group having from 1 to 3 carbon atoms.

As the compound represented by Formula (3), a compound wherein m is from 1 to 100, R¹ and R² are hydrogen atoms and X is -CH₂- is preferred.

The maleimide compound is particularly preferably at least one bismaleimide compound selected from the following specific examples.

That is, specific examples of a particularly preferred bismaleimide compound include:
1,1'-(methylenedi-4,1-phenylene)bismaleimide,
*N*,*N*'-(1,1'-biphenyl-4,4'-diyl)bismaleimide,
*N*,*N*'-(4-methyl-1,3-phenylene)bismaleimide,
1,1'-(3,3'-dimethyl-1,1'-biphenyl-4,4'-diyl)bismaleimide,
*N*,*N*'-ethylenedimaleimide,
*N*,*N*'-(1,2-phenylene)dimaleimide,
*N*,*N*'-(1,3-phenylene)dimaleimide,
*N,N*'*-*ketone dimaleimide,
*N,N*'-methylenebismaleimide,
bismaleimide methyl ether,
1,2-bis-(maleimide)-1,2-ethanediol,
*N*,*N*'-4,4'-diphenyl ether bismaleimide, and
4,4'-bis(maleimide)-diphenyl sulfone.

The compound (B) is preferably at least one compound selected from the group consisting of barbituric acid and derivatives thereof.

As barbituric acid and derivatives thereof, compounds represented by Formula (5) are more preferred.

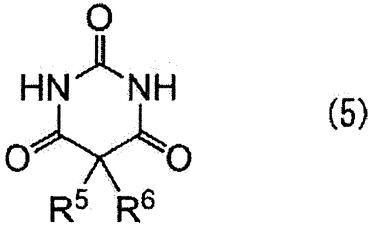

In Formula (5), R⁵ and R⁶ each independently represent a hydrogen atom, a methyl group, an ethyl group, a phenyl group, an isopropyl group, an isobutyl group, an isopentyl group, or a 2-pentyl group.

It is preferred that the specific polymer is a reaction product of a maleimide compound and the compound (B).

It is also preferred that the specific polymer contains a nitrogen atom (that is, a nitrogen-containing polymer).

Further, as described above, it is preferred that the specific polymer has a reactive double bond. Since this allows the reaction between the specific polymer and the active material to proceed more readily, not only the discharge capacity retention ratio after repeated charging and discharging is further improved but also an increase in the battery resistance is further suppressed.

It is more preferred that the specific polymer has a plurality of reactive double bonds.

In cases where a maleimide compound is used as a raw material of the specific polymer, the reactive double bonds are preferably contained in the maleimide skeleton.

Further, in cases where a maleimide compound and a compound represented by Formula (5) are used as raw materials of the specific polymer, the reactions yielding the specific polymer preferably include a reaction between a double bond in the maleimide skeleton of the maleimide compound and at least either of -NH- or -CR⁵R⁶- in the cyclic structure of the compound represented by Formula (5).

In this case, in the reactions yielding the specific polymer, it is more preferred that some of the plural double bonds in the whole maleimide compound undergo reaction and the rest remains as reactive double bonds.

The weight-average molecular weight (Mw) of the specific polymer is not particularly restricted; however, it is preferably from 1,000 to 500,000, more preferably from 2,000 to 200,000, still more preferably from 10,000 to 100,000, particularly preferably from 10,000 to 50,000.

Further, the molecular weight distribution [Mw/Mn], which is the ratio of the weight-average molecular weight (Mw) and the number-average molecular weight (Mn), is preferably 200 or less, more preferably 100 or less, particularly preferably 70 or less.

The molecular weight distribution [Mw/Mn] is ideally 1; however, it may be 5 or higher, or 10 or higher.

It is noted here that Mw and Mn each mean a value measured by gel permeation chromatography (GPC) in terms of PEG/PEO.

In at least one of the positive electrode or the negative electrode, the specific polymer may be contained singly, or two or more thereof may be contained in combination.

Further, it is preferred that the specific polymer(s) is/are contained in the composite layer of at least one of the positive electrode or the negative electrode.

The composite layer will be described later.

The electrodes for the lithium secondary battery according to the first embodiment (that is, the positive electrode and the negative electrode) may each comprise a current collector and a composite layer.

It is preferred that at least a part of the current collector is in contact with at least a part of the composite layer.

### <Current Collector>

As the current collector, a variety of current collectors such as metals and alloys can be used.

Examples of the current collector in the positive electrode include aluminum, nickel, and SUS.

Examples of the current collector in the negative electrode include copper, nickel, and SUS.

### <Composite Layer>

The composite layer may contain an active material (a positive electrode active material or a negative electrode active material) and a binder. The composite layer may further contain a conductive aid.

Particularly, the composite layer of the positive electrode preferably contains a conductive aid.

It is preferred that at least one of the composite layer of the positive electrode or the composite layer of the negative electrode contains the above-described specific polymer.

The specific polymer is more preferably contained at least in the composite layer of the positive electrode.

In the composite layer, the specific polymer may be contained singly, or two or more thereof may be contained in combination.

The content of the specific polymer in the composite layer (total content when two or more specific polymers are contained) is not particularly restricted; however, from the standpoint of allowing the effects of the first embodiment to be exerted more effectively, the content of the specific polymer is in a range of preferably from 0.001% by mass to 20% by mass, more preferably from 0.01% by mass to 5% by mass, with respect to the total amount of the composite layer.

### (Active Materials)

The composite layer that may be provided in the positive electrode may contain a positive electrode active material as the active material.

The composite layer that may be provided in the negative electrode may contain a negative electrode active material as the active material.

Preferred modes of each of the positive electrode active material and the negative electrode active material are as described above.

### (Binder)

As the binder, an aqueous binder or a non-aqueous binder may be used.

Examples of the non-aqueous binder include those described in "Latest Lithium Ion Secondary Batteries - Material Development toward Improvement in Safety and Functionality" (p.235, published by Johokiko Co., Ltd., 2008).

The non-aqueous binder is particularly preferably polyvinylidene fluoride.

The aqueous binder is particularly preferably an SBR latex.

### (Conductive Aid)

As the conductive aid, for example, acetylene black and a carbon material, which is a known conductive aid, can be used in combination.

The known conductive aid is not particularly restricted as long as it is a carbon material having electrical conductivity and, for example, graphites, carbon blacks, conductive carbon fibers (carbon nanotubes, carbon nanofibers, carbon fibers) and fullerene may be used singly, or in combination of two or more thereof.

Examples of commercially available carbon black include TOKABLACK #4300, #4400, #4500, #5500 and the like (furnace black, manufactured by Tokai Carbon Co., Ltd.); PRINTEX L and the like (furnace black, manufactured by Degussa-Hüls AG); RAVEN 7000, 5750, 5250, 5000 ULTRA III, 5000 ULTRA and the like, CONDUCTEX SC ULTRA, CONDUCTEX 975ULTRA and the like, PURE BLACK 100, 115, 205 and the like (furnace black, manufactured by Columbian Chemicals Company, Inc.); #2350, #2400B, #2600B, #30050B, #3030B, #3230B, #3350B, #3400B, #5400B and the like (furnace black, manufactured by Mitsubishi Chemical Corporation); MONARCH 1400, 1300, 900, VULCAN XC-72R, BLACK PEARLS 2000 and the like (furnace black, manufactured by Cabot Corporation); ENSACO 250G, ENSACO 260G, ENSACO 350G and SUPER P-Li (manufactured by Timcal Ltd.); KETJEN BLACK EC-300J and EC-600JD (manufactured by AkzoNobel N.V.); and DENKA BLACK, DENKA BLACK HS-100 and FX-35 (acetylene black, manufactured by Denka Co., Ltd.).

Examples of the graphites include, but not limited to, artificial graphites, and natural graphites such as flake graphite, bulk graphite and earthy graphite.

The amount of acetylene black contained in the conductive aid is preferably not less than 5% by mass.

### (Other Components)

The composite layer may also contain other component(s) other than the above-described ones.

For example, in cases where the composite layer is formed from a composite slurry, the composite layer may contain various components originating from the composite slurry.

Examples of the various components originating from the composite slurry include thickening agents, surfactants, dispersants, wetting agents, and antifoaming agents. Specific examples of these various components will be described in the following section "Method of Forming Composite Layer".

### (Method of Forming Composite Layer)

The composite layer can be produced by, for example, preparing a composite slurry and then applying and drying the composite slurry on a current collector.

In the case of forming a composite layer containing the specific polymer (hereinafter, referred to as "specific composite layer"), for example, the specific composite layer may be formed by applying a composite slurry containing the active material, binder, conductive aid and specific polymer; or the specific composite layer may be formed by first applying a composite slurry containing the active material, binder and conductive aid to obtain a coating film and then applying a solution containing the specific polymer onto the thus obtained coating film.

It is preferred that the composite slurry contains a solvent.

As the solvent, an aprotic polar solvent represented by *N-*methylpyrrolidone, dimethyl sulfoxide, propylene carbonate, dimethylformamide, γ-butyrolactone or the like, or a mixture thereof can be selected.

Alternatively, a protic polar solvent such as water can be selected as the solvent.

When an aprotic polar solvent is used as the solvent, it is preferred to use a non-aqueous binder as the binder.

When a protic polar solvent is used as the solvent, it is preferred to use an aqueous binder as the binder.

The composite slurry may also contain a thickening agent.

As the thickening agent, any known thickening agent used for electrochemical cells can be used, and examples thereof include cellulose-based polymers such as carboxymethyl cellulose, methyl cellulose and hydroxypropyl cellulose, as well as their ammonium salts and alkali metal salts; (modified) poly(meth)acrylic acids and their ammonium salts and alkali metal salts; polyvinyl alcohols, such as (modified) polyvinyl alcohols, copolymers of acrylic acid or a salt thereof and a vinyl alcohol, and copolymers of a vinyl alcohol and maleic anhydride, maleic acid or fumaric acid; polyethylene glycols; polyethylene oxides; polyvinylpyrrolidones; modified polyacrylic acids; oxidized starch; starch phosphate; casein; and various modified starches.

If necessary, the composite slurry may also contain an additive(s).

The additives are not particularly restricted, and examples thereof include surfactants, dispersants, wetting agents, and antifoaming agents.

The composite slurry can be prepared by, for example, adding the active material and the binder (as well as, if necessary, other components such as the conductive aid, the specific polymer and the solvent) to a stirrer and stirring these materials.

In the preparation of the composite slurry, the type of the stirrer is not restricted.

Examples of the stirrer include a ball mill, a sand mill, a pigment disperser, a grinder, an ultrasonic disperser, a homogenizer, a planetary mixer, a Hobart mixer, and a high-speed stirrer.

In the application and drying of the composite slurry on a current collector, neither the application method nor the drying method is particularly restricted.

Examples of the application method include slot-die coating, slide coating, curtain coating, and gravure coating.

Examples of the drying method include drying with warm air, hot air or low-humidity air, vacuum drying, and drying with (far)infrared radiation. The drying time and the drying temperature are not particularly restricted; however, the drying time is, for example, from 1 minute to 30 minutes, and the drying temperature is, for example, from 40°C to 180°C.

The method of producing the electrodes for the lithium secondary battery according to the first embodiment is not particularly restricted; however, a production method which comprises the step of forming a composite layer on a current collector by the above-described method of forming a composite layer is preferably employed.

It is more preferred that such a preferred production method further comprises, after the step of forming a composite layer, the step of reducing the porosity of the resulting composite layer by a pressure treatment using a press mold, a roll press or the like.

### <Positive Electrode (Positive Electrode Plate)>

As the positive electrode in the first embodiment, a plate-form positive electrode (hereinafter, also referred to as "positive electrode plate") is suitable.

The positive electrode (e.g., positive electrode plate) is suitably obtained by the above-described method of forming a composite layer, using a positive electrode active material as the active material.

The positive electrode (e.g., positive electrode plate) can be obtained by preparing a composite slurry containing at least the positive electrode active material and a binder and subsequently performing the step of applying the thus obtained composite slurry on a current collector to form a composite layer.

As the binder, any aqueous binder or non-aqueous binder may be used.

It is noted here, however, that it is preferred to use a non-aqueous binder such as polyvinylidene fluoride as the binder for the formation of a composite layer containing the specific polymer.

### <Negative Electrode (Negative Electrode Plate)>

As the negative electrode in the first embodiment, a plate-form negative electrode (hereinafter, also referred to as "negative electrode plate") is suitable.

As the negative electrode (e.g., negative electrode plate), a negative electrode having a conventionally known constitution, or a negative electrode which is the above-described electrode for lithium secondary battery can be used.

The negative electrode (e.g., negative electrode plate) can be produced by, for example, preparing a composite slurry containing a negative electrode active material and subsequently applying and drying the thus obtained composite slurry on the surface of a current collector.

As for the method of preparing the composite slurry, the application method and the drying method, reference can be made to the above-described method of forming a composite layer.

In the preparation of the composite slurry, any aqueous binder or non-aqueous binder may be used; however, it is preferred to use an aqueous binder such as an SBR latex.

The composite slurry may also contain a conductive aid such as carbon black.

### «Non-aqueous Electrolytic Solution»

In the lithium secondary battery according to the first embodiment, the non-aqueous electrolytic solution contains an additive (X).

The additive (X) is at least one compound selected from the group consisting of:
a carbonate compound having a carbon-carbon unsaturated bond;
a carbonate compound having a halogen atom and not having a carbon-carbon unsaturated bond;
an alkali metal salt;
a sulfonic acid ester compound;
a sulfuric acid ester compound;
a nitrile compound;
a dioxane compound; and
an aromatic hydrocarbon compound substituted with at least one substituent selected from the group consisting of a halogen atom, an alkyl group, a halogenated alkyl group, an alkoxy group, a halogenated alkoxy group, an aryl group and a halogenated aryl group.

By incorporating the additive (X) into the non-aqueous electrolytic solution, the discharge capacity retention ratio after repeated charging and discharging (particularly after trickle charging) is improved. In more detail, a decrease in the capacity from the initial discharge capacity after repeated charging and discharging (particularly after trickle charging) is reduced.

The reason why such an effect is obtained is speculated as follows.

That is, as one of the factors to cause a decrease in the discharge capacity, decomposition of the solvent on the negative electrode surface is considered. Specifically, on the negative electrode surface, it is believed that, under the charging conditions, reductive decomposition reaction of the solvent occurs due to the presence of lithium metal in the negative electrode active material. If such reductive decomposition reaction occurs continuously, the discharge capacity would consequently be decreased.

In this respect, according to the lithium secondary battery of the first embodiment, a decrease in the discharge capacity after repeated charging and discharging is effectively suppressed by a combination of the additive (X) contained in the non-aqueous electrolytic solution and the specific polymer contained in at least one of the positive electrode or the negative electrode.

Therefore, the lithium secondary battery according to the first embodiment is expected to have an effect of extending battery service life (that is, an effect of improving battery service life under the actual use conditions where charging and discharging are repeated).

The content of the additive (X) in the non-aqueous electrolytic solution (total content when two or more additives (X) are contained) is not particularly restricted; however, from the standpoint of more effectively maintaining the discharge capacity after repeated charging and discharging, the content of the additive (X) is preferably from 0.001% by mass to 20% by mass, more preferably from 0.05% by mass to 10% by mass, particularly preferably from 0.1% by mass to 5% by mass, with respect to the total amount of the non-aqueous electrolytic solution.

### <Carbonate Compound Having Carbon-Carbon Unsaturated Bond>

The non-aqueous electrolytic solution may contain a carbonate compound having a carbon-carbon unsaturated bond as the additive (X).

The carbonate compound having a carbon-carbon unsaturated bond is preferably at least one selected from the group consisting of chain carbonate compounds represented by Formula (X1), cyclic carbonate compounds represented by Formula (X2), cyclic carbonate compounds represented by Formula (X3) and cyclic carbonate compounds represented by Formula (X4).

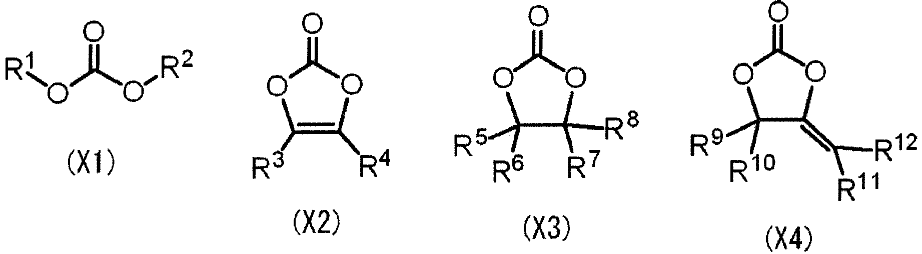

In Formula (X1), R¹ and R² each independently represent a group having from 1 to 12 carbon atoms, which optionally has a carbon-carbon unsaturated bond, an ether bond or a carbon-halogen bond. At least one of R¹ or R² has a carbon-carbon unsaturated bond.

In Formula (X2), R³ and R⁴ each independently represent a hydrogen atom, or a group having from 1 to 12 carbon atoms which optionally has a carbon-carbon unsaturated bond, an ether bond or a carbon-halogen bond.

In Formula (X3), R⁵ to R⁸ each independently represent a hydrogen atom, or a group having from 1 to 12 carbon atoms which optionally has a carbon-carbon unsaturated bond, an ether bond or a carbon-halogen bond. At least one of R⁵ to R⁸ has a carbon-carbon unsaturated bond. R⁵ or R⁶ and R⁷ or R⁸ may be combined to form, in combination with the carbon atoms to which they are respectively bonded, a benzene ring structure or a cyclohexyl ring structure.

In Formula (X4), R⁹ to R¹² each independently represent a hydrogen atom, or a group having from 1 to 12 carbon atoms which optionally has a carbon-carbon unsaturated bond, an ether bond or a carbon-halogen bond.

Specific examples of the carbonate compound having a carbon-carbon unsaturated bond include chain carbonates, such as methylvinyl carbonate, ethylvinyl carbonate, divinyl carbonate, methylallyl carbonate, ethylallyl carbonate, diallyl carbonate, methylpropynyl carbonate, ethylpropynyl carbonate, dipropynyl carbonate, methylphenyl carbonate, ethylphenyl carbonate and diphenyl carbonate; and cyclic carbonates, such as vinylene carbonate, methylvinylene carbonate, 4,4-dimethylvinylene carbonate, 4,5-dimethylvinylene carbonate, vinylethylene carbonate, 4,4-divinylethylene carbonate, 4,5-divinylethylene carbonate, allylethylene carbonate, 4,4-diallylethylene carbonate, 4,5-diallylethylene carbonate, methylene ethylene carbonate, 4,4-dimethyl-5-methylene ethylene carbonate, ethinylethylene carbonate, 4,4-diethinylethylene carbonate, 4,5-diethinylethylene carbonate, propynylethylene carbonate, 4,4-dipropynylethylene carbonate, 4,5-dipropynylethylene carbonate, phenylethylene carbonate, 4,5-diphenylethylene carbonate and phenylene carbonate.

Thereamong, methylphenyl carbonate, ethylphenyl carbonate, diphenyl carbonate, vinylene carbonate, vinylethylene carbonate, 4,4-divinylethylene carbonate, 4,5-divinylethylene carbonate, ethinylethylene carbonate or phenylene carbonate is preferred, and vinylene carbonate, vinylethylene carbonate, ethinylethylene carbonate or phenylene carbonate is more preferred.

In the non-aqueous electrolytic solution, the carbonate compound having a carbon-carbon unsaturated bond may be contained singly, or in combination of two or more thereof.

In cases where the non-aqueous electrolytic solution contains a carbonate compound having a carbon-carbon unsaturated bond as the additive (X), the content thereof (total content when two or more thereof are contained) is not particularly restricted; however, from the standpoint of more effectively maintaining the discharge capacity even after repeated charging and discharging, the content of the carbonate compound is preferably from 0.001% by mass to 20% by mass, more preferably from 0.05% by mass to 10% by mass, particularly preferably from 0.1% by mass to 5% by mass, with respect to the total amount of the non-aqueous electrolytic solution.

### <Carbonate Compound Having Halogen Atom And Not Having Carbon-Carbon Unsaturated Bond>

The non-aqueous electrolytic solution may contain, as the additive (X), a carbonate compound having a halogen atom and not having a carbon-carbon unsaturated bond.

The halogen atom in the carbonate compound is preferably a fluorine atom, a chlorine atom or a bromine atom, more preferably a fluorine atom or a chlorine atom, particularly preferably a fluorine atom.

The carbonate compound having a halogen atom and not having a carbon-carbon unsaturated bond is preferably ethylene carbonate substituted with at least one halogen atom (preferably a fluorine atom or a chlorine atom, more preferably a fluorine atom).

The carbonate compound having a halogen atom and not having a carbon-carbon unsaturated bond is particularly preferably 4-fluoroethylene carbonate (FEC), 4,4-difluoroethylene carbonate, or 4,5-difluoroethylene carbonate.

In the non-aqueous electrolytic solution, the carbonate compound having a halogen atom and not having a carbon-carbon unsaturated bond may be contained singly, or in combination of two or more thereof.

In cases where the non-aqueous electrolytic solution contains a carbonate compound having a halogen atom and not having a carbon-carbon unsaturated bond as the additive (X), the content thereof (total content when two or more thereof are contained) is not particularly restricted; however, from the standpoint of more effectively maintaining the discharge capacity even after repeated charging and discharging, the content of the carbonate compound is preferably from 0.001% by mass to 20% by mass, more preferably from 0.05% by mass to 10% by mass, particularly preferably from 0.1 % by mass to 5% by mass, with respect to the total amount of the non-aqueous electrolytic solution.

### <Alkali Metal Salt>

The non-aqueous electrolytic solution may contain an alkali metal salt as the additive (X).

The alkali metal salt is preferably at least one selected from the group consisting of a monofluorophosphate salt, a difluorophosphate salt, an oxalato salt, a sulfonate salt, a carboxylate salt, an imide salt and a methide salt.

The monofluorophosphate salt, difluorophosphate salt, oxalato salt, sulfonate salt, carboxylate salt, imide salt and methide salt are all alkali metal salts.

Examples of an alkali metal in the alkali metal salt include Li, Na, K, Rb, and Cs and, from the standpoint of allowing the effects of the first embodiment to be exerted more effectively, the alkali metal is preferably Li, Na or K, more preferably Li.

Among the alkali metal salts, from the standpoint of allowing the effects of the first embodiment to be exerted more effectively, at least one selected from the group consisting of a monofluorophosphate salt, a difluorophosphate salt, an oxalato salt and a fluorosulfonate salt is preferred. These salts may be used singly, or in combination of two or more thereof.

### (Monofluorophosphate Salt and Difluorophosphate Salt)

The monofluorophosphate salt is preferably lithium monofluorophosphate (LiPO₃F).

The difluorophosphate salt is preferably lithium difluorophosphate (LiPO₂F₂).

### (Oxalato Salt)

Examples of the oxalato salt include those represented by the following Formula (V).

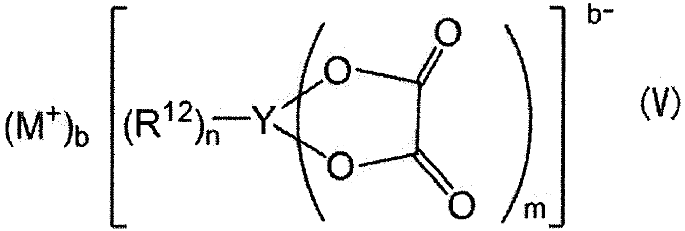

In Formula (V), M represents an alkali metal; Y represents an element of Group 13, 14, 15 or 16 of the periodic table; and b represents an integer from 1 to 3. Further, in Formula (V), m represents an integer from 1 to 4, and n represents an integer from 0 to 8. R¹² represents a halogen atom, an alkyl group having from 1 to 10 carbon atoms, a halogenated alkyl group having from 1 to 10 carbon atoms, an aryl group having from 6 to 20 carbon atoms, a halogenated aryl group having from 6 to 20 carbon atoms (these groups optionally contain a substituent or a heteroatom in their structures and, when n in Formula (V) is 2 to 8, n R¹²s are optionally bonded to each other to form a ring), or -Q³R¹³. Q³ represents O, S, or NR¹⁴. R¹³ and R¹⁴ each independently represent a hydrogen atom, an alkyl group having from 1 to 10 carbon atoms, a halogenated alkyl group having from 1 to 10 carbon atoms, an aryl group having from 6 to 20 carbon atoms, or a halogenated aryl group having from 6 to 20 carbon atoms (these groups optionally contain a substituent or a heteroatom in their structures and, when there are plural R¹³s and/or R¹⁴s, they are optionally bonded to each other to form a ring).

In the salts represented by Formula (V), M is an alkali metal, and Y is an element of Group 13, 14, 15 or 16 of the periodic table. Particularly, Y is preferably Al, B, Ti, Si, Ge, Sn, Ga, Bi, P, As, Sb or S, more preferably Al, B, P or S. When Y is Al, B or P, an anionic compound can be synthesized relatively easily, and the production cost can thus be reduced. The symbol b, which represents the valence of anion and the number of cations, is an integer from 1 to 3, preferably 1. When b is larger than 3, there is a tendency that the resulting salt of the anionic compound does not readily dissolve in a mixed organic solvent, which is not preferred. Further, the constants m and n in Formula (V) are values relating to the number of ligands and determined in accordance with the type of Y; however, in Formula (V), m is an integer from 1 to 4, and n is an integer from 0 to 8.

R¹² represents a halogen atom, an alkyl group having from 1 to 10 carbon atoms, a halogenated alkyl group having from 1 to 10 carbon atoms, an aryl group having from 6 to 20 carbon atoms, a halogenated aryl group having from 6 to 20 carbon atoms, or -Q³R¹³ (Q³ and R¹³ will be described below).

The alkyl group, halogenated alkyl group, aryl group or halogenated aryl group represented by R¹² may contain a substituent or a heteroatom in its structure and, when n in Formula (V) is 2 to 8, n R¹²s may be bonded to each other to form a ring. R¹² is preferably an electron-withdrawing group, particularly preferably a fluorine atom.

Q³ represents O, S, or NR¹⁴. That is, a ligand is bonded to Y via any of these heteroatoms.

R¹³ and R¹⁴ each independently represent a hydrogen atom, an alkyl group having from 1 to 10 carbon atoms, a halogenated alkyl group having from 1 to 10 carbon atoms, an aryl group having from 6 to 20 carbon atoms, or a halogenated aryl group having from 6 to 20 carbon atoms. The alkyl group, halogenated alkyl group, aryl group or halogenated aryl group may contain a substituent or a heteroatom in its structure. Further, when there are plural R¹³s and R¹⁴s, they may be bonded to each other to form a ring.

Examples of the alkali metal represented by M include Li, Na, K, Rb, and Cs. Thereamong, from the standpoint of allowing the effects of the first embodiment to be exerted more effectively, the alkali metal is preferably Li, Na or K, more preferably Li.

In Formula (V), n is preferably an integer from 0 to 4.

In the non-aqueous electrolytic solution of the first embodiment, from the standpoint of obtaining the effects of the invention, an alkali metal salt represented by the following Formula (V-1) can also be used in place of or in addition to the oxalato salt.

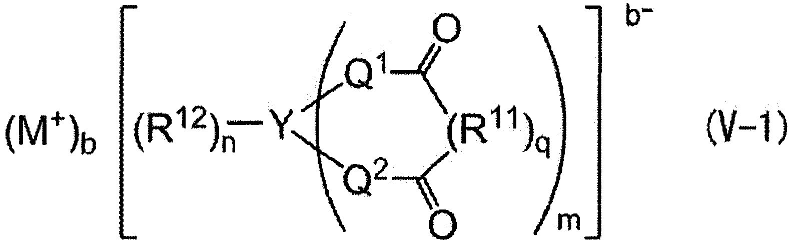

In Formula (V-1), M, Y, m, n, R¹², R¹³ and R¹⁴ have the same meanings as those in Formula (V). Further, b represents an integer from 1 to 3, and q represents 0 or 1. R¹¹ represents an alkylene group having from 1 to 10 carbon atoms, a halogenated alkylene group having from 1 to 10 carbon atoms, an arylene group having from 6 to 20 carbon atoms, or a halogenated arylene group having from 6 to 20 carbon atoms (these groups optionally contain a substituent or a heteroatom in their structures and, when q in Formula (V-1) is 1 and m in Formula (V-1) is 2 to 4, m R¹¹s are optionally bonded to each other). In Formula (V 1), Q¹ and Q² have the same meaning as Q³ in Formula (V).

In the salt represented by Formula (V-1), when the constant q is 1, the chelate ring is a 6-membered ring.

In Formula (V-1), R¹¹ represents an alkylene group having from 1 to 10 carbon atoms, a halogenated alkylene group having from 1 to 10 carbon atoms, an arylene group having from 6 to 20 carbon atoms, or a halogenated arylene group having from 6 to 20 carbon atoms. These alkylene group, halogenated alkylene group, arylene group and halogenated arylene group may contain a substituent or a heteroatom in their structures. Specifically, these groups may contain a halogen atom, a chain or cyclic alkyl group, an aryl group, an alkenyl group, an alkoxy group, an aryloxy group, a sulfonyl group, an amino group, a cyano group, a carbonyl group, an acyl group, an amide group or a hydroxyl group as a substituent in place of a hydrogen atom. Further, these groups may also have a structure in which a nitrogen atom, a sulfur atom or an oxygen atom is introduced in place of a carbon atom. Moreover, when q in Formula (V) is 1 and m in Formula (V) is 2 to 4, m R¹¹s may be bonded to each other. In such a case, examples of a ligand include ethylenediamine tetraacetic acid.

Q¹, Q² and Q³ each independently represent O, S, or NR¹⁴. That is, a ligand is bonded to Y via any of these heteroatoms.

When the non-aqueous electrolytic solution of the first embodiment contains an oxalato salt represented by Formula (V), the oxalato salt represented by Formula (V) may be contained singly, or in combination of two or more thereof. The same also applies to the electrolyte compound represented by Formula (V-1).

Examples of the oxalato salt include at least one salt selected from the group consisting of oxalato salts represented by the following Formula (VI), oxalato salts represented by the following Formula (VII), oxalato salts represented by the following Formula (VIII) and oxalato salts represented by the following Formula (IX); and lithium bisoxalatoborate, among which oxalato salts represented by Formulae (VI) to (IX) are preferred. Further, among those oxalato salts represented by Formulae (VI) to (IX), for example, salts wherein M is lithium, sodium or potassium are preferred as the oxalato salts represented by Formula (V).

Specific examples of the oxalato salts represented by Formulae (VI) to (IX) include lithium difluoro(oxalato)borate, which is represented by Formula (VI) wherein M is lithium; lithium tetrafluoro(oxalato)phosphate, which is represented by Formula (VII) wherein M is lithium; lithium difluorobis(oxalato)phosphate, which is represented by Formula (VIII) wherein M is lithium; and lithium tris(oxalato)phosphate, which is represented by Formula (IX) wherein M is lithium.

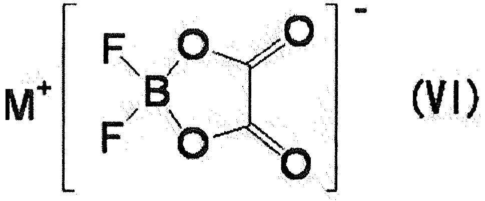

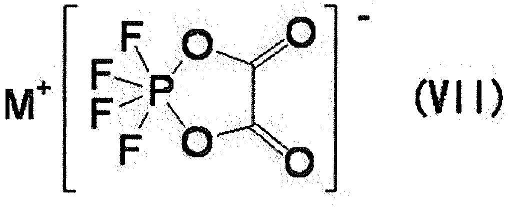

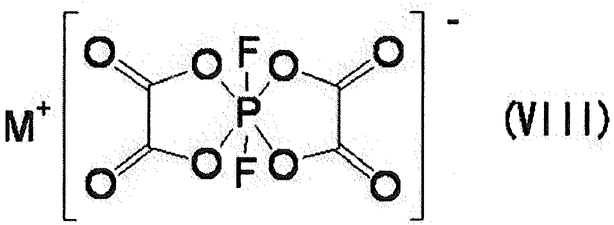

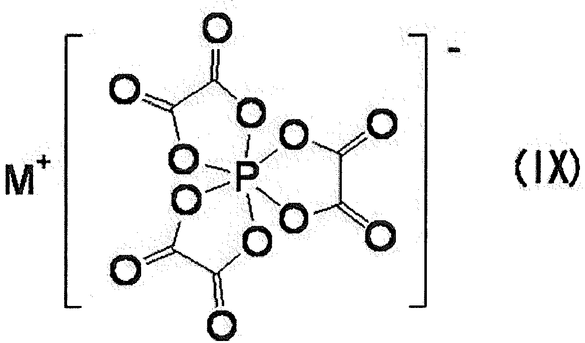

Moreover, among these oxalato salts, ones having one or more fluorine atoms in one molecule are more preferred, and oxalato salts represented by Formulae (VI) to (VIII) are still more preferred. Thereamong, lithium difluoro(oxalato)borate, lithium difluorobis(oxalato)phosphate and lithium tetrafluoro(oxalato)phosphate are particularly preferred.

In Formulae (VI) to (IX), M has the same meaning as in Formula (V).

### (Sulfonate Salt)

Examples of the sulfonate salt include CH₃SO₃Li, CH₂FSO₃Li, CHF₂SO₃Li, CF₃SO₃Li, CF₃CF₂SO₃Li, CF₃CF₂CF₂SO₃Li, CF₃CF₂CF₂CF₂SO₃Li, LiFSO₃, NaFSO₃, KFSO₃, and C_{S}FSO₃. Thereamong, fluorosulfonate salts are preferred.

Examples of the fluorosulfonate salts include those represented by Formula (F1): M(FSO₃). In Formula (F1), M is an alkali metal. Examples of the alkali metal include Li, Na, K, Rb, and Cs. Examples of preferred fluorosulfonate salts include LiFSO₃, NaFSO₃, KFSO₃, and CsFSO₃. Thereamong, LiFSO₃, NaFSO₃ and KFSO₃ are particularly preferred and, from the standpoint of the solubility in an electrolytic solution, LiFSO₃ is most preferred.

### (Carboxylate Salt)

Examples of the carboxylate salt include HCO₂Li, CH₃CO₂Li, CH₂FCO₂Li, CHF₂CO₂Li, CF₃CO₂Li, CF₃CH₂CO₂Li, CF₃CF₂CO₂Li, CF₃CF₂CF₂CO₂Li, and CF₃CF₂CF₂CF₂CO₂Li.

### (Imide Salt)

Examples of the imide salt include LiN(FCO₂)₂, LiN(FCO)(FSO₂), LiN(FSO₂)₂, LiN(FSO₂)(CF₃SO₂), LiN(CF₃SO₂)₂, LiN(C₂F₅SO₂)₂, lithium cyclic 1,2-perfluoroethane disulfonylimide, lithium cyclic 1,3-perfluoropropane disulfonylimide, and LiN(CF₃SO₂)(C₄F₉SO₂).

### (Methide Salt)

Examples of the methide salt include LiC(FSO₂)₃, LiC(CF₃SO₂)₃, and LiC(C₂F₅SO₂)₃.

### (Other Alkali Metal Salts)

As the additive (X), an alkali metal salt other than the above-described ones can also be used.

Examples of such other alkali metal salt include LiAlF₄ and LiSbF₆.

In cases where the non-aqueous electrolytic solution contains an alkali metal salt as the additive (X), the content thereof (total content when two or more alkali metal salts are contained) is not particularly restricted; however, from the standpoint of more effectively maintaining the discharge capacity even after repeated charging and discharging, the content of the alkali metal salt is preferably from 0.001% by mass to 20% by mass, more preferably from 0.05% by mass to 10% by mass, particularly preferably from 0.1% by mass to 5% by mass, with respect to the total amount of the non-aqueous electrolytic solution.

### <Sulfonic Acid Ester Compound>

The non-aqueous electrolytic solution may contain a sulfonic acid ester compound as the additive (X).

The sulfonic acid ester compound is preferably at least one selected from the group consisting of chain sulfonic acid ester compounds represented by Formula (X6), cyclic sulfonic acid ester compounds represented by Formula (X7), cyclic sulfonic acid ester compounds represented by Formula (X8) and disulfonic acid ester compounds represented by Formula (X9).

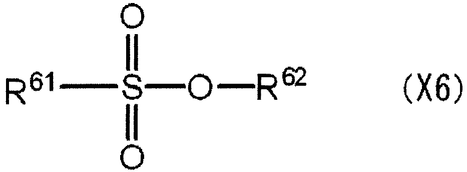

In Formula (X6), R⁶¹ and R⁶² each independently represent a linear or branched aliphatic hydrocarbon group having from 1 to 12 carbon atoms, an aryl group having from 6 to 12 carbon atoms, or a heterocyclic group having from 6 to 12 carbon atoms. Each of these groups may be substituted with a halogen atom.

The aliphatic hydrocarbon group may be substituted with at least one of an alkoxy group, an alkenyloxy group, or an alkynyloxy group. Further, a heteroatom contained in the heterocyclic group is preferably an oxygen atom or a nitrogen atom.

Specific examples of the chain sulfonic acid ester compounds represented by Formula (X6) include chain sulfonic acid ester compounds represented by Formulae (X6-1) to (X6-3).

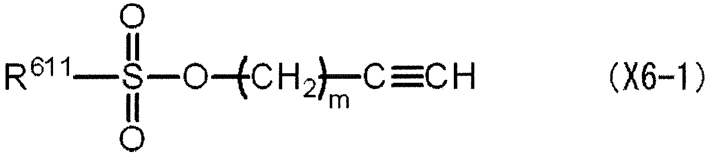

In Formula (X6-1), R⁶¹¹ represents an alkyl group having from 1 to 12 carbon atoms, a halogenated alkyl group having from 1 to 12 carbon atoms, or an aryl group having from 6 to 12 carbon atoms, and m is 1 or 2.

In Formula (X6-1), R⁶¹¹ is preferably an alkyl group having from 1 to 6 carbon atoms, a halogenated alkyl group having from 1 to 6 carbon atoms, or an aryl group having from 6 to 12 carbon atoms.

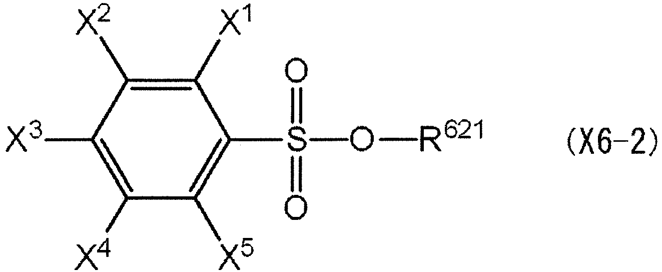

In Formula (X6-2), X¹ to X⁵ each independently represent a fluorine atom or a hydrogen atom.

In Formula (X6-2), R⁶²¹ represents an alkynyl group having from 3 to 6 carbon atoms, or an aryl group having from 6 to 12 carbon atoms. Examples of the alkynyl group having from 3 to 6 carbon atoms include a 2-propynyl group (same as a propargyl group), a 2-butynyl group, a 3-butynyl group, a 4-pentynyl group, a 5-hexynyl group, a 1-methyl-2-propynyl group, a 1-methyl-2-butynyl group, and a 1,1-dimethyl-2-propynyl group. Examples of the aryl group include a phenyl group and a biphenyl group.

Specific examples of the sulfonic acid ester compounds represented by Formula (X6-2) wherein X¹ is a fluorine atom and X², X³, X⁴ and X⁵ are hydrogen atoms include propargyl 2-fluorobenzenesulfonate, 2-butynyl 2-fluorobenzenesulfonate, 3-butynyl 2-fluorobenzenesulfonate, 4-pentynyl 2-fluorobenzenesulfonate, 5-hexynyl 2-fluorobenzenesulfonate, 1 -methyl-2-propynyl 2-fluorobenzenesulfonate, 1-methyl-2-butynyl 2-fluorobenzenesulfonate, 1,1-dimethyl-2-propynyl 2-fluorobenzenesulfonate, phenyl 2-fluorobenzenesulfonate, and biphenyl 2-fluorobenzenesulfonate.

Further, examples of 3-fluorobenzenesulfonates, 4-fluorobenzenesulfonates, 2,4-difluorobenzenesulfonates, 2,6-difluorobenzenesulfonates, 2,4,6-trifluorobenzenesulfonates and 2,3,4,5,6-pentafluorobenzenesulfonates include, in the same manner as above, corresponding sulfonic acid ester compounds.

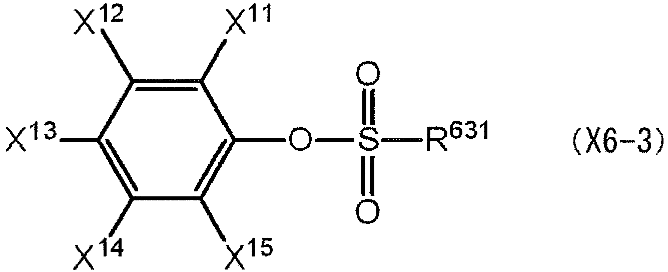

In Formula (X6-3), X¹¹ to X¹⁵ each independently represent a fluorine atom or a hydrogen atom, with two to four of X¹¹ to X¹⁵ being fluorine atoms; and R⁶³¹ represents a linear or branched alkyl group having from 1 to 6 carbon atoms, a linear or branched alkyl group having from 1 to 6 carbon atoms in which at least one hydrogen atom is substituted with a halogen atom, or an aryl group having from 6 to 9 carbon atoms.

Examples of the linear or branched alkyl group having from 1 to 6 carbon atoms, which is R⁶³¹ in Formula (X6-3), include a methyl group, an ethyl group, an *n*-propyl group, an isopropyl group, an *n*-butyl group, an isobutyl group, a *sec*-butyl group, a *tert*-butyl group, an *n*-pentyl group, a neopentyl group, a *sec*-pentyl group, a *tert*-pentyl group, an *n*-hexyl group, and a 2-hexyl group. Examples of the linear or branched alkyl group having from 1 to 6 carbon atoms in which at least one hydrogen atom is substituted with a halogen atom, which alkyl group is R⁶³¹ in Formula (X6-3), include the above-exemplified alkyl groups in which at least one hydrogen atom is substituted with a halogen atom, and specific examples thereof include a trifluoromethyl group and a 2,2,2-trifluoroethyl group.

Examples of the aryl group having from 6 to 9 carbon atoms, which is R⁶³¹ in Formula (X6-3), include a phenyl group, a tosyl group, and a mesityl group.

Preferred examples of the sulfonic acid ester compounds represented by Formula (X6-3) wherein R⁶³¹ is a methyl group include 2,3-difluorophenyl methanesulfonate, 2,4-difluorophenyl methanesulfonate, 2,5-difluorophenyl methanesulfonate, 2,6-difluorophenyl methanesulfonate, 3,4-difluorophenyl methanesulfonate, 3,5-difluorophenyl methanesulfonate, 2,3,4-trifluorophenyl methanesulfonate, 2,3,5-trifluorophenyl methanesulfonate, 2,3,6-trifluorophenyl methanesulfonate, 2,4,5-trifluorophenyl methanesulfonate, 2,4,6-trifluorophenyl methanesulfonate, 3,4,5-trifluorophenyl methanesulfonate, and 2,3,5,6-tetrafluorophenyl methanesulfonate.

Further, when R⁶³¹ is an ethyl group, an *n*-propyl group, an isopropyl group, an *n*-butyl group, an isobutyl group, a *sec*-butyl group, a *tert*-butyl group, an *n*-pentyl group, a neopentyl group, a *sec*-pentyl group, a *tert*-pentyl group, an *n*-hexyl group, a 2-hexyl group or the like, preferred examples of the sulfonic acid ester compounds represented by Formula (X6-3) include, in the same manner as above, corresponding sulfonic acid ester compounds.

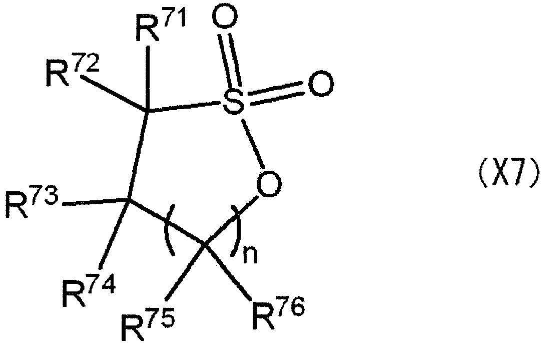

In Formula (X7), R⁷¹ to R⁷⁶ each independently represent a hydrogen atom, a halogen atom, or an alkyl group having from 1 to 6 carbon atoms, and n is an integer from 0 to 3.

In Formula (X7), the alkyl group having from 1 to 6 carbon atoms may be an alkyl group which is optionally substituted with a halogen atom.

In Formula (X7), specific examples of the "halogen atom" include a fluorine atom, a chlorine atom, a bromine atom, and an iodine atom.

The halogen atom is preferably a fluorine atom.

In Formula (X7), the "alkyl group having from 1 to 6 carbon atoms" is a linear or branched alkyl group having from 1 to 6 carbon atoms, and specific examples thereof include a methyl group, an ethyl group, a propyl group, an isopropyl group, a butyl group, an isobutyl group, a *sec*-butyl group, a *tert*-butyl group, a pentyl group, a 2-methylbutyl group, a 1-methylpentyl group, a neopentyl group, a 1-ethylpropyl group, a hexyl group, and a 3,3-dimethylbutyl group.

The alkyl group having from 1 to 6 carbon atoms is more preferably an alkyl group having from 1 to 3 carbon atoms.

In Formula (X7), the "alkyl group having from 1 to 6 carbon atoms which is optionally substituted with a halogen atom" is a linear or branched halogenated alkyl group having from 1 to 6 carbon atoms, and specific examples thereof include a fluoromethyl group, a difluoromethyl group, a trifluoromethyl group, a 2,2,2-trifluoroethyl group, a perfluoroethyl group, a perfluoropropyl group, a perfluorobutyl group, a perfluoropentyl group, a perfluorohexyl group, a perfluoroisopropyl group, a perfluoroisobutyl group, a chloromethyl group, a chloroethyl group, a chloropropyl group, a bromomethyl group, a bromoethyl group, a bromopropyl group, an iodomethyl group, an iodoethyl group, and an iodopropyl group.

The halogenated alkyl group having from 1 to 6 carbon atoms is more preferably a halogenated alkyl group having from 1 to 3 carbon atoms.

Examples of a preferred combination of R⁷¹ to R⁷⁶ include combinations in which R⁷¹ and R⁷² are each independently a hydrogen atom, a fluorine atom, or an alkyl group having 1 or 2 carbon atoms which optionally contains a fluorine atom; R⁷³ and R⁷⁴ are each independently a hydrogen atom, a fluorine atom, or an alkyl group having 1 or 2 carbon atoms; R⁷⁵ is a hydrogen atom, a fluorine atom, or an alkyl group having 1 or 2 carbon atoms which optionally contains a fluorine atom; R⁷⁶ is a hydrogen atom, a fluorine atom, or an alkyl group having 1 or 2 carbon atoms which optionally contains a fluorine atom; and, in Formula (7), n is from 1 to 3.

In Formula (X7), n is preferably 1 to 3, more preferably 1 or 2, particularly preferably 1.

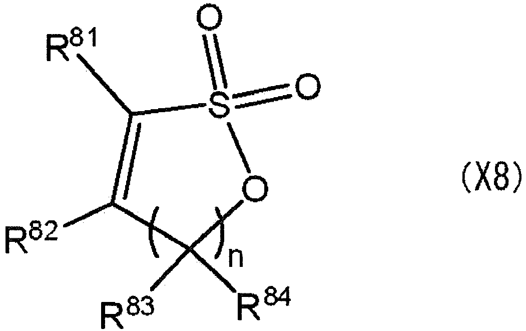

In Formula (X8), R⁸¹ to R⁸⁴ each independently represent a hydrogen atom, a halogen atom, or an alkyl group having from 1 to 6 carbon atoms, and n is an integer from 0 to 3.

The alkyl group having from 1 to 6 carbon atoms may be an alkyl group which is optionally substituted with a halogen atom.

In Formula (X8), the "halogen atom" has the same meaning as the "halogen atom" in Formula (X7), and specific examples and preferred scope of the "halogen atom" in Formula (X8) are the same as those of the "halogen atom" in Formula (7).

In Formula (X8), the "alkyl group having from 1 to 6 carbon atoms" has the same meaning as the "alkyl group having from 1 to 6 carbon atoms" in Formula (X7), and specific examples of the "alkyl group having from 1 to 6 carbon atoms" in Formula (X8) are the same as those of the "alkyl group having from 1 to 6 carbon atoms" in Formula (X7).

In Formula (X8), the "alkyl group having from 1 to 6 carbon atoms which is optionally substituted with a halogen atom" has the same meaning as the "alkyl group having from 1 to 6 carbon atoms which is optionally substituted with a halogen atom" in Formula (X7), and specific examples of the "alkyl group having from 1 to 6 carbon atoms which is optionally substituted with a halogen atom" in Formula (X8) are the same as those of the "alkyl group having from 1 to 6 carbon atoms which is optionally substituted with a halogen atom" in Formula (X7).

Examples of a preferred combination of R⁸¹ to R⁸⁴ include combinations in which R⁸¹ is a hydrogen atom, a fluorine atom, or an alkyl group having 1 or 2 carbon atoms which optionally contains a fluorine atom; R⁸² is a hydrogen atom, a fluorine atom, or an alkyl group having 1 or 2 carbon atoms; R⁸³ is a hydrogen atom, a fluorine atom, or an alkyl group having 1 or 2 carbon atoms which optionally contains a fluorine atom; R⁸⁴ is a hydrogen atom, a fluorine atom, or an alkyl group having 1 or 2 carbon atoms which optionally contains a fluorine atom; and n in Formula (X8) is from 1 to 3.

In Formula (X8), n is preferably 1 to 3, more preferably 1 or 2, particularly preferably 1.

Specific examples of the cyclic sulfonic acid ester compounds (unsaturated sultone compounds) represented by Formula (X8) include the following compounds.

It is noted here, however, that the cyclic sulfonic acid ester compounds (unsaturated sultone compounds) represented by Formula (X8) are not restricted to the following compounds.

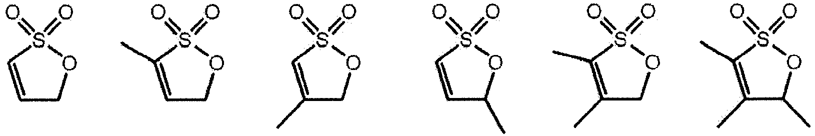

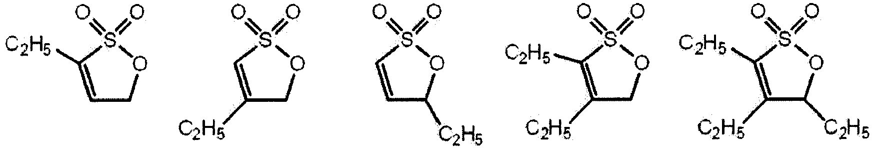

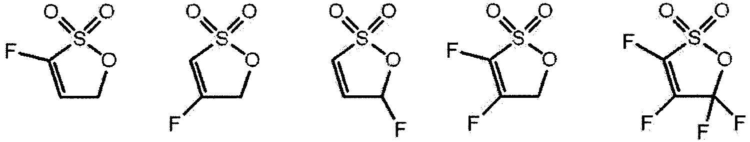

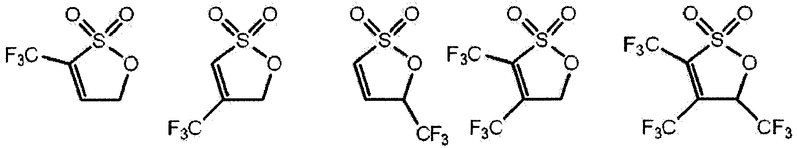

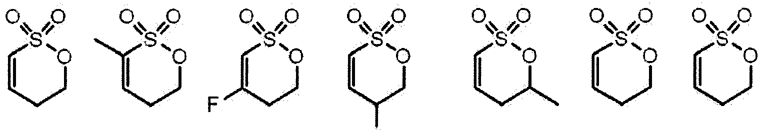

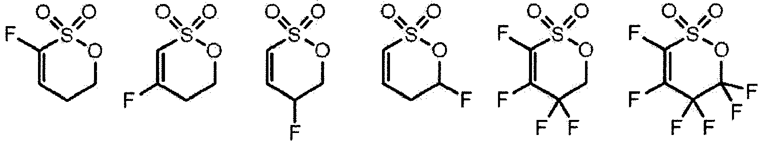

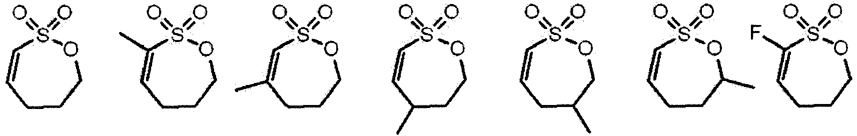

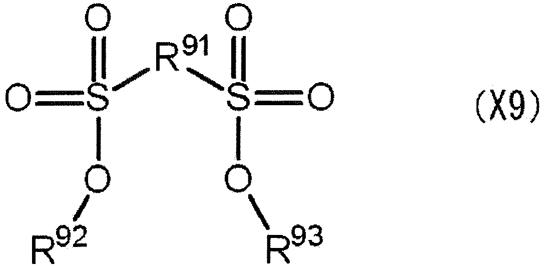

In Formula (X9), R⁹¹ represents an aliphatic hydrocarbon group having from 1 to 10 carbon atoms, or a halogenated alkylene group having from 1 to 3 carbon atoms.

In Formula (X9), R⁹² and R⁹³ each independently represent an alkyl group having from 1 to 6 carbon atoms, or an aryl group; or R⁹² and R⁹³ are combined to represent an alkylene group having from 1 to 10 carbon atoms, or a 1,2-phenylene group which is optionally substituted with a halogen atom, an alkyl group having from 1 to 12 carbon atoms or a cyano group.

In Formula (X9), with regard to R⁹¹, the aliphatic hydrocarbon group having from 1 to 10 carbon atoms is a linear or branched aliphatic hydrocarbon group having from 1 to 10 carbon atoms (preferably a linear or branched alkylene group having from 1 to 10 carbon atoms).

Examples of the aliphatic hydrocarbon group having from 1 to 10 carbon atoms include a methylene group (-CH₂- group), a dimethylene group (-(CH₂)₂- group), a trimethylene group (-(-CH2)3- group), a tetramethylene group (-(CH₂)₄- group), a pentamethylene group (-(CH₂)₅- group), a hexamethylene group (-(CH₂)₆- group), a heptamethylene group (-(CH₂)₇- group), an octamethylene group (-(CH₂)₈- group), a nonamethylene group (-(CH₂)₉- group), and a decamethylene group (-(CH₂)₁₀- group).

Examples of the aliphatic hydrocarbon group having from 1 to 10 carbon atoms also include substituted methylene groups, such as a methylmethylene group (-CH(CH₃)- group), a dimethylmethylene group (-C(CH₃)₂- group), a vinylmethylene group, a divinylmethylene group, an allylmethylene group and a diallylmethylene group.

The aliphatic hydrocarbon group having from 1 to 10 carbon atoms is more preferably an alkylene group having from 1 to 3 carbon atoms, still more preferably a methylene group, a dimethylene group, a trimethylene group or a dimethylmethylene group, yet still more preferably a methylene group or a dimethylene group.

In Formula (X9), with regard to R⁹¹, the halogenated alkylene group having from 1 to 3 carbon atoms is a linear or branched halogenated alkylene group having from 1 to 3 carbon atoms, examples of which include a fluoromethylene group (-CHF- group), a difluoromethylene group (-CF₂- group), and a tetrafluorodimethylene group (-CF₂CF₂-group).

In Formula (X9), R⁹² and R⁹³ each independently represent an alkyl group having from 1 to 6 carbon atoms, or a phenyl group; or R⁹² and R⁹³ are combined to represent an alkylene group having from 1 to 10 carbon atoms, or a 1,2-phenylene group which is optionally substituted with a halogen atom, an alkyl group having from 1 to 12 carbon atoms or a cyano group.

In Formula (X9), with regard to R⁹² and R⁹³, the alkyl group having from 1 to 6 carbon atoms is a linear or branched alkyl group having from 1 to 6 carbon atoms, and specific examples thereof include a methyl group, an ethyl group, a propyl group, an isopropyl group, a butyl group, an isobutyl group, a *sec*-butyl group, a *tert*-butyl group, a pentyl group, a 2-methylbutyl group, a 1-methylpentyl group, a neopentyl group, a 1-ethylpropyl group, a hexyl group, and a 3,3-dimethylbutyl group.

In Formula (X9), with regard to R⁹² and R⁹³, specific examples of the halogen atom include a fluorine atom, a chlorine atom, a bromine atom, and an iodine atom.

In Formula (X9), when R⁹² and R⁹³ are combined to represent an alkylene group having from 1 to 10 carbon atoms, the alkylene group having from 1 to 10 carbon atoms is a linear or branched alkylene group having from 1 to 10 carbon atoms.

When R⁹² and R⁹³ are combined to represent an alkylene group having from 1 to 10 carbon atoms, examples and preferred scope of the alkylene group having from 1 to 10 carbon atoms are the same as those of the aliphatic hydrocarbon group having from 1 to 10 carbon atoms which is represented R⁹¹.

In Formula (X9), with regard to R⁹² and R⁹³, the alkyl group having from 1 to 12 carbon atoms is a linear or branched alkyl group having from 1 to 12 carbon atoms, examples of which include a methyl group, an ethyl group, a propyl group, an isopropyl group, a butyl group, an isobutyl group, a *sec*-butyl group, a *tert*-butyl group, a pentyl group, a 2-methylbutyl group, a 1-methylpentyl group, a neopentyl group, a 1-ethylpropyl group, a hexyl group, a 3,3-dimethylbutyl group, a heptyl group, an octyl group, a nonyl group, a decyl group, an undecanyl group, and a dodecanyl group.

The alkyl group having from 1 to 12 carbon atoms is preferably an alkyl group having from 1 to 6 carbon atoms, more preferably an alkyl group having from 1 to 4 carbon atoms, particularly preferably an alkyl group having from 1 to 3 carbon atoms.

Among the disulfonic acid ester compounds represented by Formula (X9), compounds of a mode in which R⁹² and R⁹³ each independently represent an alkyl group having from 1 to 6 carbon atoms or a phenyl group are represented by the following Formula (X9-1).

Among the disulfonic acid ester compounds represented by Formula (X9), compounds of a mode in which R⁹² and R⁹³ are combined to represent an alkylene group having from 1 to 10 carbon atoms are represented by the following Formula (X9-2).

Further, among the disulfonic acid ester compounds represented by Formula (X9), compounds of a mode in which R⁹² and R⁹³ are combined to represent a 1,2-phenylene group which is optionally substituted with a halogen atom, an alkyl group having from 1 to 12 carbon atoms or a cyano group are represented by the following Formula (X9-3).

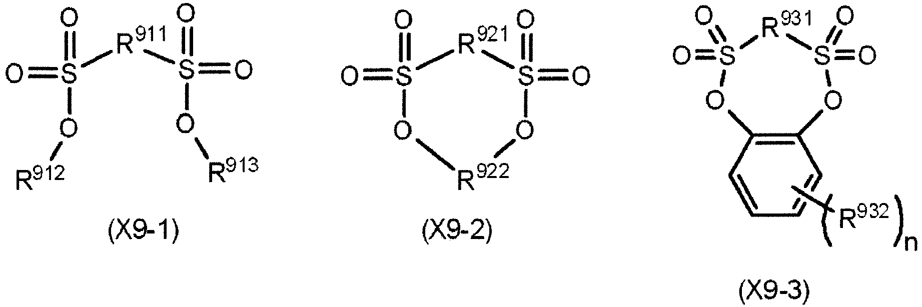

In Formulae (X9-1) to (X9-3), R⁹¹¹, R⁹²¹ and R⁹³¹ have the same meaning as R⁹¹ in Formula (X9).

In Formula (X9-1), R⁹¹² and R⁹¹³ each independently represent an alkyl group having from 1 to 6 carbon atoms, or a phenyl group.

In Formula (X9-2), R⁹²² represents an alkylene group having from 1 to 10 carbon atoms.

In Formula (X9-3), R⁹³² represents a halogen atom, an alkyl group having from 1 to 12 carbon atoms, or a cyano group, and n represents an integer from 0 to 4 (preferably 0, 1 or 2, particularly preferably 0).

In cases where the non-aqueous electrolytic solution contains a sulfonic acid ester compound as the additive (X), the content thereof (total content when two or more sulfonic acid ester compounds are contained) is not particularly restricted; however, from the standpoint of more effectively maintaining the discharge capacity even after repeated charging and discharging, the content of the sulfonic acid ester compound is preferably from 0.001% by mass to 20% by mass, more preferably from 0.05% by mass to 10% by mass, particularly preferably from 0.1 % by mass to 5% by mass, with respect to the total amount of the non-aqueous electrolytic solution.

### <Sulfuric Acid Ester Compound>

The non-aqueous electrolytic solution may contain a sulfuric acid ester compound as the additive (X).

The sulfuric acid ester compound is preferably at least one compound selected from the group consisting of chain sulfuric acid ester compounds represented by Formula (X10) and cyclic sulfuric acid ester compounds represented by Formula (X11).

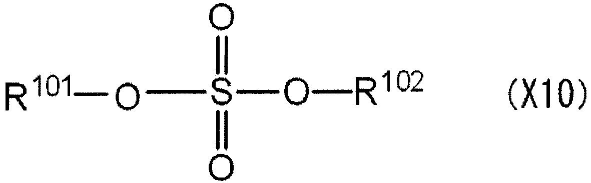

In Formula (X10), R¹⁰¹ and R¹⁰² each independently represent a linear or branched aliphatic hydrocarbon group having from 1 to 12 carbon atoms, an aryl group having from 6 to 12 carbon atoms, or a heterocyclic group having from 6 to 12 carbon atoms. Each of these groups may be substituted with a halogen atom.

The aliphatic hydrocarbon group may be substituted with at least one of an alkoxy group, an alkenyloxy group, or an alkynyloxy group. Further, a heteroatom contained in the heterocyclic group is preferably an oxygen atom or a nitrogen atom.

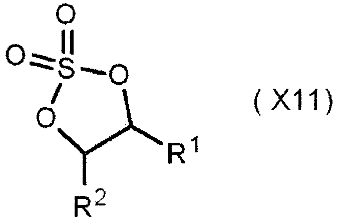

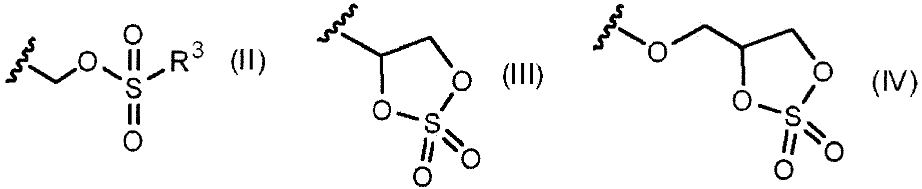

In Formula (X11), R¹ and R² each independently represent a hydrogen atom, an alkyl group having from 1 to 6 carbon atoms, a phenyl group, a group represented by Formula (II) or a group represented by Formula (III), or R¹ and R² are combined to represent, in combination with the carbon atoms to which R¹ and R² are bonded, a group forming a benzene ring or a cyclohexyl ring.

In Formula (II), R³ represents a halogen atom, an alkyl group having from 1 to 6 carbon atoms, a halogenated alkyl group having from 1 to 6 carbon atoms, an alkoxy group having from 1 to 6 carbon atoms, or a group represented by Formula (IV). The wavy lines in Formulae (II), (III) and (IV) each represent a bonding position.

When a cyclic sulfuric acid ester compound represented by Formula (X11) contains two groups each represented by Formula (II), the two groups each represented by Formula (II) may be the same or different from each other.

In Formula (II), specific examples of the "halogen atom" include a fluorine atom, a chlorine atom, a bromine atom, and an iodine atom.

The halogen atom is preferably a fluorine atom.

In Formulae (X11) and (II), the "alkyl group having from 1 to 6 carbon atoms" is a linear or branched alkyl group having from 1 to 6 carbon atoms, and specific examples thereof include a methyl group, an ethyl group, a propyl group, an isopropyl group, a butyl group, an isobutyl group, a *sec*-butyl group, a *tert*-butyl group, a pentyl group, a 2-methylbutyl group, a 1-methylpentyl group, a neopentyl group, a 1-ethylpropyl group, a hexyl group, and a 3,3-dimethylbutyl group.

The alkyl group having from 1 to 6 carbon atoms is more preferably an alkyl group having from 1 to 3 carbon atoms.

In Formula (II), the "halogenated alkyl group having from 1 to 6 carbon atoms" is a linear or branched halogenated alkyl group having from 1 to 6 carbon atoms, and specific examples thereof include a fluoromethyl group, a difluoromethyl group, a trifluoromethyl group, a 2,2,2-trifluoroethyl group, a perfluoroethyl group, a perfluoropropyl group, a perfluorobutyl group, a perfluoropentyl group, a perfluorohexyl group, a perfluoroisopropyl group, a perfluoroisobutyl group, a chloromethyl group, a chloroethyl group, a chloropropyl group, a bromomethyl group, a bromoethyl group, a bromopropyl group, an iodomethyl group, an iodoethyl group, and an iodopropyl group.

The halogenated alkyl group having from 1 to 6 carbon atoms is more preferably a halogenated alkyl group having from 1 to 3 carbon atoms.

In Formula (II), the "alkoxy group having from 1 to 6 carbon atoms" is a linear or branched alkoxy group having from 1 to 6 carbon atoms, and specific examples thereof include a methoxy group, an ethoxy group, a propoxy group, an isopropoxy group, a butoxy group, an isobutoxy group, a *sec*-butoxy group, a *tert*-butoxy group, a pentyloxy group, a 2-methylbutoxy group, a 1-methylpentyloxy group, a neopentyloxy group, a 1-ethylpropoxy group, a hexyloxy group, and a 3,3-dimethylbutoxy group.

The alkoxy group having from 1 to 6 carbon atoms is more preferably an alkoxy group having from 1 to 3 carbon atoms.

A preferred mode of Formula (X11) is a mode in which R¹ is a group represented by Formula (II) (wherein, R³ is preferably a fluorine atom, an alkyl group having from 1 to 3 carbon atoms, a halogenated alkyl group having from 1 to 3 carbon atoms, an alkoxy group having from 1 to 3 carbon atoms, or a group represented by Formula (IV)) or a group represented by Formula (III), and R² is a hydrogen atom, an alkyl group having from 1 to 3 carbon atoms, a group represented by Formula (II) or a group represented by Formula (III); or R¹ and R² are combined to form, in combination with the carbon atoms to which R¹ and R² are bonded, a group forming a benzene ring or a cyclohexyl ring.

In Formula (X11), R² is more preferably a hydrogen atom, an alkyl group having from 1 to 3 carbon atoms, a group represented by Formula (II) (wherein, R³ is more preferably a fluorine atom, an alkyl group having from 1 to 3 carbon atoms, a halogenated alkyl group having from 1 to 3 carbon atoms, an alkoxy group having from 1 to 3 carbon atoms, or a group represented by Formula (IV)) or a group represented by Formula (III), still more preferably a hydrogen atom or a methyl group.

When R¹ in Formula (X11) is a group represented by Formula (II), R³ in Formula (II) is, as described above, a halogen atom, an alkyl group having from 1 to 6 carbon atoms, a halogenated alkyl group having from 1 to 6 carbon atoms, an alkoxy group having from 1 to 6 carbon atoms or a group represented by Formula (IV), and R³ is more preferably a fluorine atom, an alkyl group having from 1 to 3 carbon atoms, a halogenated alkyl group having from 1 to 3 carbon atoms, an alkoxy group having from 1 to 3 carbon atoms or a group represented by Formula (IV), still more preferably a fluorine atom, a methyl group, an ethyl group, a trifluoromethyl group, a methoxy group, an ethoxy group or a group represented by Formula (IV).

When R² in Formula (X11) is a group represented by Formula (II), the preferred scope of R³ in Formula (II) is the same as that of R³ in the case where R¹ in Formula (I) is a group represented by Formula (II).

A preferred combination of R¹ and R² in Formula (X11) is a combination in which R¹ is a group represented by Formula (II) (wherein, R³ is preferably a fluorine atom, an alkyl group having from 1 to 3 carbon atoms, a halogenated alkyl group having from 1 to 3 carbon atoms, an alkoxy group having from 1 to 3 carbon atoms, or a group represented by Formula (IV)) or a group represented by Formula (III), and R² is a hydrogen atom, an alkyl group having from 1 to 3 carbon atoms, a group represented by Formula (II) (wherein, R³ is preferably a fluorine atom, an alkyl group having from 1 to 3 carbon atoms, a halogenated alkyl group having from 1 to 3 carbon atoms, an alkoxy group having from 1 to 3 carbon atoms, or a group represented by Formula (IV)) or a group represented by Formula (III).

A more preferred combination of R¹ and R² in Formula (X11) is a combination in which R¹ is a group represented by Formula (II) (wherein, R³ is preferably a fluorine atom, a methyl group, an ethyl group, a trifluoromethyl group, a methoxy group, an ethoxy group, or a group represented by Formula (IV)) or a group represented by Formula (III), and R² is a hydrogen atom or a methyl group.

Examples of the cyclic sulfuric acid ester compounds represented by Formula (X11) include catechol sulfate, 1,2-cyclohexyl sulfate, and compounds represented by the following Exemplary Compounds 1 to 30. However, the cyclic sulfuric acid ester compounds represented by Formula (X11) are not restricted thereto.

In the structures of the following Exemplary Compounds, "Me", "Et", "Pr", "iPr", "Bu", "tBu", "Pent", "Hex", "OMe", "OEt", "OPr", "OBu", "OPent" and "OHex" represent a methyl group, an ethyl group, a propyl group, an isopropyl group, a butyl group, a tertiary butyl group, a pentyl group, a hexyl group, a methoxy group, an ethoxy group, a propoxy group, a butoxy group, a pentyloxy group and a hexyloxy group, respectively. Further, the wavy lines in R¹ to R³ each represent a bonding position.

Stereoisomers derived from substituents at the 4- and 5-positions of a 2,2-dioxo-1,3,2-dioxathiolane ring may occur, and both of the stereoisomers are compounds that correspond to the sulfuric acid ester compounds represented by Formula (X11).

Further, among the sulfuric acid ester compounds represented by Formula (X11), those compounds containing two or more asymmetric carbons in the molecule each have stereoisomers (diastereomers) and, unless otherwise specified, such compounds are each a mixture of corresponding diastereomers.

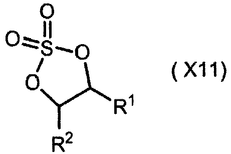

| | R¹ | R² | R³ |
|---|---|---|---|
| 1 | 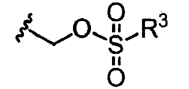 | H | Me |
| 2 | 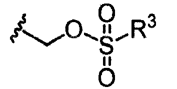 | H | Et |
| 3 | 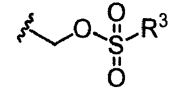 | H | Pr |
| 4 | 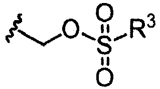 | H | iPr |
| 5 | 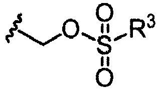 | H | Bu |
| 6 | 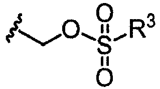 | H | tBu |
| 7 | 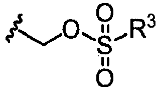 | H | Pent |
| 8 | 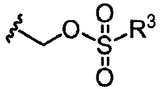 | H | Hex |
| 9 | 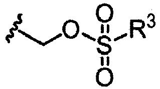 | H | CF₃ |
| 10 | 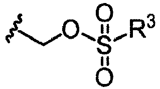 | H | CHF₂ |

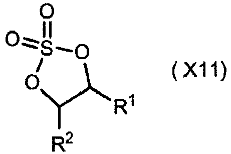

| Exemplary Compound No. | R¹ | R² | R³ |
|---|---|---|---|
| 11 | 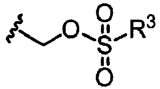 | H | CH₂CF₃ |
| 12 | 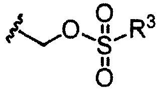 | H | CH₂CH₂CF₃ |
| 13 | 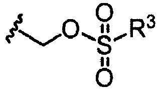 | H | CH₂CH₂CH₂CF₃ |
| 14 | 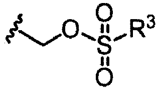 | H | CH₂CH₂CH₂CH₂CF₃ |
| 15 | 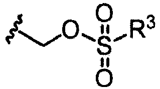 | H | CH₂CH₂CH₂CH₂CH₂CF₃ |
| 16 | 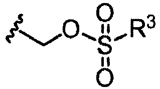 | H | 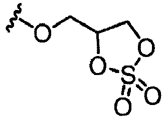 |
| 17 | 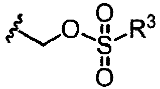 | Me | Me |
| 18 | 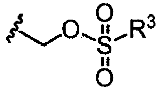 | Et | Me |
| 19 | 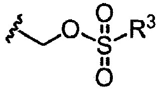 | Hex | Me |
| 20 | 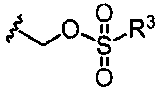 | 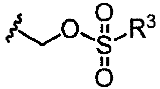 | Me |

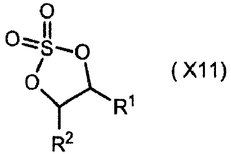

| Exemplary Compound No. | R¹ | R² | R³ |
|---|---|---|---|
| 21 | 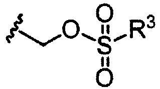 | 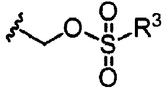 | Et |
| 22 | 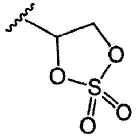 | H | - |
| 23 | 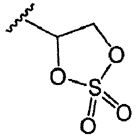 | 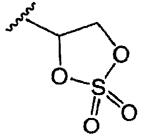 | - |
| 24 | 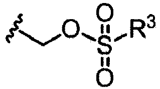 | H | F |
| 25 | 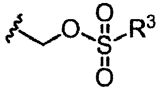 | H | OMe |
| 26 | 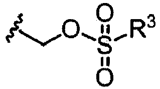 | H | OEt |
| 27 | 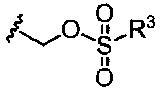 | H | OPr |
| 28 | 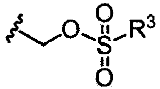 | H | OBu |
| 29 | 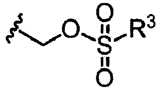 | H | OPent |
| 30 | 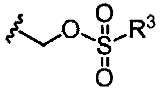 | H | OHex |

In cases where the non-aqueous electrolytic solution contains a sulfuric acid ester compound as the additive (X), the content thereof (total content when two or more sulfuric acid ester compounds are contained) is not particularly restricted; however, from the standpoint of more effectively maintaining the discharge capacity even after repeated charging and discharging, the content of the sulfuric acid ester compound is preferably from 0.001% by mass to 20% by mass, more preferably from 0.05% by mass to 10% by mass, particularly preferably from 0.1 % by mass to 5% by mass, with respect to the total amount of the non-aqueous electrolytic solution.

### <Nitrile Compound>

The non-aqueous electrolytic solution may contain a nitrile compound as the additive (X).

The nitrile compound is not particularly restricted as long as it is a compound which contains at least one nitrile group in one molecule.

In the present specification, the term "nitrile group" refers to a -CN group (that is, a cyano group).

As the nitrile compound, any known nitrile compound described in, for example, Japanese Patent No. 5289091, JP-A No. 2004-179146, JP-ANo. H7-176322, JP-ANo. 2009-32653 or JP-A No. 2010-15968 can be used with no particular restriction.

The number of nitrile groups contained in one molecule of the nitrile compound is not particularly restricted; however, it is preferably 1 to 4, more preferably 1 to 3, still more preferably 1 or 2, particularly preferably 2.

More specifically, examples of the nitrile compound include:
compounds containing one nitrile group (cyano group) in one molecule (mononitrile compounds), such as acetonitrile, propionitrile, butyronitrile, valeronitrile, hexanenitrile, octanenitrile, undecanenitrile, decanenitrile, cyclohexanecarbonitrile, benzonitrile and phenylacetonitrile;
compounds containing two nitrile groups (cyano groups) in one molecule (dinitrile compounds), such as malononitrile, succinonitrile, glutaronitrile, adiponitrile, pimelonitrile, suberonitrile, azelanitrile, sebaconitrile, undecanedinitrile, dodecanedinitrile, methylmalononitrile, ethylmalononitrile, isopropylmalononitrile, *tert*-butylmalononitrile, methylsuccinonitrile, 2,2-dimethylsuccinonitrile, 2,3-dimethylsuccinonitrile, trimethylsuccinonitrile, tetramethylsuccinonitrile, 3,3'-oxydipropionitrile, 3,3'-thiodipropionitrile, 3,3'-(ethylenedioxy)dipropionitrile, 3,3'-(ethylenedithio)dipropionitrile, 1,2-benzodinitrile, 1,3-benzodinitrile, 1,4-benzodinitrile, 1,2-dicyanocyclobutane, 1,1-dicyanoethylacetate, 2,3-dicyanohydroquinone, 4,5-dicyanoimidazole, 2,4-dicyano-3-methylglutamide, 9-dicyanomethylene-2,4,7-trinitrofluorene and 2,6-dicyanotoluene;
compounds containing three nitrile groups (cyano groups) in one molecule (trinitrile compounds), such as 1,2,3-propanetricarbonitrile, 1,3,5-pentanetricarbonitrile, 1,2,3-tris(2-cyanoethoxy)propane and 1,3,5-benzenetricarbonitrile; and
compounds containing four nitrile groups (cyano groups) in one molecule (tetranitrile compounds), such as tetracyanoethylene, tetracyanoethylene oxide, 7,7,8,8-tetracyanoquinodimethane and 1,1,3,3-tetracyanopropane.

From the standpoint of allowing the effects of the first embodiment to be exerted more effectively, the nitrile compound is preferably a nitrile compound represented by Formula (X12).

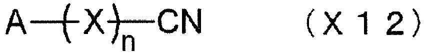

In Formula (X12), A represents a hydrogen atom or a nitrile group.

In Formula (X12), X represents -CH₂-, -CFH-, -CF₂-, -CHR¹¹-, -CFR¹²-, -CR¹³R¹⁴-, -C(=O)-, -O-, -S-, -NH-, or -NR¹⁵-.

In Formula (X12), R¹¹ to R¹⁵ each independently represent a hydrocarbon group having from 1 to 5 carbon atoms which optionally has a substituent, or a nitrile group.

In Formula (X12), n represents an integer of 1 or larger.

In Formula (X12), when n is an integer of 2 or larger, plural Xs may be the same or different from each other.

In Formula (X12), when there are plural R¹¹s to R¹⁵s, the plural R¹¹s to R¹⁵s may be the same or different from each other.

In Formula (X12), the upper limit of n is not particularly restricted; however, n is preferably an integer from 1 to 12, more preferably an integer from 1 to 8, particularly preferably an integer from 1 to 6.

In Formula (X12), with regard to R¹¹ to R¹⁵, examples of the substituent in the "hydrocarbon group having from 1 to 5 carbon atoms which optionally has a substituent" include halogen atoms (preferably a fluorine atom), alkoxy groups (preferably an alkoxy group having from 1 to 3 carbon atoms, more preferably a methoxy group or an ethoxy group), and a nitrile group.

In Formula (X12), with regard to R¹¹ to R¹⁵, examples of the "hydrocarbon group having from 1 to 5 carbon atoms which optionally has a substituent" include, as unsubstituted hydrocarbon groups having from 1 to 5 carbon atoms, alkyl groups having from 1 to 5 carbon atoms such as a methyl group, an ethyl group, a propyl group, an isopropyl group, a butyl group, a 1-methylpropyl group, a 2-methylbutyl group, a *tert*-butyl group, a pentyl group, a 1-methylbutyl group, a 2-methylbutyl group, a 3-methylbutyl group, and a neopentyl group.

The hydrocarbon group having from 1 to 5 carbon atoms is more preferably an alkyl group having from 1 to 3 carbon atoms.

In Formula (X12), with regard to R¹¹ to R¹⁵, examples of the "hydrocarbon group having from 1 to 5 carbon atoms which optionally has a substituent" include, as substituted hydrocarbon groups having from 1 to 5 carbon atoms, a fluoromethyl group, a difluoromethyl group, a trifluoromethyl group, a 2-fluoroethyl group, a 2,2-difluoroethyl group, a 2,2,2-trifluoroethyl group, a 1,1,2,2,2-pentafluoroethyl group, a methoxymethyl group, an ethoxymethyl group, a 2-methoxyethyl group, a cyanomethyl group, a 2-cyanoethyl group, a 3-cyanopropyl group, a methoxycarbonylmethyl group, an ethoxycarbonylmethyl group, and a 2-methoxycarbonylethyl group.

In Formula (X12), from the standpoint of the stability inside the battery, X is preferably --CH₂-, -CFH-, -CF₂-, -CHR¹¹-, -CFR¹²-, -CR¹³R¹⁴-, -O-, -S- or -NR¹⁵-, more preferably -CH₂-, -CFH-, -CF₂-, -CHR¹¹-, -CFR¹²-, -CR¹³R¹⁴- or -O-, still more preferably -CH₂-, -CFH-, -CF₂-, -CHR¹¹-, -CFR¹²- or -CR¹³R¹⁴-, most preferably -CH₂-.

Further, A in Formula (X12) is, as described above, a hydrogen atom or a nitrile group, and it is preferably a nitrile group.

In a more preferred mode of Formula (X12),
n is an integer from 1 to 8;
X is -CH₂-, -CFH-, -CF₂-, -CHR¹¹-, -CFR¹²-, or -CR¹³R¹⁴-; and
R¹¹ to R¹⁵ are each independently an alkyl group having from 1 to 5 carbon atoms.

In a still more preferred mode of Formula (X12),
n is an integer from 1 to 6; and
X is -CH₂-.

In a particularly preferred mode of Formula (X12),
n is an integer from 1 to 6;
X is -CH₂-; and
A is a nitrile group.

In cases where the non-aqueous electrolytic solution contains a nitrile compound as the additive (X), the content thereof (total content when two or more nitrile compounds are contained) is not particularly restricted; however, from the standpoint of more effectively maintaining the discharge capacity even after repeated charging and discharging, the content of the nitrile compound is preferably from 0.001% by mass to 20% by mass, more preferably from 0.05% by mass to 10% by mass, particularly preferably from 0.1 % by mass to 5% by mass, with respect to the total amount of the non-aqueous electrolytic solution.

### <Dioxane Compound>

The non-aqueous electrolytic solution may contain a dioxane compound as the additive (X).

Examples of the dioxane compound include substituted or unsubstituted 1,3-dioxane, and substituted or unsubstituted 1,4-dioxane.

Examples of a substituent in substituted 1,3-dioxane and substituted 1,4-dioxane include alkyl groups having from 1 to 6 carbon atoms.

The dioxane compound is particularly preferably unsubstituted 1,3-dioxane (hereinafter, also simply referred to as "1,3-dioxane" or "13DOX").

The non-aqueous electrolytic solution may contain only one dioxane compound, or two or more dioxane compounds.

In cases where the non-aqueous electrolytic solution contains a dioxane compound as the additive (X), the content thereof (total content when two or more dioxane compounds are contained) is not particularly restricted; however, from the standpoint of more effectively maintaining the discharge capacity even after repeated charging and discharging, the content of the dioxane compound is preferably from 0.001% by mass to 20% by mass, more preferably from 0.05% by mass to 10% by mass, particularly preferably from 0.1% by mass to 5% by mass, with respect to the total amount of the non-aqueous electrolytic solution.

### <Substituted Aromatic Hydrocarbon Compound>

The non-aqueous electrolytic solution may contain, as the additive (X), an aromatic hydrocarbon compound substituted with at least one substituent selected from the group consisting of a halogen atom, an alkyl group, a halogenated alkyl group, an alkoxy group, a halogenated alkoxy group, an aryl group and a halogenated aryl group (hereinafter, also referred to as "specific aromatic hydrocarbon compound").

Examples of the "halogen atom" in the specific aromatic hydrocarbon compound include a fluorine atom, a chlorine atom, a bromine atom and an iodine atom, and the "halogen atom" is preferably a fluorine atom or a chlorine atom, more preferably a fluorine atom.

Examples of the "alkyl group" in the specific aromatic hydrocarbon compound include linear or branched alkyl groups having from 1 to 10 carbon atoms, and the "alkyl group" is preferably an alkyl group having from 1 to 6 carbon atoms. The alkyl group having from 1 to 6 carbon atoms is more preferably an alkyl group having from 1 to 3 carbon atoms.

The term "halogenated alkyl group" refers to an alkyl group substituted with at least one halogen atom. Specific examples of the "halogenated alkyl group" in the specific aromatic hydrocarbon compound include linear or branched halogenated alkyl groups having from 1 to 10 carbon atoms, and the "halogenated alkyl group" is preferably a halogenated alkyl group having from 1 to 6 carbon atoms. The halogenated alkyl group having from 1 to 6 carbon atoms is more preferably a halogenated alkyl group having from 1 to 3 carbon atoms.

Examples of the "alkoxy group" in the specific aromatic hydrocarbon compound include linear or branched alkoxy groups having from 1 to 10 carbon atoms, and the "alkoxy group" is preferably an alkoxy group having from 1 to 6 carbon atoms. The alkoxy group having from 1 to 6 carbon atoms is more preferably an alkoxy group having from 1 to 3 carbon atoms.

The term "halogenated alkoxy group" refers to an alkoxy group substituted with at least one halogen atom. Specific examples of the "halogenated alkoxy group" in the specific aromatic hydrocarbon compound include linear or branched halogenated alkoxy groups having from 1 to 10 carbon atoms, and the "halogenated alkoxy group" is preferably a halogenated alkoxy group having from 1 to 6 carbon atoms. The halogenated alkoxy group having from 1 to 6 carbon atoms is more preferably a halogenated alkoxy group having from 1 to 3 carbon atoms.

Examples of the "aryl group" in the specific aromatic hydrocarbon compound include aryl groups having from 6 to 20 carbon atoms, and the "aryl group" is preferably an aryl group having from 6 to 12 carbon atoms.

The term "halogenated aryl group" refers to an aryl group substituted with at least one halogen atom. Specific examples of the "halogenated aryl group" in the specific aromatic hydrocarbon compound include halogenated aryl groups having from 6 to 20 carbon atoms, and the "halogenated aryl group" is preferably a halogenated aryl group having from 6 to 12 carbon atoms.

The specific aromatic hydrocarbon compound is an aromatic hydrocarbon compound substituted with at least one substituent selected from the group consisting of a halogen atom, an alkyl group, a halogenated alkyl group, an alkoxy group, a halogenated alkoxy group, an aryl group and a halogenated aryl group (in other words, a substituted aromatic hydrocarbon compound obtained by substitution of an unsubstituted aromatic hydrocarbon compound with at least one substituent selected from the group consisting of a halogen atom, an alkyl group, a halogenated alkyl group, an alkoxy group, a halogenated alkoxy group, an aryl group and a halogenated aryl group), preferably an aromatic hydrocarbon compound substituted with at least one substituent selected from the group consisting of a fluorine atom, a chlorine atom, an alkyl group having from 1 to 6 carbon atoms, a halogenated alkyl group having from 1 to 6 carbon atoms, an alkoxy group having from 1 to 6 carbon atoms, a halogenated alkoxy group having from 1 to 6 carbon atoms, an aryl group having from 6 to 12 carbon atoms and a halogenated aryl group having from 6 to 12 carbon atoms.

Examples of the unsubstituted aromatic compound include aromatic hydrocarbon compounds, such as benzene, naphthalene, anthracene, biphenyl and terphenyl; and heteroaromatic compounds, such as pyridine and dibenzofurane.

The unsubstituted aromatic hydrocarbon compound is preferably an unsubstituted aromatic hydrocarbon compound having 6 to 20 carbon atoms, more preferably an unsubstituted aromatic hydrocarbon compound having 6 to 12 carbon atoms, particularly preferably benzene or biphenyl.

Examples of the specific aromatic hydrocarbon compound include halogenated benzenes, such as fluorobenzene, chlorobenzene, 1,2-difluorobenzene, 1,2-dichlorobenzene, 1,3-difluorobenzene, 1,3-dichlorobenzene, 1,4-difluorobenzene, 1,4-dichlorobenzene, 1,2,3-trifluorobenzene, 1,2,4-trifluorobenzene, 1,3,5-trifluorobenzene, 1,2,4,5-tetrafluorobenzene, pentafluorobenzene and hexafluorobenzene; halogenated toluenes, such as 2-fluorotoluene, 2-chlorotoluene, 3-fluorotoluene, 3-chlorotoluene, 4-fluorotoluene, 4-chlorotoluene, 2,3-difluorotoluene, 2,4-difluorotoluene, 2,5-difluorotoluene, 2,6-difluorotoluene, *α*-fluorotoluene, *α,α*-difluorotoluene, *α,α,α*-trifluorotoluene, tetrafluorotoluene, pentafluorotoluene and 1-fluoro-4-*tert*-butylbenzene; chain alkylbenzenes, such as toluene, xylene, ethylbenzene, propylbenzene, isopropylbenzene, butylbenzene, *sec*-butylbenzene, *tert*-butylbenzene, 1,3-di-*tert*-butylbenzene, pentylbenzene, *tert*-amylbenzene and hexylbenzene; cyclic alkylbenzenes and halogenated alkylbenzenes, such as cyclopentylbenzene, cyclohexylbenzene, 1-fluoro-4-*tert*-butylbenzene, 1-fluoro-2-cyclohexylbenzene, 1-fluoro-3-cyclohexylbenzene and 1-fluoro-4-cyclohexylbenzene; biphenyl, alkylbiphenyls and halogenated biphenyls, such as biphenyl, 4-methylbiphenyl, 4,4-dimethylbiphenyl, 2-fluorobiphenyl, 2-chlorobiphenyl, 3-fluorobiphenyl, 3-chlorobiphenyl, 4-fluorobiphenyl, 4-chlorobiphenyl, 2,2'-difluorobiphenyl, 3,3'-difluorobiphenyl and 4,4'-difluorobiphenyl; terphenyls and partial hydrogenation products thereof, such as *o*-terphenyl, *m*-terphenyl, *p*-terphenyl, 1,2-dicyclohexylbenzene, 2-phenylbicyclohexyl, 1,2-diphenylcyclohexane and *o*-cyclohexylbiphenyl; alkoxybenzenes and halogenated alkoxybenzenes, for example, fluorine-containing anisole compounds such as diphenyl ether, 2,4-difluoroanisole, 2,5-difluoroanisole, 2,6-difluoroanisole and 3,5-difluoroanisole; and heteroaromatic hydrocarbon compounds such as dibenzofuran.

Thereamong, the specific aromatic hydrocarbon compound is preferably fluorobenzene, 2-fluorotoluene, 3-fluorotoluene, biphenyl, 2-fluorobiphenyl, cyclohexylbenzene, *tert*-butylbenzene, or *tert*-amylbenzene.

In cases where the non-aqueous electrolytic solution contains the specific aromatic hydrocarbon compound, the content thereof (total content when two or more specific aromatic hydrocarbon compounds are contained) is not particularly restricted; however, from the standpoint of more effectively maintaining the discharge capacity even after repeated charging and discharging, the content of the specific aromatic hydrocarbon compound is preferably from 0.001% by mass to 20% by mass, more preferably from 0.05% by mass to 10% by mass, particularly preferably from 0.1% by mass to 5% by mass, with respect to the total amount of the non-aqueous electrolytic solution.

Next, other components of the non-aqueous electrolytic solution will be described.

The non-aqueous electrolytic solution generally contains an electrolyte and a non-aqueous solvent.

### <Non-aqueous Solvent>

The non-aqueous solvent can be selected as appropriate from a variety of known solvents, and it is preferred to use at least one selected from cyclic aprotic solvents and chain aprotic solvents.

When it is intended to increase the flash point of the solvent for improving the safety of the battery, it is preferred to use a cyclic aprotic solvent as the non-aqueous solvent.

### (Cyclic Aprotic Solvent)

Examples of the cyclic aprotic solvent that can be used include cyclic carbonates, cyclic carboxylic acid esters, cyclic sulfones, and cyclic ethers.

These cyclic aprotic solvents may be used singly, or in combination of two or more thereof.

The mixing ratio of the cyclic aprotic solvent(s) in the non-aqueous solvent is from 10% by mass to 100% by mass, more preferably from 20% by mass to 90% by mass, particularly preferably from 30% by mass to 80% by mass. By controlling the mixing ratio in this range, the conductivity of the electrolytic solution, which relates to the battery charge-discharge characteristics, can be increased.

Specific examples of the cyclic carbonates include ethylene carbonate, propylene carbonate, 1,2-butylene carbonate, 2,3-butylene carbonate, 1,2-pentylene carbonate, and 2,3-pentylene carbonate. Thereamong, ethylene carbonate and propylene carbonate, which have a high dielectric constant, can be suitably used. In the case of a battery comprising graphite as the negative electrode active material, ethylene carbonate is more preferred. These cyclic carbonates may be used in combination of two or more thereof.

Specific examples of the cyclic carboxylic acid esters include *γ*-butyrolactone, *δ*-valerolactone, and alkyl-substituted products thereof such as methyl-*γ*-butyrolactone, ethyl-*γ*-butyrolactone and ethyl-*δ*-valerolactone.

These cyclic carboxylic acid esters not only have a low vapor pressure, a low viscosity and a high dielectric constant, but also are capable of reducing the viscosity of the electrolytic solution without lowering the flash point of the electrolytic solution and the dissociation degree of the electrolyte. Accordingly, the cyclic carboxylic acid esters have a characteristic feature of being capable of increasing the conductivity of the electrolytic solution, which is an index relating to the battery discharge characteristics, without increasing the flammability of the electrolytic solution; therefore, when it is intended to improve the flash point of the solvent, it is preferred to use a cyclic carboxylic acid ester as the cyclic aprotic solvent. Among the cyclic carboxylic acid esters, *γ*-butyrolactone is most preferred.

It is also preferred to use a cyclic carboxylic acid ester in the form of a mixture with other cyclic aprotic solvent(s). For example, a mixture of a cyclic carboxylic acid ester with a cyclic carbonate and/or a chain carbonate can be used.

Examples of the cyclic sulfones include sulfolane, 2-methylsulfolane, 3-methylsulfolane, dimethylsulfone, diethylsulfone, dipropylsulfone, methylethylsulfone, and methylpropylsulfone.

Examples of the cyclic ethers include dioxolane.

### (Chain Aprotic Solvent)

Examples of a chain aprotic solvent that can be used include chain carbonates, chain carboxylic acid esters, chain ethers, and chain phosphoric acid esters.

The mixing ratio of the chain aprotic solvent in the non-aqueous solvent is from 10% by mass to 100% by mass, more preferably from 20% by mass to 90% by mass, particularly preferably from 30% by mass to 80% by mass.

Specific examples of the chain carbonates include dimethyl carbonate, methylethyl carbonate, diethyl carbonate, methylpropyl carbonate, methylisopropyl carbonate, ethylpropyl carbonate, dipropyl carbonate, methylbutyl carbonate, ethylbutyl carbonate, dibutyl carbonate, methylpentyl carbonate, ethylpentyl carbonate, dipentyl carbonate, methylheptyl carbonate, ethylheptyl carbonate, diheptyl carbonate, methylhexyl carbonate, ethylhexyl carbonate, dihexyl carbonate, methyloctyl carbonate, ethyloctyl carbonate, dioctyl carbonate, and methyltrifluoroethyl carbonate. These chain carbonates may be used in combination of two or more thereof.

Specific examples of the chain carboxylic acid esters include methyl pivalate. Specific examples of the chain ethers include dimethoxyethane.

Specific examples of the chain phosphoric acid esters include trimethyl phosphate.

### (Combination of Solvents)

In the non-aqueous electrolytic solution, the above non-aqueous solvents may be used singly, or in combination of two or more thereof.

Further, one or a plurality of only the above cyclic aprotic solvents, or one or a plurality of only the above chain aprotic solvents may be used, or the cyclic aprotic solvents and the chain aprotic solvents may be used in the form of a mixture. When it is particularly intended to improve the load characteristics and low-temperature characteristics of the battery, it is preferred to use a combination of a cyclic aprotic solvent and a chain aprotic solvent as the non-aqueous solvent.

From the standpoint of the electrochemical stability of the electrolytic solution, it is most preferred to use a cyclic carbonate as the cyclic aprotic solvent and a chain carbonate as the chain aprotic solvent. The conductivity of the electrolytic solution, which relates to the battery charge-discharge characteristics, can also be increased by using a combination of a cyclic carboxylic acid ester with a cyclic carbonate and/or a chain carbonate.

Specific examples of the combination of a cyclic carbonate and a chain carbonate include ethylene carbonate and dimethyl carbonate; ethylene carbonate and methylethyl carbonate; ethylene carbonate and diethyl carbonate; propylene carbonate and dimethyl carbonate; propylene carbonate and methylethyl carbonate; propylene carbonate and diethyl carbonate; ethylene carbonate with propylene carbonate and methylethyl carbonate; ethylene carbonate with propylene carbonate and diethyl carbonate; ethylene carbonate with dimethyl carbonate and methylethyl carbonate; ethylene carbonate with dimethyl carbonate and diethyl carbonate; ethylene carbonate with methylethyl carbonate and diethyl carbonate; ethylene carbonate with dimethyl carbonate, methylethyl carbonate and diethyl carbonate; ethylene carbonate with propylene carbonate, dimethyl carbonate and methylethyl carbonate; ethylene carbonate with propylene carbonate, dimethyl carbonate and diethyl carbonate; ethylene carbonate with propylene carbonate, methylethyl carbonate and diethyl carbonate; and ethylene carbonate with propylene carbonate, dimethyl carbonate, methylethyl carbonate and diethyl carbonate.

The mixing ratio of a cyclic carbonate and a chain carbonate (cyclic carbonate:chain carbonate) is, in terms of mass ratio, from 5:95 to 80:20, more preferably from 10:90 to 70:30, particularly preferably from 15:85 to 55:45. By controlling the mixing ratio in this range, an increase in the viscosity of the electrolytic solution can be inhibited and the dissociation degree of the electrolyte can be increased, so that the conductivity of the electrolytic solution, which relates to the battery charge-discharge characteristics, can be increased. In addition, the solubility of the electrolyte can be further increased. Accordingly, since an electrolytic solution having excellent electrical conductivity at normal temperature or at a low temperature can be obtained, the load characteristics of the battery in a low temperature to normal temperature range can be improved.

Specific examples of the combination of a cyclic carboxylic acid ester with a cyclic carbonate and/or a chain carbonate include *γ*-butyrolactone with ethylene carbonate; *γ*-butyrolactone with ethylene carbonate and dimethyl carbonate; *γ*-butyrolactone with ethylene carbonate and methylethyl carbonate; *γ*-butyrolactone with ethylene carbonate and diethyl carbonate; *γ*-butyrolactone with propylene carbonate; *γ*-butyrolactone with propylene carbonate and dimethyl carbonate; *γ*-butyrolactone with propylene carbonate and methylethyl carbonate; *γ*-butyrolactone with propylene carbonate and diethyl carbonate; *γ*-butyrolactone with ethylene carbonate and propylene carbonate; *γ*-butyrolactone with ethylene carbonate, propylene carbonate and dimethyl carbonate; *γ*-butyrolactone with ethylene carbonate, propylene carbonate and methylethyl carbonate; *γ*-butyrolactone with ethylene carbonate, propylene carbonate and diethyl carbonate; *γ*-butyrolactone with ethylene carbonate, dimethyl carbonate and methylethyl carbonate; *γ*-butyrolactone with ethylene carbonate, dimethyl carbonate and diethyl carbonate; *γ*-butyrolactone with ethylene carbonate, methylethyl carbonate and diethyl carbonate; *γ*-butyrolactone with ethylene carbonate, dimethyl carbonate, methylethyl carbonate and diethyl carbonate; *γ*-butyrolactone with ethylene carbonate, propylene carbonate, dimethyl carbonate and methylethyl carbonate; *γ*-butyrolactone with ethylene carbonate, propylene carbonate, dimethyl carbonate and diethyl carbonate; *γ*-butyrolactone with ethylene carbonate, propylene carbonate, methylethyl carbonate and diethyl carbonate; *γ*-butyrolactone with ethylene carbonate, propylene carbonate, dimethyl carbonate, methylethyl carbonate and diethyl carbonate; *γ*-butyrolactone with sulfolane; *γ*-butyrolactone with ethylene carbonate and sulfolane; *γ*-butyrolactone with propylene carbonate and sulfolane; *γ*-butyrolactone with ethylene carbonate, propylene carbonate and sulfolane; and *γ*-butyrolactone with sulfolane and dimethyl carbonate.

### (Other Solvent)

The non-aqueous electrolytic solution may contain, as the non-aqueous solvent, other solvent(s) other than the above-described solvents. Specific examples of other solvents include amides such as dimethylformamide; chain carbamates such as methyl-*N*,*N*-dimethyl carbamate; cyclic amides such as *N*-methylpyrrolidone; cyclic ureas such as *N*,*N*-dimethylimidazolidinone; boron compounds, such as trimethyl borate, triethyl borate, tributyl borate, trioctyl borate and trimethylsilyl borate; and polyethylene glycol derivatives represented by the following formulae:
HO(CH₂CH₂O)ₐH,
HO[CH₂CH(CH₃)O]_{b}H,
CH₃O(CH₂CH₂O)_{c}H,
CH₃O[CH₂CH(CH₃)O]_{d}H,
CH₃O(CH₂CH₂O)ₑCH₃,
CH₃O[CH₂CH(CH₃)O]_{f}CH₃,
C₉H₁₉PhO(CH₂CH₂O)_{g}[CH(CH₃)O]ₕCH₃ (wherein, Ph is a phenyl group), and
CH₃O[CH₂CH(CH₃)O]ᵢCO[OCH(CH₃)CH₂]ⱼOCH₃.

In the above formulae, a to f each represent an integer from 5 to 250; g to j each represent an integer from 2 to 249; 5 ≤ g + h ≤ 250; and 5 ≤ i + j ≤ 250.

### <Electrolyte>

The non-aqueous electrolytic solution may contain a variety of known electrolytes.

As an electrolyte, any electrolyte that is usually used as an electrolyte for a non-aqueous electrolytic solution can be used.

Specific examples of the electrolyte include the above-described alkali metal salts.

Specific examples of the electrolyte also include tetraalkylammonium salts such as (C₂H₅)₄NPF₆, (C₂H₅)₄NBF₄, (C₂H₅)₄NClO₄, (C₂H₅)₄NAsF₆, (C₂H₅)₄N₂SiF₆, (C₂H₅)₄NOSO₂CₖF₍₂ₖ₊₁₎ (k = an integer from 1 to 8), and (C₂H₅)₄NPFₙ[CₖF₍₂ₖ₊₁₎]₍₆₋ₙ₎ (n = 1 to 5, k = an integer from 1 to 8); lithium salts such as LiPF₆, LiBF₄, LiClO₄, LiAsF₆, Li₂SiF₆, LiOSO₂CₖF₍₂ₖ₊₁₎ (k = an integer from 1 to 8), and LiPFₙ[CₖF₍₂ₖ₊₁₎]₍₆₋ₙ₎ (n = 1 to 5, k = an integer from 1 to 8). Further, lithium salts represented by the following formulae can also be used:

LiC(SO₂R²⁷)(SO₂R²⁸)(SO₂R²⁹), LiN(SO₂OR³⁰)(SO₂OR³¹), and LiN(SO₂R³²)(SO₂R³³) (wherein, R²⁷ to R³³ may be the same or different from each other, representing a perfluoroalkyl group having from 1 to 8 carbon atoms). These electrolytes may be used singly, or in combination of two or more thereof.

Thereamong, lithium salts are particularly desirable, and LiPF₆, LiBF₄, LiOSO₂CₖF₍₂ₖ₊₁₎ (k = an integer from 1 to 8), LiClO₄, LiAsF₆, LiNSO₂[CₖF₍₂ₖ₊₁₎]₂ (k= an integer from 1 to 8) and LiPFₙ[CₖF₍₂ₖ₊₁₎]₍₆₋ₙ₎ (n = 1 to 5, and k = an integer from 1 to 8) are preferred.

The electrolyte(s) is/are contained in the non-aqueous electrolytic solution at a concentration of usually from 0.1 mol/L to 3 mol/L, preferably from 0.5 mol/L to 2 mol/L.

In cases where a cyclic carboxylic acid ester such as *γ*-butyrolactone is used in combination as the non-aqueous solvent in the non-aqueous electrolytic solution, it is particularly desired that the non-aqueous electrolytic solution contains LiPF₆. LiPF₆ has a high degree of dissociation and, therefore, not only can increase the conductivity of the electrolytic solution but also shows an action of suppressing the reductive decomposition reaction of the electrolytic solution on the negative electrode. LiPF₆ may be used singly, or in combination with other electrolyte. As such other electrolyte, any electrolyte can be used as long as it is usually used as an electrolyte for a non-aqueous electrolytic solution; however, among the above-described specific examples of lithium salts, a lithium salt other than LiPF₆ is preferred.

Specific examples include LiPF₆ and LiBF₄; LiPF₆ and LiN[SO₂CₖF₍₂ₖ₊₁₎]₂ (k = an integer from 1 to 8); LiPF₆ with LiBF₄ and LiN[SO₂CₖF₍₂ₖ₊₁₎] (k = an integer from 1 to 8).

It is desired that the ratio of LiPF₆ in the lithium salts is from 1% by mass to 100% by mass, preferably from 10% by mass to 100% by mass, more preferably from 50% by mass to 100% by mass. It is preferred that such electrolytes are contained in the non-aqueous electrolytic solution at a concentration of from 0.1 mol/L to 3 mol/L, preferably from 0.5 mol/L to 2 mol/L.

### «Preferred Modes of Lithium Secondary Battery»

As described above, the lithium secondary battery according to the first embodiment comprises: a positive electrode which contains a positive electrode active material capable of absorbing and desorbing lithium; a negative electrode which contains a negative electrode active material capable of absorbing and desorbing lithium; and a non-aqueous electrolytic solution.

A preferred mode of the lithium secondary battery according to the first embodiment is, for example, a mode in which the lithium secondary battery comprises a positive electrode plate as the positive electrode, a negative electrode plate as the negative electrode, a separator, a non-aqueous electrolytic solution, and an exterior material, wherein the positive electrode plate and the negative electrode plate face each other with the separator being sandwiched therebetween, and the battery is entirely filled with the non-aqueous electrolytic solution.

In this preferred mode, the preferred scope of the positive electrode plate and that of the negative electrode plate are as described above.

### <Separator>

In the preferred mode described above, the separator is arranged between the positive electrode plate and the negative electrode plate.

Examples of the material of the separator include (micro)porous polyethylene, (micro)porous polypropylene, TEFLON (registered trademark) films, polyamide films, polyvinyl chloride films, polyvinylidene fluoride films, polyaniline films, polyimide films, nonwoven fabrics, polyethylene terephthalate, polystyrene cellulose, and multilayer composite structures in which two or more of these polymers are used in combination. The multilayer composite structures may be coated with other resin having excellent thermal stability.

In the above-described case, a porous heat-resistant layer which contains a heat-resistant filler and a binder may exist between the negative electrode plate and the separator.

Examples of the heat-resistant filler that can be used include inorganic oxides, such as alumina, silica, titania, zirconia, magnesia and yttria; ceramics; and glass. These may be used singly, or in combination of two or more thereof.

As the binder, any of the aqueous and non-aqueous binders described above for the method of forming a composite layer can be used; however, it is preferred to use a non-aqueous binder such as polyvinylidene fluoride. It is preferred that the binder is used in an amount of from 0.5 parts by mass to 20 parts by mass (in terms of solid content) with respect to 100 parts by mass of the heat-resistant filler.

### <Exterior Material>

In the preferred mode described above, an exterior material is used.

The exterior material is preferably a metal can, for example, a can made of iron, stainless steel, aluminum or the like.

Alternatively, as the exterior material, a film bag produced by disposing a resin on an ultrathin aluminum may be used.

The exterior material may take any shape, such as a cylindrical shape, a rectangular shape, a thin shape or a coin shape.

The lithium secondary battery according to the first embodiment can be formed in a variety of known shapes, such as a cylindrical shape, a coin shape, a rectangular shape, a film shape and other arbitrary shapes.

However, the battery has the same basic structure regardless of its shape, and design modifications can be made in accordance with the purpose.

One example of the lithium secondary battery according to the first embodiment is a coin-type battery shown in FIG. 1.

In the coin-type battery shown in FIG. 1, a disc-shaped negative electrode 2, a separator 5 into which a non-aqueous electrolytic solution is injected, a disc-shaped positive electrode 1 and, as required, spacer plates 7 and 8 made of stainless steel, aluminum or the like, which are layered in this order, are accommodated between a positive electrode can 3 (hereinafter, also referred to as "battery can") and a sealing plate 4 (hereinafter, also referred to as "battery can lid"). The positive electrode can 3 and the sealing plate 4 are sealed by caulking via a gasket 6.

In this example, as the non-aqueous electrolytic solution injected into the separator 5, the non-aqueous electrolytic solution according to the first embodiment can be used.

The lithium secondary battery according to the first embodiment may be a lithium secondary battery obtained by charging and discharging a lithium secondary battery (a lithium secondary battery that has not been charged or discharged) which contains a negative electrode, a positive electrode and a non-aqueous electrolytic solution.

That is, the lithium secondary battery according to the first embodiment may be a lithium secondary battery (a charged and discharged lithium secondary battery) obtained by first preparing a lithium secondary battery which contains a negative electrode, a positive electrode and a non-aqueous electrolytic solution and has not been charged or discharged and subsequently charging and discharging the thus obtained lithium secondary battery at least once.

### [Second Embodiment]

The lithium secondary battery according to the second embodiment is a lithium secondary battery comprising: a positive electrode which contains a positive electrode active material capable of absorbing and desorbing lithium; a negative electrode; and a non-aqueous electrolytic solution, wherein at least one of the positive electrode or the negative electrode contains a polymer that is a reaction product of at least one compound (A), which is selected from the group consisting of an amine compound, an amide compound, an imide compound, a maleimide compound and an imine compound, and a compound (B) which has two or more carbonyl groups in one molecule and is different from the compound (A), and the non-aqueous electrolytic solution contains an additive (X) which is a carbonate compound having a carbon-carbon unsaturated bond.

Conventionally, a cyclic carbonate additive having an unsaturated bond structure is incorporated into a non-aqueous electrolytic solution (see Patent Documents 1 and 2). However, according to the studies conducted by the present inventors, incorporation of a cyclic carbonate additive having an unsaturated bond structure into the non-aqueous electrolytic solution is sometimes accompanied by an increase in the battery resistance.

In addition, when the nitrogen-containing polymer described in Patent Document 9 was added to the positive electrode, a reduction in the capacity retention ratio was observed in some cases.

In these respects, according to the lithium secondary battery of the second embodiment, the discharge capacity retention ratio after repeated charging and discharging is improved, and an increase in the battery resistance is suppressed.

The lithium secondary battery according to the second embodiment and the lithium secondary battery according to the first embodiment are the same except for the following point, and their preferred scopes are also the same.

That is, the lithium secondary battery according to the second embodiment is different from the lithium secondary battery according to the first embodiment in that the additive (X) is a carbonate compound having a carbon-carbon unsaturated bond in the former, whereas the additive (X) is not restricted to a carbonate compound having a carbon-carbon unsaturated bond in the latter.

The preferred scope of the carbonate compound having a carbon-carbon unsaturated bond in the second embodiment is the same as that of the carbonate compound having a carbon-carbon unsaturated bond in the first embodiment.

### [Third Embodiment]

The lithium secondary battery according to the third embodiment is a lithium secondary battery comprising: a positive electrode which contains a positive electrode active material capable of absorbing and desorbing lithium; a negative electrode; and a non-aqueous electrolytic solution, wherein at least one of the positive electrode or the negative electrode contains a polymer that is a reaction product of at least one compound (A), which is selected from the group consisting of an amine compound, an amide compound, an imide compound, a maleimide compound and an imine compound, and a compound (B) which has two or more carbonyl groups in one molecule and is different from the compound (A), and the non-aqueous electrolytic solution contains an additive (X) which is a carbonate compound having a halogen atom and not having a carbon-carbon unsaturated bond.

According to the lithium secondary battery of the third embodiment, an increase in the battery resistance (particularly an increase in the battery resistance caused by repeated charging and discharging (including trickle charging)) is suppressed. Further, an effect of improving the discharge capacity retention ratio after repeated charging and discharging is also expected to be obtained.

The lithium secondary battery according to the third embodiment and the lithium secondary battery according to the first embodiment are the same except for the following point, and their preferred scopes are also the same.

That is, the lithium secondary battery according to the third embodiment is different from the lithium secondary battery according to the first embodiment in that the additive (X) is a carbonate compound having a halogen atom and not having a carbon-carbon unsaturated bond in the former, whereas the additive (X) is not restricted to a carbonate compound having a halogen atom and not having a carbon-carbon unsaturated bond in the latter.

The preferred scope of the carbonate compound having a halogen atom and not having a carbon-carbon unsaturated bond in the third embodiment is the same as that of the carbonate compound having a halogen atom and not having a carbon-carbon unsaturated bond in the first embodiment.

### [Fourth Embodiment]

The lithium secondary battery according to the fourth embodiment is a lithium secondary battery comprising: a positive electrode which contains a positive electrode active material capable of absorbing and desorbing lithium; a negative electrode which contains a negative electrode active material capable of absorbing and desorbing lithium; and a non-aqueous electrolytic solution, wherein at least one of the positive electrode or the negative electrode contains a polymer that is a reaction product of at least one compound (A), which is selected from the group consisting of an amine compound, an amide compound, an imide compound, a maleimide compound and an imine compound, and a compound (B) which has two or more carbonyl groups in one molecule and is different from the compound (A), and the non-aqueous electrolytic solution contains an additive (X) which is at least one alkali metal salt selected from the group consisting of a monofluorophosphate salt, a difluorophosphate salt, an oxalato salt, a sulfonate salt, a carboxylate salt, an imide salt and a methide salt.

According to the lithium secondary battery of the fourth embodiment, a change in the battery resistance caused by trickle charging is suppressed, and the capacity retention ratio after trickle charging is improved.

The lithium secondary battery according to the fourth embodiment and the lithium secondary battery according to the first embodiment are the same except for the following point, and their preferred scopes are also the same.

That is, the lithium secondary battery according to the fourth embodiment is different from the lithium secondary battery according to the first embodiment in that the additive (X) is, in the former, at least one alkali metal salt selected from the group consisting of a monofluorophosphate salt, a difluorophosphate salt, an oxalato salt, a sulfonate salt, a carboxylate salt, an imide salt and a methide salt, whereas the additive (X) is not restricted to such an alkali metal salt in the latter. The preferred scope of the alkali metal salt in the fourth embodiment is the same as that of the alkali metal salt in the first embodiment.

### [Fifth Embodiment]

The lithium secondary battery according to the fifth embodiment is a lithium secondary battery comprising: a positive electrode which contains a positive electrode active material capable of absorbing and desorbing lithium; a negative electrode which contains a negative electrode active material capable of absorbing and desorbing lithium; and a non-aqueous electrolytic solution, wherein at least one of the positive electrode or the negative electrode contains a polymer that is a reaction product of at least one compound (A), which is selected from the group consisting of an amine compound, an amide compound, an imide compound, a maleimide compound and an imine compound, and a compound (B) which has two or more carbonyl groups in one molecule and is different from the compound (A), and the non-aqueous electrolytic solution contains an additive (X) which is at least one compound selected from the group consisting of a sulfonic acid ester compound and a sulfuric acid ester compound.

According to the lithium secondary battery of the fifth embodiment, a change in the battery resistance caused by trickle charging is suppressed, and the capacity retention ratio after trickle charging is improved.

The lithium secondary battery according to the fifth embodiment and the lithium secondary battery according to the first embodiment are the same except for the following point, and their preferred scopes are also the same.

That is, the lithium secondary battery according to the fifth embodiment is different from the lithium secondary battery according to the first embodiment in that the additive (X) is, in the former, at least one compound selected from the group consisting of a sulfonic acid ester compound and a sulfuric acid ester compound, whereas the additive (X) is not restricted to such a compound in the latter. The preferred scope of the sulfonic acid ester compound and that of the sulfuric acid ester compound in the fifth embodiment are respectively the same as those in the first embodiment.

### [Sixth Embodiment]

The lithium secondary battery according to the sixth embodiment is a lithium secondary battery comprising: a positive electrode which contains a positive electrode active material capable of absorbing and desorbing lithium; a negative electrode which contains a negative electrode active material capable of absorbing and desorbing lithium; and a non-aqueous electrolytic solution, wherein at least one of the positive electrode or the negative electrode contains a polymer that is a reaction product of at least one compound (A), which is selected from the group consisting of an amine compound, an amide compound, an imide compound, a maleimide compound and an imine compound, and a compound (B) which has two or more carbonyl groups in one molecule and is different from the compound (A), and the non-aqueous electrolytic solution contains an additive (X) which is a nitrile compound.

According to the lithium secondary battery of the sixth embodiment, the battery capacity retention ratio is improved. For example, even after charge-discharge cycles, the discharge capacity is not reduced and the initial capacity is maintained.

The lithium secondary battery according to the sixth embodiment and the lithium secondary battery according to the first embodiment are the same except for the following point, and their preferred scopes are also the same.

That is, the lithium secondary battery according to the sixth embodiment is different from the lithium secondary battery according to the first embodiment in that the additive (X) is a nitrile compound in the former, whereas the additive (X) is not restricted to a nitrile compound in the latter. The preferred scope of the nitrile compound in the sixth embodiment is the same as that of the nitrile compound in the first embodiment.

### [Seventh Embodiment]

The lithium secondary battery according to the seventh embodiment is a lithium secondary battery comprising: a positive electrode which contains a positive electrode active material capable of absorbing and desorbing lithium; a negative electrode which contains a negative electrode active material capable of absorbing and desorbing lithium; and a non-aqueous electrolytic solution, wherein at least one of the positive electrode or the negative electrode contains a polymer that is a reaction product of at least one compound (A), which is selected from the group consisting of an amine compound, an amide compound, an imide compound, a maleimide compound and an imine compound, and a compound (B) which has two or more carbonyl groups in one molecule and is different from the compound (A), and the non-aqueous electrolytic solution contains an additive (X) which is a dioxane compound.

According to the lithium secondary battery of the seventh embodiment, an increase in the battery resistance (particularly an increase in the battery resistance caused by repeated charging and discharging (including trickle charging)) is suppressed. Further, an effect of improving the discharge capacity retention ratio after repeated charging and discharging is also expected to be obtained.

The lithium secondary battery according to the seventh embodiment and the lithium secondary battery according to the first embodiment are the same except for the following point, and their preferred scopes are also the same.

That is, the lithium secondary battery according to the seventh embodiment is different from the lithium secondary battery according to the first embodiment in that the additive (X) is a dioxane compound in the former, whereas the additive (X) is not restricted to a dioxane compound in the latter. The preferred scope of the dioxane compound in the seventh embodiment is the same as that of the dioxane compound in the first embodiment.

### [Eighth Embodiment]

The lithium secondary battery according to the eighth embodiment is a lithium secondary battery comprising: a positive electrode which contains a positive electrode active material capable of absorbing and desorbing lithium; a negative electrode which contains a negative electrode active material capable of absorbing and desorbing lithium; and a non-aqueous electrolytic solution, wherein at least one of the positive electrode or the negative electrode contains a polymer that is a reaction product of at least one compound (A), which is selected from the group consisting of an amine compound, an amide compound, an imide compound, a maleimide compound and an imine compound, and a compound (B) which has two or more carbonyl groups in one molecule and is different from the compound (A), and the non-aqueous electrolytic solution contains an additive (X) which is an aromatic hydrocarbon compound substituted with at least one substituent selected from the group consisting of a halogen atom, an alkyl group, a halogenated alkyl group, an alkoxy group, a halogenated alkoxy group, an aryl group and a halogenated aryl group.

According to the lithium secondary battery of the eighth embodiment, the battery capacity retention ratio is improved. For example, even after charge-discharge cycles, the discharge capacity is not reduced and the initial capacity is maintained.

The lithium secondary battery according to the eighth embodiment and the lithium secondary battery according to the first embodiment are the same except for the following point, and their preferred scopes are also the same.

That is, the lithium secondary battery according to the eighth embodiment is different from the lithium secondary battery according to the first embodiment in that the additive (X) is the above-described aromatic hydrocarbon compound in the former, whereas the additive (X) is not restricted to the aromatic hydrocarbon compound in the latter. The preferred scope of the aromatic hydrocarbon compound in the eighth embodiment is the same as that of the specific aromatic hydrocarbon compound in the first embodiment.

### [Ninth Embodiment]

The lithium secondary battery according to the ninth embodiment is a lithium secondary battery comprising: a positive electrode which contains a positive electrode active material capable of absorbing and desorbing lithium; a negative electrode which contains a negative electrode active material capable of absorbing and desorbing lithium; and a non-aqueous electrolytic solution,
wherein the ratio of the battery resistance R1 at 150°C with respect to the battery resistance R0 at 30°C (R1/R0) is 3.8 or higher, and
the non-aqueous electrolytic solution contains an additive (X) which is at least one compound selected from the group consisting of:
a carbonate compound having a carbon-carbon unsaturated bond;
a carbonate compound having a halogen atom and not having a carbon-carbon unsaturated bond;
an alkali metal salt;
a sulfonic acid ester compound;
a sulfuric acid ester compound;
a nitrile compound;
a dioxane compound; and
an aromatic hydrocarbon compound substituted with at least one substituent selected from the group consisting of a halogen atom, an alkyl group, a halogenated alkyl group, an alkoxy group, a halogenated alkoxy group, an aryl group and a halogenated aryl group.

According to the lithium secondary battery of the ninth embodiment, the discharge capacity retention ratio after repeated charging and discharging is improved, and an increase in the battery resistance is suppressed.

The reasons for this are not necessarily clear; however, they are speculated as follows.

The ratio (R1/R0) of 3.8 or higher, that is, an increase in the resistance of a lithium secondary battery associated with an increase in the temperature of the lithium secondary battery, means that protective films covering each active material are effectively formed during repeated charging and discharging.

Further, as described above in relation to the first embodiment, the additive (X) has an action of forming protective films covering the surface of each active material in the early stage of charging and discharging.

Therefore, it is believed that, the discharge capacity retention ratio after repeated charging and discharging is improved and an increase in the battery resistance is suppressed by a combination of the feature that the ratio (R1/R0) is 3.8 or higher and the feature that the non-aqueous electrolytic solution contains the additive (X).

The preferred scope of the ratio (R1/R0) in the ninth embodiment is the same as that of the ratio (R1/R0) in the first embodiment.

In the ninth embodiment, as a means for achieving that "the ratio (R1/R0) is 3.8 or higher", the means for incorporating the specific polymer into at least one of the positive electrode or the negative electrode, which is described above for the first embodiment, is particularly effective.

However, in the ninth embodiment, the above-described means is not restricted, and it is also possible to employ a means for incorporating a substance other than the specific polymer, which reacts with the active materials during repeated charging and discharging, into at least one of the positive electrode or the negative electrode.

As described above, the lithium secondary battery according to the ninth embodiment is not restricted to that at least one of the positive electrode or the negative electrode contains the specific polymer.

The lithium secondary battery according to the ninth embodiment and the lithium secondary battery according to the first embodiment are the same except for this point, and their preferred scopes are also the same.

### EXAMPLES

The invention will now be described more concretely by way of Examples and Comparative Examples thereof; however, the invention is not restricted thereto.

In the following Examples, unless otherwise specified, "part(s)" denotes "part(s) by mass" and "wt%" denotes "% by mass".

Further, in the following Examples, the term "added amount" indicates the content in the eventually obtained non-aqueous electrolytic solution (that is, the amount with respect to the total amount of the eventually obtained non-aqueous electrolytic solution).

### [Preparation of Polymer P1 Solution]

First, an NMP solution of polymer P1 was prepared as a polymer P1 solution.

It is noted here that the polymer P1 is one example of the above-described specific polymer (a polymer that is a reaction product of at least one compound (A), which is selected from the group consisting of an amine compound, an amide compound, an imide compound, a maleimide compound and an imine compound, and a compound (B) which has two or more carbonyl groups in one molecule and is different from the compound (A)).

A 190 mL-capacity SUS autoclave and a stir bar were thoroughly dried.

To a container of the thus dried autoclave, 5.82 g of *N*,*N*-diphenylmethane bismaleimide (manufactured by Daiwa Kasei Industry Co., Ltd.) (hereinafter, also simply referred to as "maleimide") and 1.01 g of barbituric acid (manufactured by Ruicheng County XINYU chemical plant Co., Ltd.) were added (maleimide:barbituric acid = 2:1 (mol)).

It is noted here that *N*,*N*'-diphenylmethane bismaleimide is a compound represented by the above-described Formula (1), wherein all of R¹s, R²s and R³s are hydrogen atoms; X is -CH₂-; and n is 1.

Further, barbituric acid is a compound represented by the above-described Formula (5), wherein R⁵ and R⁶ are both hydrogen atoms.

Next, 129.82 g of *N*-methyl-2-pyrrolidone (NMP) was added to the container (containing maleimide and barbituric acid), and the container was tightly sealed (total mass = 136.65 g, solid concentration = 5% by mass, 70% by volume of the container was occupied). Operations of introducing nitrogen gas to the sealed container until the inner pressure of the container reached 5.0 MPa and subsequently bringing the inner pressure of the container back to normal pressure were repeated 5 times. By this, the container was purged with nitrogen.

Then, the autoclave was placed on a heating block and heated to an inner temperature of 100°C while stirring the contents at 120 rpm.

With the point at which the inner temperature reached 100°C being defined as the reaction starting point, the stirring was continued for 24 hours while maintaining the inner temperature at 100°C (hereinafter, this operation is referred to as "reaction"). After the completion of the reaction, the container was cooled, whereby a brown polymer P1 solution was obtained.

### [HPLC Analysis of Polymer P1 Solution]

The thus obtained polymer P1 solution was analyzed by high-performance liquid chromatography (HPLC) of a water (0.1 %-by-mass aqueous phosphoric acid solution)/acetonitrile mixed system.

As a HPLC sample solution, a solution obtained by diluting a mixture of 600 mg of the polymer P1 solution and 800 mg of an internal standard substance diluted solution (*N*-phenylsuccinimide/acetonitrile = 10 mg/g) with acetonitrile to a volume of 50 mL was used.

As a HPLC column, "ATLANTIS T3" (5 µm, 4.6 × 250 mm) manufactured by Nihon Waters K.K. was used.

As HPLC eluents, 0.1%-by-mass aqueous phosphoric acid solution (hereinafter, simply referred to as "water") and acetonitrile were used.

As for HPLC gradient conditions, the volume ratio (water/acetonitrile) was continuously changed from 99/1 to 20/80 over a period of 25 minutes and the volume ratio (water/acetonitrile) was maintained at 20/80 for 10 minutes, after which the volume ratio (water/acetonitrile) was changed from 20/80 to 99/1 over a period of 3 minutes.

As a detector, a UV detector manufactured by Shimadzu Corporation was used. The detection wavelength was set at 210 nm until 4.83 minutes after the initiation of the analysis and at 230 nm thereafter.

As a result of the HPLC analysis, maleimide (25.5 min), barbituric acid (4.6 min) and the internal standard substance (17.2 min) were detected. As a result of quantification, the conversion ratio was found to be 94 mol% for maleimide and 99 mol% for barbituric acid. Accordingly, it was confirmed that, in the above-described production of the polymer P1 solution, a polymer P1 was produced as a reaction product of maleimide and barbituric acid.

### [Weight-Average Molecular Weight (Mw) and Molecular Weight Distribution (Mw/Mn) of Polymer P1]

Using gel permeation chromatography (GPC), the weight-average molecular weight (Mw) and molecular weight distribution (Mw/Mn) of the polymer P1 were determined as follows.

First, the polymer P1 solution (600 mg) was diluted with a developing solvent to prepare a sample solution (3 g) having a polymer P1 concentration of 10 mg/mL.

Then, 0.1 mL of the thus obtained sample solution was introduced to columns with a solvent (dimethylformamide (30 mM lithium bromide and 1 wt% of phosphoric acid)) at a temperature of 40°C and a flow rate of 0.8 mL/min, and the sample concentration in the sample solution separated by the columns was measured using a differential refractometer. Separately, a universal calibration curve was created using a PEG/PEO standard sample and, based on this universal calibration curve and the result of measuring the sample concentration, the weight-average molecular weight (Mw) and molecular weight distribution (Mw/Mn) of the polymer P1 were calculated.

As a GPC analyzer and its columns, the followings were used.

### -GPC Analyzer-

GPC manufactured by Shimadzu Science East Co.

### -Columns-

PL gel 5 µm MIXED-D 300 × 7.5 mm and PL gel 5 µm MIXED-C 300 × 7.5 mm (both columns were manufactured by Agilent Technologies, Ltd.)

As a result of the above measurement, the polymer P1 was found to have a Mw of from 17,000 to 23,000 and a Mw/Mn of from 10 to 70.

### [Hydrogenation Experiment of Polymer P1]

To a 70-mL autoclave container, a palladium catalyst (E1533 xRSA/W 5%Pd, manufactured by N.E. Chemcat Corporation) (107.7 mg) and the polymer P1 solution (10.183 g) were added, and the container was tightly sealed. The inside of the sealed container was pressurized with nitrogen gas to a pressure of 0.3 MPa, and the absence of gas leak was confirmed.

Next, the container was purged with hydrogen gas and further pressurized with hydrogen gas to a pressure of 0.248 MPa. In this state, the contents of the container were stirred while immersing the container in a 20°C water bath (hydrogenation reaction). After continuing the stirring for 1 hour, the point at which the pressure change in the container was confirmed to be stabilized was defined as the end point of the hydrogenation reaction. The pressure at this point (at the end point of the hydrogenation reaction) was 0.232 MPa, and the pressure change from the initiation of the stirring was thus 0.016 MPa.

From this pressure change, the hydrogen consumption in the hydrogenation reaction was calculated to be 0.39 mmol.

From the above results, it was confirmed that the polymer P1 had reactive double bonds.

### -Control Experiment-

A control solution A was prepared in the same manner as the polymer P1 solution, except that *N*,*N*'-diphenylmethane bismaleimide was not used.

In addition, a control solution B was prepared in the same manner as the polymer P1 solution, except that barbituric acid was not used.

The control solution A and the control solution B were each subjected to the same hydrogenation experiment as that of the polymer P1.

As a result, the hydrogen consumption in the hydrogenation reaction was found to be 0 mmol for the control solution A and 2.4 mmol for the control solution B.

From the above-described results of hydrogenation experiments, it was found that the reactive double bonds existing in the polymer P1 were derived from *N*,*N*'-diphenylmethane bismaleimide.

In addition, it was found that the amount of the reactive double bonds in the polymer P1 was 16 mol% [equation: (0.39 mmol/2.4 mmol) × 100] with respect to the amount of reactive double bonds in *N*,*N*'-diphenylmethane bismaleimide used as a raw material of the polymer P1.

From the above-described results, it was found that the reactions yielding the polymer P1 included reactions between the reactive double bonds of maleimide and barbituric acid.

Moreover, it was found that, in the reactions yielding the polymer P1, some of the reactive double bonds of maleimide used as a raw material underwent reaction and the rest of the reactive double bonds remained in the polymer P1.

### [Example 1-1]

A coin-type lithium secondary battery (coin-type battery) was produced by the following procedures.

### <Preparation of Negative Electrode>

A paste-form negative electrode composite slurry was prepared by kneading 100 parts of artificial graphite, 1.1 parts of carboxymethyl cellulose and 1.5 parts of an SBR latex in water solvent.

Then, this negative electrode composite slurry was coated and dried on a negative electrode current collector made of a 18 µm-thick strip-form copper foil, and the resultant was subsequently compressed using a roll press to obtain a sheet-form negative electrode composed of the negative electrode current collector and a negative electrode active material layer. In this process, the coating density and the filling density of the negative electrode active material layer were 10.8 mg/cm² and 1.3 g/mL, respectively.

### <Preparation of Positive Electrode>

A paste-form positive electrode composite slurry was prepared by kneading LiNi_{0.5}Mn_{0.3}Co_{0.2}O₂ (90 parts), acetylene black SP (4 parts), graphite KS6 (2 parts), polyvinylidene fluoride (2 parts), the polymer P1 solution (in an amount corresponding to a solid content of 0.5 parts) and *N*-methylpyrrolidone as a solvent.

Then, this positive electrode composite slurry was coated and dried on a positive electrode current collector made of a 20 µm-thick strip-form aluminum foil, and the resultant was subsequently compressed using a roll press to obtain a sheet-form positive electrode composed of the positive electrode current collector and a positive electrode active material layer. In this process, the coating density and the filling density of the positive electrode active material layer were 20 mg/cm² and 2.9 g/mL, respectively.

### <Preparation of Non-aqueous Electrolytic Solution>

A mixed solvent was obtained by mixing ethylene carbonate (EC) and methylethyl carbonate (EMC) as non-aqueous solvents at a ratio of 30:70 (volume ratio).

In the thus obtained mixed solvent, an electrolyte LiPF₆ was dissolved such that the concentration of the electrolyte in a non-aqueous electrolytic solution to be eventually obtained would be 1 mol/L.

To the resulting solution, vinylene carbonate (hereinafter, referred to as "VC") was added as the additive (X) (added amount = 2 wt%), whereby a non-aqueous electrolytic solution was obtained.

VC is one example of the carbonate compound having a carbon-carbon unsaturated bond that is used as the additive (X).

### <Production of Coin-type Battery>

The above-prepared negative electrode and positive electrode were punched out in the form of discs having a diameter of 14.5 mm and 13 mm, respectively, to obtain coin-shaped electrodes (negative electrode and positive electrode). In addition, a 20 µm-thick microporous polyethylene film was punched out in the form of a disc having a diameter of 16 mm to obtain a separator.

The thus obtained coin-shaped negative electrode, separator and coin-shaped positive electrode were layered in this order inside a stainless-steel battery can (size 2032), and 40 µL of the above-prepared non-aqueous electrolytic solution was injected therein to impregnate the separator, the positive electrode and the negative electrode with the non-aqueous electrolytic solution.

Further, an aluminum plate (thickness: 1.2 mm, diameter: 16 mm) and a spring were placed on the positive electrode, and a battery can lid was caulked via a polypropylene gasket to tightly seal the resulting battery, whereby a coin-type battery (coin-type lithium secondary battery) having the configuration shown in FIG. 1 with a diameter of 20 mm and a height of 3.2 mm was produced.

### [Comparative Example 1-1]

A coin-type battery was produced in the same manner as in Example 1-1, except that the polymer P1 solution was not used in the preparation of the positive electrode.

### [Comparative Example 1-2]

A coin-type battery was produced in the same manner as in Example 1-1, except that VC was not used in the preparation of the non-aqueous electrolytic solution.

### [Comparative Example 1-3]

A coin-type battery was produced in the same manner as in Example 1-1, except that the polymer P1 solution was not used in the preparation of the positive electrode and VC was not used in the preparation of the non-aqueous electrolytic solution.

### [Evaluations at Voltage of 4.2 V]

The coin-type batteries produced in Example 1-1 and Comparative Examples 1-1 to 1-3 were each evaluated at a voltage of 4.2 V. The details thereof are described below.

The evaluations at a voltage of 4.2 V were performed using ASKA charge-discharge system (ACD-M01A; ASKA Electronic Co., Ltd., Japan), Bio-Logic standard potentiostat/galvanostat (VMP3, VSP, SP-150; Hokuto Denko Corp., Japan) and a thermostatic chamber (LU-113; ESPEC Corp., Japan).

### <Conditioning and Confirmation of Capacity before Trickle Charging>

In the thermostatic chamber (25°C), each coin-type battery was subjected to 4 cycles of a process of being charged to 4.2 V under a CC-CV condition at a current of 0.2 C and subsequently CC-discharged at a current of 0.2 C (conditioning). The discharge capacity in the fourth cycle was checked and defined as the capacity before trickle charging.

The results thereof are shown in Table 1.

The capacity before trickle charging was used as a reference value of the below-described capacity retention ratio.

The terms "CC", "CV" and "CC-CV" used herein denote "Constant Current", "Constant Voltage" and "Constant Current-Constant Voltage", respectively (the same applies below).

### <Trickle Charging>

Each coin-type battery whose capacity before trickle charging had been checked was subjected to trickle charging at 55°C. Trickle charging is, as described above, an operation of continuously charging a secondary battery with a microcurrent for the purpose of compensating the self-discharge of the secondary battery.

In more detail, for each coin-type battery whose capacity before trickle charging had been checked, trickle charging was performed for 7 days in the thermostatic chamber (55°C) under a CC-CV condition at a current of 0.2 C and a voltage of 4.2 V.

### <Confirmation of Residual Capacity after Trickle Charging>

Each coin-type battery subjected to the 7-day trickle charging was CC-discharged at 25°C and a current of 0.2 C. The discharge capacity at this point was checked as the residual capacity after trickle charging. The results thereof are shown in Table 1.

### <Residual Capacity Retention Ratio>

A value obtained by dividing the residual capacity after trickle charging by the reference value (the capacity before trickle charging, that is, the discharge capacity in the fourth cycle at a current of 0.2 C) and then multiplying the quotient by 100 was defined as the residual capacity retention ratio (%). The results thereof are shown in Table 1.

**[Table 1]**

| | Coin-type battery | | Evaluation results at voltage of 4.2 V | | |
|---|---|---|---|---|---|
| | Polymer P1 | VC | Capacity before trickle charging [mAhg⁻¹] | Residual capacity after trickle charging [mAhg⁻¹] | Residual capacity retention ratio [%] |
| Example 1-1 | present | present | 162.3 | 151.8 | 93.5 |
| Comparative Example 1-1 | absent | present | 159.8 | 147.4 | 92.2 |
| Comparative Example 1-2 | present | absent | 162.5 | 145.9 | 89.7 |
| Comparative Example 1-3 | absent | absent | 160.1 | 147.8 | 92.3 |

As shown in Table 1, the coin-type battery of Example 1-1 containing both the polymer P1 and VC exhibited a high capacity retention ratio of 93.5%.

In contrast, it is seen that the capacity retention ratio was reduced in the coin-type batteries of Comparative Examples 1-1 to 1-3 which did not contain either or both of the polymer P1 and VC.

Between Example 1-1 and Comparative Examples, a difference of 1% or larger in the capacity retention ratio was found after the 7-day trickle charging. Thus, even a larger difference in the capacity retention ratio is expected to be found after the lapse of time under actual use. Therefore, it is seen that the combination of the polymer P1 and VC greatly contributes to an improvement in the battery service life.

### [Evaluations at Voltage of 4.3 V]

The coin-type batteries produced in Example 1-1 and Comparative Examples 1-1 to 1-3 were each evaluated at a voltage of 4.3 V. The details thereof are described below.

It is noted here, however, that the below-described evaluations relating to the direct-current resistance were performed only for the coin-type batteries produced in Example 1-1, Comparative Example 1-1 and Comparative Example 1-3.

The evaluations at a voltage of 4.3 V were performed using the same apparatuses as those used in the evaluations at a voltage of 4.2 V.

### <Conditioning and Confirmation of Capacity Retention Ratio before Trickle Charging>

In the thermostatic chamber (25°C), each coin-type battery was subjected to 4 cycles of a process of being charged to 4.3 V under a CC-CV condition at a current of 0.2 C and subsequently CC-discharged at a current of 0.2 C (conditioning). The discharge capacity in the fourth cycle was used as a reference value of the respective capacity retention ratios described below.

Then, each coin-type battery was charged to 4.3 V under a CC-CV condition at a current of 0.2 C and subsequently CC-discharged at a current of 1 C, after which each coin-type battery was again charged to 4.3 V under a CC-CV condition at a current of 0.2 C and subsequently CC-discharged at a current of 2 C.

A value obtained by dividing the discharge capacity at the time of the CC-discharging at a current of 1 C by the "discharge capacity in the fourth cycle" and then multiplying the quotient by 100 was defined as the capacity retention ratio at 1 C before trickle charging (%). The results thereof are shown in Table 2.

Further, a value obtained by dividing the discharge capacity at the time of the CC-discharging at a current of 2 C by the "discharge capacity in the fourth cycle" and then multiplying the quotient by 100 was defined as the capacity retention ratio at 2 C before trickle charging (%). The results thereof are shown in Table 2.

In Table 2, for comparison purposes, a value obtained by dividing the "discharge capacity in the fourth cycle" (reference value) by the "discharge capacity in the fourth cycle" (reference value) and then multiplying the quotient by 100 (that is, 100%) is also shown as the capacity retention ratio at 0.2 C before trickle charging (%).

### <Measurement of Direct-Current Resistance before Trickle Charging>

Next, the direct-current resistance before trickle charging was measured for each coin-type battery whose capacity retention ratio before trickle charging had been checked. The following operations were performed inside the thermostatic chamber (25°C).

In more detail, first, the SOC (State of Charge) of each coin-type battery whose capacity retention ratio before trickle charging had been checked was adjusted to 50%.

Next, each coin-type battery thus adjusted to have an SOC of 50% was subjected to CC10s discharging at a current of 0.2 C, CC-CV10s charging at a current of 0.2 C, CC10s discharging at a current of 1 C, CC-CV10s charging at a current of 1 C, CC10s discharging at a current of 2 C, CC-CV10s charging at a current of 2 C, CC10s discharging at a current of 5 C and CC-CV10s charging at a current of 5 C in this order. The terms "CC10s discharging" and "CC-CV10s charging" used herein mean 10-second CC discharging and 10-second CC-CV charging, respectively (the same applies below).

Then, the relationship between the current in each charging current described above (hereinafter, also referred to as "resting current") and the voltage at the 10th second after the initiation of discharging (hereinafter, also referred to as "resting voltage") was plotted, and the direct-current resistance was determined from the slope of a straight line obtained from the 4 plotted points.

The thus obtained results are shown in Table 3.

### <Trickle Charging>

For each coin-type battery whose capacity before trickle charging had been checked, trickle charging was performed for 7 days in the thermostatic chamber (55°C) under a CC-CV condition at a current of 0.2 C and a voltage of 4.3 V.

### <Confirmation of Capacity Retention Ratio after Trickle Charging>

Each coin-type battery subjected to the 7-day trickle charging was CC-discharged at 25°C and a current of 0.2 C. The discharge capacity at this point was checked as the residual capacity after trickle charging. Then, each coin-type battery was charged to 4.3 V under a CC-CV condition at a current of 0.2 C and subsequently CC-discharged at a current of 1 C, after which each coin-type battery was again charged to 4.3 V under a CC-CV condition at a current of 0.2 C and subsequently CC-discharged at a current of 2 C.

A value obtained by dividing the residual capacity after trickle charging by the reference value (the discharge capacity in the fourth cycle at 0.2 C) and then multiplying the quotient by 100 was defined as the residual capacity retention ratio after trickle charging (%).

A value obtained by dividing the discharge capacity at a current of 1 C by the reference value and then multiplying the quotient by 100 was defined as the capacity retention ratio at 1 C after trickle charging (%).

A value obtained by dividing the discharge capacity at a current of 2 C by the reference value and then multiplying the quotient by 100 was defined as the capacity retention ratio at 2 C after trickle charging (%).

The thus obtained results are shown in Table 2.

### <Measurement of Direct-Current Resistance after Trickle Charging>

For each coin-type battery whose capacity retention ratio after trickle charging had been checked, the direct-current resistance after trickle charging was measured in the same manner as in the measurement of the direct-current resistance before trickle charging.

The results thereof are shown in Table 3.

### <Rate of Change in Direct-Current Resistance Caused by Trickle Charging>

The rate of change in the direct-current resistance caused by trickle charging was determined using the following equation. The results thereof are shown in Table 3.

Rate of change in direct-current resistance (%) = ((Direct-current resistance after trickle charging - Direct-current resistance before trickle charging)/Direct-current resistance before trickle charging) × 100

**[Table 2]**

| | Coin-type battery | | Evaluation results at voltage of 4.3 V | | | | | |
|---|---|---|---|---|---|---|---|---|
| | Polymer P1 | VC | Capacity retention ratio (%) | | | | | |
| | | | before trickle charging | | | after trickle charging | | |
| | | | 0.2 C | 1 C | 2 C | residual | 1 C | 2 C |
| Example 1-1 | present | present | 100 | 93 | 78 | 96 | 88 | 48 |
| Comparative Example 1-1 | absent | present | 100 | 93 | 83 | 94 | 83 | 43 |
| Comparative Example 1-2 | present | absent | 100 | 93 | 76 | 95 | 78 | 48 |
| Comparative Example 1-3 | absent | absent | 100 | 93 | 81 | 95 | 75 | 40 |

As shown in Table 2, in the evaluations at a voltage of 4.3 V, the coin-type battery of Example 1-1 containing both the polymer P1 and VC exhibited a high residual capacity retention ratio of 96% even after trickle charging in the same manner as in the evaluations at a voltage of 4.2 V. In contrast, it is seen that the residual capacity retention ratio was reduced in the coin-type batteries of Comparative Examples 1-1 to 1-3 which did not contain either or both of the polymer P1 and VC.

Further, the coin-type battery of Example 1-1 containing both the polymer P1 and VC also exhibited a high capacity retention ratio at 1 C of 88% after trickle charging. In contrast, it is seen that the capacity retention ratio at 1 C after trickle charging was reduced in the coin-type batteries of Comparative Examples 1-1 to 1-3 which did not contain either or both of the polymer P1 and VC.

From the above, it was found that the capacity retention ratio after trickle charging can be increased and the battery service life can be improved by using the polymer P1 and VC in combination.

**[Table 3]**

| | Coin-type battery | | Evaluation results at voltage of 4.3 V | | |
|---|---|---|---|---|---|
| | Polymer P1 | VC | Direct-current resistance [Ω] | | Rate of change in direct-current resistance (%) |
| | | | before trickle charging | after trickle charging | |
| Example 1-1 | present | present | 17 | 23 | 36 |
| Comparative Example 1-1 | absent | present | 14 | 24 | 71 |
| Comparative Example 1-3 | absent | absent | 15 | 22 | 47 |

As shown in Table 3, as compared to the coin-type battery of Comparative Example 1-3 containing neither the polymer P1 nor VC, the coin-type battery of Comparative Example 1-1 containing VC and not containing the polymer P1 exhibited a higher rate of change in the direct-current resistance of 71%. Thus, it was found that the addition of VC alone resulted in an increase in the direct-current resistance.

In contrast to the coin-type battery of Comparative Example 1-1, the rate of change in the direct-current resistance was reduced to 36% in the coin-type battery of Example 1-1 containing both the polymer P1 and VC.

From the above, the use of a combination of the polymer P1 and VC was confirmed to have an unexpected effect of suppressing the direct-current resistance after trickle charging.

### [Example 1-2]

A coin-type battery was produced in the same manner as in Example 1-1, except that vinylethylene carbonate (VEC) was used in place of VC in the preparation of the non-aqueous electrolytic solution.

VEC is one example of the carbonate compound having a carbon-carbon unsaturated bond that is used as the additive (X).

### [Comparative Example 1-4]

A coin-type battery was produced in the same manner as in Example 1-2, except that the polymer P1 solution was not used in the preparation of the positive electrode.

### [Example 1-3]

A coin-type battery was produced in the same manner as in Example 1-1, except that 4-fluoroethylene carbonate (FEC) was used in place of VC in the preparation of the non-aqueous electrolytic solution.

FEC is one example of the carbonate compound having a halogen atom and not having a carbon-carbon unsaturated bond that is used as the additive (X).

### [Comparative Example 1-5]

A coin-type battery was produced in the same manner as in Example 1-3, except that the polymer P1 solution was not used in the preparation of the positive electrode.

For each of the coin-type batteries of Example 1-2, Comparative Example 1-4, Example 1-3 and Comparative Example 1-5, the evaluations relating to the direct-current resistance under "Evaluations at Voltage of 4.3 V" were performed in the same manner as in Example 1-1. The results thereof are shown in Tables 4 and 5.

**[Table 4]**

| | Coin-type battery | | Evaluation results at voltage of 4.3 V | | |
|---|---|---|---|---|---|
| | Polymer P1 | VEC | Direct-current resistance [Ω] | | Rate of change in direct-current resistance (%) |
| | | | before trickle charging | after trickle charging | |
| Example 1-2 | present | present | 17 | 23 | 36 |
| Comparative Example 1-4 | absent | present | 18 | 26 | 44 |
| Comparative Example 1-3 | absent | absent | 15 | 22 | 47 |

**[Table 5]**

| | Coin-type battery | | Evaluation results at voltage of 4.3 V | | |
|---|---|---|---|---|---|
| | Polymer P1 | FEC | Direct-current resistance [Ω] | | Rate of change in direct-current resistance (%) |
| | | | before trickle charging | after trickle charging | |
| Example 1-3 | present | present | 15 | 21 | 40 |
| Comparative Example 1-5 | absent | present | 16 | 24 | 50 |
| Comparative Example 1-3 | absent | absent | 15 | 22 | 47 |

As shown in Table 4, the combination of the polymer P1 and VEC was confirmed to have an effect of suppressing the direct-current resistance after trickle charging.

As shown in Table 5, the combination of the polymer P1 and FEC was also confirmed to have an effect of suppressing the direct-current resistance after trickle charging.

### [Measurement of Ratio (R1/R0)]

For each of the coin-type batteries produced in Example 1-1 and Comparative Example 1-1, the temperature was increased from 30°C to 165°C at a rate of 4°C/min and, in the process thereof, the battery resistance was continuously measured at 1 kHz. In this period, the battery resistance at 30°C (R0) and the battery resistance at 150°C (R1) were read out, and the ratio thereof (R1/R0) was determined.

The results thereof are shown in Table 6.

**[Table 6]**

| | Coin-type battery | | Evaluation results at voltage of 4.3 V | | |
|---|---|---|---|---|---|
| | Polymer P1 | VC | Direct-current resistance [Ω] | | Ratio (R1/R0) |
| | | | R0 (30°C) | R1 (150°C) | |
| Example 1-1 | present | present | 0.453 | 2.656 | 5.9 |
| Comparative Example 1-1 | absent | present | 0.336 | 1.251 | 3.7 |

As shown in Table 6, the coin-type battery of Example 1-1 containing the polymer P1 and VC satisfied the condition of having a ratio (R1/R0) of 3.8 or higher.

### [Example 2-1]

A coin-type battery was produced in the same manner as in Example 1-1, except that VC (added amount = 2 wt%) used as the additive (X) in the preparation of the non-aqueous electrolytic solution was changed to lithium difluorobis(oxalato)phosphate (hereinafter, referred to as "LiFOP") (added amount = 0.5 wt%).

The thus obtained coin-type battery was evaluated in the same manner as in "Evaluations at Voltage of 4.3 V" performed in Example 1-1.

The results thereof are shown in Tables 7 and 8.

It is noted here that LiFOP is one example of the alkali metal salt used as the additive (X).

### [Example 2-2]

A coin-type battery was produced in the same manner as in Example 2-1, except that LiFOP (added amount = 0.5 wt%) used as the additive (X) was changed to lithium bis(oxalato)borate (hereinafter, referred to as "LiBOB") (added amount = 0.5 wt%).

The thus obtained coin-type battery was evaluated in the same manner as in the evaluations relating to the direct-current resistance performed under "Evaluations at Voltage of 4.3 V" in Example 2-1.

The results thereof are shown in Table 9.

It is noted here that LiBOB is one example of the alkali metal salt used as the additive (X).

### [Comparative Example 2-1]

The same operations as in Example 2-1 were performed, except that the polymer P1 solution was not used in the preparation of the positive electrode. The results thereof are shown in Tables 7 and 8.

### [Comparative Example 2-2]

The same operations as in Example 2-1 were performed, except that the polymer P1 solution was not used in the preparation of the positive electrode and LiFOP was not used in the preparation of the non-aqueous electrolytic solution. The results thereof are shown in Tables 7 and 8.

### [Comparative Example 2-3]

The same operations as in Example 2-1 were performed, except that LiFOP was not used in the preparation of the non-aqueous electrolytic solution. The results thereof are shown in Tables 7 and 8.

### [Comparative Example 2-4]

The same operations as in Example 2-2 were performed, except that the polymer P1 solution was not used in the preparation of the positive electrode. The results thereof are shown in Table 9.

**[Table 7]**

| | Coin-type battery | | Evaluation results at voltage of 4.3 V | | | | | |
|---|---|---|---|---|---|---|---|---|
| | Polymer P1 | LiFOP | Capacity retention ratio (%) | | | | | |
| | | | before trickle charging | | | after trickle charging | | |
| | | | 0.2 C | 1 C | 2 C | residual | 1 C | 2 C |
| Example 2-1 | present | present | 100 | 93 | 78 | 94 | 82 | 57 |
| Comparative Example 2-1 | absent | present | 100 | 94 | 85 | 92 | 69 | 37 |
| Comparative Example 2-2 | absent | absent | 100 | 93 | 82 | 90 | 64 | 32 |
| Comparative Example 2-3 | present | absent | 100 | 93 | 76 | 92 | 75 | 45 |

As shown in Table 7, the coin-type battery of Example 2-1 containing both the polymer P1 and LiFOP exhibited high capacity retention ratios after trickle charging (residual, 1 C, and 2 C).

In contrast, the coin-type battery of Comparative Example 2-1 containing LiFOP and not containing the polymer P1, the coin-type battery of Comparative Example 2-3 containing the polymer P1 and not containing LiFOP, and the coin-type battery of Comparative Example 2-2 containing neither the polymer P1 nor LiFOP all exhibited lower capacity retention ratios after trickle charging than the coin-type battery of Example 2-1.

From the above, it was found that an effect of improving the capacity retention ratio after trickle charging can be obtained by using the polymer P1 and LiFOP in combination.

**[Table 8]**

| | Coin-type battery | | Evaluation results at voltage of 4.3 V | | |
|---|---|---|---|---|---|
| | Polymer P1 | LiFOP | Direct-current resistance [Ω] | | Rate of change in direct-current resistance (%) |
| | | | before trickle charging | after trickle charging | |
| Example 2-1 | present | present | 15.0 | 18.0 | 20.0 |
| Comparative Example 2-1 | absent | present | 14.7 | 19.9 | 35.4 |
| Comparative Example 2-2 | absent | absent | 14.8 | 22.3 | 50.7 |
| Comparative Example 2-3 | present | absent | 16.6 | 22.2 | 33.7 |

As shown in Table 8, the coin-type battery of Example 2-1 containing both the polymer P1 and LiFOP exhibited a low rate of change in the direct-current resistance after trickle charging.

In contrast, the coin-type battery of Comparative Example 2-1 containing LiFOP and not containing the polymer P1, the coin-type battery of Comparative Example 2-3 containing the polymer P1 and not containing LiFOP, and the coin-type battery of Comparative Example 2-2 containing neither the polymer P1 nor LiFOP all exhibited a higher rate of change in the direct-current resistance after trickle charging than the coin-type battery of Example 2-1.

From the above, it was found that an effect of reducing the rate of change in the direct-current resistance after trickle charging, namely an effect of suppressing a change in the battery resistance caused by trickle charging, can be obtained by using the polymer P1 and LiFOP in combination.

**[Table 9]**

| | Coin-type battery | | Evaluation results at voltage of 4.3 V | | |
|---|---|---|---|---|---|
| | Polymer P1 | LiBOB | Direct-current resistance [Ω] | | Rate of change in direct-current resistance (%) |
| | | | before trickle charging | after trickle charging | |
| Example 2-2 | present | present | 16.4 | 19.6 | 19.5 |
| Comparative Example 2-4 | absent | present | 15.0 | 21.2 | 41.3 |
| Comparative Example 2-2 | absent | absent | 14.8 | 22.3 | 50.7 |
| Comparative Example 2-3 | present | absent | 16.6 | 22.2 | 33.7 |

As shown in Table 9, the coin-type battery of Example 2-2 containing both the polymer P1 and LiBOB exhibited a low rate of change in the direct-current resistance after trickle charging.

In contrast, the coin-type battery of Comparative Example 2-4 containing LiBOB and not containing the polymer P1, the coin-type battery of Comparative Example 2-3 containing the polymer P1 and not containing LiBOB, and the coin-type battery of Comparative Example 2-2 containing neither the polymer P1 nor LiBOB all exhibited a higher rate of change in the direct-current resistance after trickle charging than the coin-type battery of Example 2-2.

From the above, it was found that an effect of reducing the rate of change in the direct-current resistance after trickle charging, namely an effect of suppressing a change in the battery resistance caused by trickle charging, can be obtained by using the polymer P1 and LiBOB in combination.

### [Example 2-3]

A coin-type battery was produced in the same manner as in Example 2-1, except that LiFOP (added amount = 0.5 wt%) used as the additive (X) was changed to lithium difluorophosphate (hereinafter, referred to as "LiDFP") (added amount = 0.5 wt%).

The thus obtained coin-type battery was evaluated in the same manner as in the evaluations relating to the direct-current resistance performed under "Evaluations at Voltage of 4.3 V" in Example 2-1.

The results thereof are shown in Table 10.

### [Comparative Example 2-5]

The same operations as in Example 2-3 were performed, except that the polymer P1 solution was not used in the preparation of the positive electrode.

The results thereof are shown in Table 10.

**[Table 10]**

| | Coin-type battery | | Evaluation results at voltage of 4.3 V | | |
|---|---|---|---|---|---|
| | Polymer P1 | LiDFP | Direct-current resistance [Ω] | | Rate of change in direct-current resistance (%) |
| | | | before trickle charging | after trickle charging | |
| Example 2-3 | present | present | 16.0 | 16.0 | 0.0 |
| Comparative Example 2-5 | absent | present | 14.0 | 18.0 | 29.0 |
| Comparative Example 2-2 | absent | absent | 14.8 | 22.3 | 50.7 |
| Comparative Example 2-3 | present | absent | 16.6 | 22.2 | 33.7 |

As shown in Table 10, the combination of the polymer P1 and LiDFP was confirmed to have an effect of suppressing a change in the battery resistance caused by trickle charging.

### [Example 2-4]

A coin-type battery was produced in the same manner as in Example 1-1, except that LiFOP (added amount = 0.5 wt%) used as the additive (X) was changed to lithium tetrafluoroborate (hereinafter, referred to as "LiBF₄") (added amount = 0.5 wt%).

The thus obtained coin-type battery was evaluated in the same manner as in "Evaluations at Voltage of 4.3 V" performed in Example 1-1.

The results thereof are shown in Tables 11 and 12.

### [Comparative Example 2-6]

The same operations as in Example 2-4 were performed, except that the polymer P1 solution was not used in the preparation of the positive electrode. The results thereof are shown in Tables 11 and 12.

**[Table 11]**

| | Coin-type battery | | Evaluation results at voltage of 4.3 V | | | | | |
|---|---|---|---|---|---|---|---|---|
| | Polymer P1 | LiBF₄ | Capacity retention ratio (%) | | | | | |
| | | | before trickle charging | | | after trickle charging | | |
| | | | 0.2 C | 1 C | 2 C | residual | 1 C | 2 C |
| Example 2-4 | present | present | 100 | 93 | 87 | 89 | 87 | 74 |
| Comparative Example 2-6 | absent | present | 100 | 93 | 87 | 89 | 83 | 64 |
| Comparative Example 2-2 | absent | absent | 100 | 93 | 82 | 90 | 64 | 32 |
| Comparative Example 2-3 | present | absent | 100 | 93 | 76 | 92 | 75 | 45 |

**[Table 12]**

| | Coin-type battery | | Evaluation results at voltage of 4.3 V | | |
|---|---|---|---|---|---|
| | Polymer P1 | LiBF₄ | Direct-current resistance [Ω] | | Rate of change in direct-current resistance (%) |
| | | | before trickle charging | after trickle charging | |
| Example 2-4 | present | present | 15.0 | 18.0 | 20.0 |
| Comparative Example 2-6 | absent | present | 16.0 | 20.0 | 25.0 |
| Comparative Example 2-2 | absent | absent | 14.8 | 22.3 | 50.7 |
| Comparative Example 2-3 | present | absent | 16.6 | 22.2 | 33.7 |

As shown in Tables 11 and 12, the combination of the polymer P1 and LiBF₄ was confirmed to have not only an effect of improving the capacity retention ratio after trickle charging but also an effect of suppressing a change in the battery resistance caused by trickle charging.

### [Example 3-1]

A coin-type battery was produced in the same manner as in Example 1-1, except that VC (added amount = 2 wt%) used as the additive (X) in the preparation of the non-aqueous electrolytic solution was changed to 1,3-propanesultone (hereinafter, referred to as "PS") (added amount = 0.5 wt%).

The thus obtained coin-type battery was evaluated in the same manner as in "Evaluations at Voltage of 4.3 V" performed in Example 1-1.

The results thereof are shown in Tables 13 and 14.

It is noted here that PS is one example of the sulfonic acid ester used as the additive (X).

### [Comparative Example 3-1]

The same operations as in Example 3-1 were performed, except that the polymer P1 solution was not used in the preparation of the positive electrode. The results thereof are shown in Tables 13 and 14.

### [Comparative Example 3-2]

The same operations as in Example 3-1 were performed, except that the polymer P1 solution was not used in the preparation of the positive electrode and PS was not used in the preparation of the non-aqueous electrolytic solution. The results thereof are shown in Tables 13 and 14.

### [Comparative Example 3-3]

The same operations as in Example 3-1 were performed, except that PS was not used in the preparation of the non-aqueous electrolytic solution. The results thereof are shown in Tables 13 and 14.

**[Table 13]**

| | Coin-type battery | | Evaluation results at voltage of 4.3 V | | | | | |
|---|---|---|---|---|---|---|---|---|
| | Polymer P1 | PS | Capacity retention ratio (%) | | | | | |
| | | | before trickle charging | | | after trickle charging | | |
| | | | 0.2 C | 1 C | 2 C | residual | 1 C | 2 C |
| Example 3-1 | present | present | 100 | 93 | 76 | 93 | 77 | 48 |
| Comparative Example 3-1 | absent | present | 100 | 94 | 85 | 91 | 61 | 27 |
| Comparative Example 3-2 | absent | absent | 100 | 93 | 82 | 90 | 64 | 32 |
| Comparative Example 3-3 | present | absent | 100 | 93 | 76 | 92 | 75 | 45 |

As shown in Table 13, the coin-type battery of Example 3-1 containing both the polymer P1 and PS exhibited high capacity retention ratios after trickle charging (residual, 1 C, and 2 C).

In contrast, the coin-type battery of Comparative Example 3-1 containing PS and not containing the polymer P1, the coin-type battery of Comparative Example 3-3 containing the polymer P1 and not containing PS, and the coin-type battery of Comparative Example 3-2 containing neither the polymer P1 nor PS all exhibited lower capacity retention ratios after trickle charging than the coin-type battery of Example 3-1.

From the above, it was found that an effect of improving the capacity retention ratio after trickle charging can be obtained by using the polymer P1 and PS in combination.

**[Table 14]**

| | Coin-type battery | | Evaluation results at voltage of 4.3 V | | |
|---|---|---|---|---|---|
| | Polymer P1 | PS | Direct-current resistance [Ω] | | Rate of change in direct-current resistance (%) |
| | | | before trickle charging | after trickle charging | |
| Example 3-1 | present | present | 16.2 | 20.5 | 26.5 |
| Comparative Example 3-1 | absent | present | 15.5 | 20.7 | 33.5 |
| Comparative Example 3-2 | absent | absent | 14.8 | 22.3 | 50.7 |
| Comparative Example 3-3 | present | absent | 16.6 | 22.2 | 33.7 |

As shown in Table 14, the coin-type battery of Example 3-1 containing both the polymer P1 and PS exhibited a low rate of change in the direct-current resistance after trickle charging.

In contrast, the coin-type battery of Comparative Example 3-1 containing PS and not containing the polymer P1, the coin-type battery of Comparative Example 3-3 containing the polymer P1 and not containing PS, and the coin-type battery of Comparative Example 3-2 containing neither the polymer P1 nor PS all exhibited a higher rate of change in the direct-current resistance after trickle charging than the coin-type battery of Example 3-1.

From the above, it was found that an effect of reducing the rate of change in the direct-current resistance after trickle charging, namely an effect of suppressing a change in the battery resistance caused by trickle charging, can be obtained by using the polymer P1 and PS in combination.

### [Example 3-2]

A coin-type battery was produced in the same manner as in Example 1-1, except that VC (added amount = 2 wt%) used as the additive (X) in the preparation of the non-aqueous electrolytic solution was changed to methylene methanedisulfonate (hereinafter, referred to as "MMDS") (added amount = 0.5 wt%).

The thus obtained coin-type battery was evaluated in the same manner as in the evaluations relating to the direct-current resistance performed under "Evaluations at Voltage of 4.3 V" in Example 1-1.

The results thereof are shown in Table 15.

It is noted here that MMDS is one example of the sulfonic acid ester used as the additive (X).

### [Comparative Example 3-4]

The same operations as in Example 3-2 were performed, except that the polymer P1 solution was not used in the preparation of the positive electrode. The results thereof are shown in Table 15.

**[Table 15]**

| | Coin-type battery | | Evaluation results at voltage of 4.3 V | | |
|---|---|---|---|---|---|
| | Polymer P1 | MMDS | Direct-current resistance [Ω] | | Rate of change in direct-current resistance (%) |
| | | | before trickle charging | after trickle charging | |
| Example 3-2 | present | present | 16.0 | 16.0 | 0.0 |
| Comparative Example 3-4 | absent | present | 15.0 | 15.0 | 0.0 |
| Comparative Example 3-2 | absent | absent | 14.8 | 22.3 | 50.7 |
| Comparative Example 3-3 | present | absent | 16.6 | 22.2 | 33.7 |

As shown in Table 15, the combination of the polymer P1 and MMDS was confirmed to have an effect of suppressing a change in the battery resistance caused by trickle charging.

### [Example 3-3]

A coin-type battery was produced in the same manner as in Example 1-1, except that VC (added amount = 2 wt%) used as the additive (X) in the preparation of the non-aqueous electrolytic solution was changed to 1,3-propenesultone (hereinafter, referred to as "PRS") (added amount = 0.5 wt%).

The thus obtained coin-type battery was evaluated in the same manner as in "Evaluations at Voltage of 4.3 V" performed in Example 1-1.

The results thereof are shown in Tables 16 and 17.

It is noted here that PRS is one example of the sulfonic acid ester used as the additive (X).

### [Comparative Example 3-5]

The same operations as in Example 3-3 were performed, except that the polymer P1 solution was not used in the preparation of the positive electrode. The results thereof are shown in Tables 16 and 17.

**[Table 16]**

| | Coin-type battery | | Evaluation results at voltage of 4.3 V | | | | | |
|---|---|---|---|---|---|---|---|---|
| | Polymer P1 | PRS | Capacity retention ratio (%) | | | | | |
| | | | before trickle charging | | | after trickle charging | | |
| | | | 0.2 C | 1 C | 2 C | residual | 1 C | 2 C |
| Example 3-3 | present | present | 100 | 93 | 86 | 95 | 91 | 79 |
| Comparative Example 3-5 | absent | present | 100 | 94 | 85 | 94 | 90 | 74 |
| Comparative Example 3-2 | absent | absent | 100 | 93 | 82 | 90 | 64 | 32 |
| Comparative Example 3-3 | present | absent | 100 | 93 | 76 | 92 | 75 | 45 |

**[Table 17]**

| | Coin-type battery | | Evaluation results at voltage of 4.3 V | | |
|---|---|---|---|---|---|
| | Polymer P1 | PRS | Direct-current resistance [Ω] | | Rate of change in direct-current resistance (%) |
| | | | before trickle charging | after trickle charging | |
| Example 3-3 | present | present | 18.0 | 18.0 | 0.0 |
| Comparative Example 3-5 | absent | present | 18.0 | 18.0 | 0.0 |
| Comparative Example 3-2 | absent | absent | 14.8 | 22.3 | 50.7 |
| Comparative Example 3-3 | present | absent | 16.6 | 22.2 | 33.7 |

As shown in Table 16, the combination of the polymer P1 and PRS was confirmed to have an effect of improving the capacity retention ratio after trickle charging.

As shown in Table 17, the combination of the polymer P1 and PRS was also confirmed to have an effect of suppressing a change in the battery resistance caused by trickle charging.

### [Example 3-4]

A coin-type battery was produced in the same manner as in Example 1-1, except that VC (added amount = 2 wt%) used as the additive (X) in the preparation of the non-aqueous electrolytic solution was changed to 4,4'-bis(2-oxo-1,3,2-dioxathiolane) (hereinafter, referred to as "HT-7986") (added amount = 0.5 wt%).

The thus obtained coin-type battery was evaluated in the same manner as in "Evaluations at Voltage of 4.3 V" performed in Example 1-1.

The results thereof are shown in Tables 18 and 19.

It is noted here that HT-7986 is one example of the sulfonic acid ester used as the additive (X).

### [Comparative Example 3-6]

The same operations as in Example 3-4 were performed, except that the polymer P1 solution was not used in the preparation of the positive electrode. The results thereof are shown in Tables 18 and 19.

**[Table 18]**

| | Coin-type battery | | Evaluation results at voltage of 4.3 V | | | | | |
|---|---|---|---|---|---|---|---|---|
| | Polymer P1 | HT-7986 | Capacity retention ratio (%) | | | | | |
| | | | before trickle charging | | | after trickle charging | | |
| | | | 0.2 C | 1 C | 2 C | residual | 1 C | 2 C |
| Example 3-4 | present | present | 100 | 93 | 87 | 95 | 69 | 44 |
| Comparative Example 3-6 | absent | present | 100 | 93 | 86 | 93 | 63 | 22 |
| Comparative Example 3-2 | absent | absent | 100 | 93 | 82 | 90 | 64 | 32 |
| Comparative Example 3-3 | present | absent | 100 | 93 | 76 | 92 | 75 | 45 |

**[Table 19]**

| | Coin-type battery | | Evaluation results at voltage of 4.3 V | | |
|---|---|---|---|---|---|
| | Polymer P1 | HT-7986 | Direct-current resistance [Ω] | | Rate of change in direct-current resistance (%) |
| | | | before trickle charging | after trickle charging | |
| Example 3-4 | present | present | 16.0 | 18.0 | 12.5 |
| Comparative Example 3-6 | absent | present | 16.0 | 22.0 | 37.5 |
| Comparative Example 3-2 | absent | absent | 14.8 | 22.3 | 50.7 |
| Comparative Example 3-3 | present | absent | 16.6 | 22.2 | 33.7 |

As shown in Table 18, the combination of the polymer P1 and HT-7986 was confirmed to have an effect of improving the capacity retention ratio after trickle charging.

Further, as shown in Table 19, the combination of the polymer P1 and HT-7986 was confirmed to have an effect of suppressing a change in the battery resistance caused by trickle charging.

### [Example 3-5]

A coin-type battery was produced in the same manner as in Example 1-1, except that VC (added amount = 2 wt%) used as the additive (X) in the preparation of the non-aqueous electrolytic solution was changed to 4-propyl-1,3,2-dioxathiolane-2,2-dioxide (hereinafter, referred to as "PEGLST") (added amount = 0.5 wt%).

The thus obtained coin-type battery was evaluated in the same manner as in "Evaluations at Voltage of 4.3 V" performed in Example 1-1.

The results thereof are shown in Tables 20 and 21.

It is noted here that PEGLST is one example of the sulfonic acid ester used as the additive (X).

### [Comparative Example 3-7]

The same operations as in Example 3-5 were performed, except that the polymer P1 solution was not used in the preparation of the positive electrode. The results thereof are shown in Tables 20 and 21.

**[Table 20]**

| | Coin-type battery | | Evaluation results at voltage of 4.3 V | | | | | |
|---|---|---|---|---|---|---|---|---|
| | Polymer P1 | PEGLST | Capacity retention ratio (%) | | | | | |
| | | | before trickle charging | | | after trickle charging | | |
| | | | 0.2 C | 1 C | 2 C | residual | 1 C | 2 C |
| Example 3-5 | present | present | 100 | 93 | 86 | 93 | 64 | 35 |
| Comparative Example 3-7 | absent | present | 100 | 93 | 87 | 89 | 31 | 9 |
| Comparative Example 3-2 | absent | absent | 100 | 93 | 82 | 90 | 64 | 32 |
| Comparative Example 3-3 | present | absent | 100 | 93 | 76 | 92 | 75 | 45 |

**[Table 21]**

| | Coin-type battery | | Evaluation results at voltage of 4.3 V | | |
|---|---|---|---|---|---|
| | Polymer P1 | PEGLST | Direct-current resistance [Ω] | | Rate of change in direct-current resistance (%) |
| | | | before trickle charging | after trickle charging | |
| Example 3-5 | present | present | 17.0 | 21.0 | 23.5 |
| Comparative Example 3-7 | absent | present | 17.0 | 25.0 | 47.1 |
| Comparative Example 3-2 | absent | absent | 14.8 | 22.3 | 50.7 |
| Comparative Example 3-3 | present | absent | 16.6 | 22.2 | 33.7 |

As shown in Table 20, the combination of the polymer P1 and PEGLST was confirmed to have an effect of improving the capacity retention ratio after trickle charging.

As shown in Table 21, the combination of the polymer P1 and PEGLST was also confirmed to have an effect of suppressing a change in the battery resistance caused by trickle charging.

### [Example 4-1]

A coin-type battery was produced in the same manner as in Example 1-1, except that VC (added amount = 2 wt%) used as the additive (X) in the preparation of the non-aqueous electrolytic solution was changed to adiponitrile (hereinafter, referred to as "ADPN") (added amount = 0.5 wt%).

The thus obtained coin-type battery was evaluated in the same manner as in "Evaluations at Voltage of 4.3 V" performed in Example 1-1.

The results thereof are shown in Tables 22 and 23.

It is noted here that ADPN is one example of the nitrile compound used as the additive (X).

### [Comparative Example 4-1]

The same operations as in Example 4-1 were performed, except that the polymer P1 solution was not used in the preparation of the positive electrode. The results thereof are shown in Tables 22 and 23.

### [Comparative Example 4-2]

The same operations as in Example 4-1 were performed, except that the polymer P1 solution was not used in the preparation of the positive electrode and ADPN was not used in the preparation of the non-aqueous electrolytic solution. The results thereof are shown in Tables 22 and 23.

### [Comparative Example 4-3]

The same operations as in Example 4-1 were performed, except that ADPN was not used in the preparation of the non-aqueous electrolytic solution. The results thereof are shown in Tables 22 and 23.

**[Table 22]**

| | Coin-type battery | | Evaluation results at voltage of 4.3 V | | | | | |
|---|---|---|---|---|---|---|---|---|
| | Polymer P1 | ADPN | Capacity retention ratio (%) | | | | | |
| | | | before trickle charging | | | after trickle charging | | |
| | | | 0.2 C | 1 C | 2 C | residual | 1 C | 2 C |
| Example 4-1 | present | present | 100 | 93 | 77 | 93 | 88 | 60 |
| Comparative Example 4-1 | absent | present | 100 | 94 | 81 | 92 | 70 | 38 |
| Comparative Example 4-2 | absent | absent | 100 | 93 | 82 | 90 | 64 | 32 |
| Comparative Example 4-3 | present | absent | 100 | 93 | 76 | 92 | 75 | 45 |

**[Table 23]**

| | Coin-type battery | | Evaluation results at voltage of 4.3 V | | |
|---|---|---|---|---|---|
| | Polymer P1 | ADPN | Direct-current resistance [Ω] | | Rate of change in direct-current resistance (%) |
| | | | before trickle charging | after trickle charging | |
| Example 4-1 | present | present | 16.0 | 20.0 | 25.0 |
| Comparative Example 4-1 | absent | present | 17.0 | 27.0 | 58.8 |
| Comparative Example 4-2 | absent | absent | 14.8 | 22.3 | 50.7 |
| Comparative Example 4-3 | present | absent | 16.6 | 22.2 | 33.7 |

As shown in Table 22, the combination of the polymer P1 and ADPN was confirmed to have an effect of improving the capacity retention ratio after trickle charging.

Further, as shown in Table 23, the combination of the polymer P1 and ADPN was confirmed to have an effect of suppressing a change in the battery resistance caused by trickle charging.

### [Example 5-1]

A coin-type battery was produced in the same manner as in Example 1-1, except that VC (added amount = 2 wt%) used as the additive (X) in the preparation of the non-aqueous electrolytic solution was changed to *ortho*-fluorotoluene (hereinafter, referred to as "OFT") (added amount = 0.5 wt%).

The thus obtained coin-type battery was evaluated in the same manner as in the evaluations relating to the direct-current resistance performed under "Evaluations at Voltage of 4.3 V" in Example 1-1.

The results thereof are shown in Table 24.

It is noted here that OFT is one example of the aromatic hydrocarbon compound which is used as the additive (X) and substituted with at least one substituent selected from the group consisting of a halogen atom, an alkyl group, a halogenated alkyl group, an alkoxy group, a halogenated alkoxy group, an aryl group and a halogenated aryl group.

### [Comparative Example 5-1]

The same operations as in Example 5-1 were performed, except that the polymer P1 solution was not used in the preparation of the positive electrode. The results thereof are shown in Table 24.

### [Comparative Example 5-2]

The same operations as in Example 5-1 were performed, except that the polymer P1 solution was not used in the preparation of the positive electrode and OFT was not used in the preparation of the non-aqueous electrolytic solution. The results thereof are shown in Table 24.

### [Comparative Example 5-3]

The same operations as in Example 5-1 were performed, except that OFT was not used in the preparation of the non-aqueous electrolytic solution. The results thereof are shown in Table 24.

**[Table 24]**

| | Coin-type battery | | Evaluation results at voltage of 4.3 V | | |
|---|---|---|---|---|---|
| | Polymer P1 | OFT | Direct-current resistance [Ω] | | Rate of change in direct-current resistance (%) |
| | | | before trickle charging | after trickle charging | |
| Example 5-1 | present | present | 16.0 | 18.0 | 12.5 |
| Comparative Example 5-1 | absent | present | 16.0 | 27.0 | 68.8 |
| Comparative Example 5-2 | absent | absent | 14.8 | 22.3 | 50.7 |
| Comparative Example 5-3 | present | absent | 16.6 | 22.2 | 33.7 |

As shown in Table 24, the combination of the polymer P1 and OFT was confirmed to have an effect of suppressing a change in the battery resistance caused by trickle charging.

### [Example 5-2]

A coin-type battery was produced in the same manner as in Example 1-1, except that VC (added amount = 2 wt%) used as the additive (X) in the preparation of the non-aqueous electrolytic solution was changed to *ortho*-chlorotoluene (hereinafter, referred to as "OCT") (added amount = 0.5 wt%).

The thus obtained coin-type battery was evaluated in the same manner as in "Evaluations at Voltage of 4.3 V" performed in Example 1-1.

The results thereof are shown in Table 25.

It is noted here that OCT is one example of the aromatic hydrocarbon compound which is used as the additive (X) and substituted with at least one substituent selected from the group consisting of a halogen atom, an alkyl group, a halogenated alkyl group, an alkoxy group, a halogenated alkoxy group, an aryl group and a halogenated aryl group.

### [Comparative Example 5-4]

The same operations as in Example 5-1 were performed, except that the polymer P1 solution was not used in the preparation of the positive electrode. The results thereof are shown in Table 25.

**[Table 25]**

| | Coin-type battery | | Evaluation results at voltage of 4.3 V | | |
|---|---|---|---|---|---|
| | Polymer P1 | OCT | Direct-current resistance [Ω] | | Rate of change in direct-current resistance (%) |
| | | | before trickle charging | after trickle charging | |
| Example 5-2 | present | present | 16.0 | 18.0 | 12.5 |
| Comparative Example 5-4 | absent | present | 19.0 | 26.0 | 36.8 |
| Comparative Example 5-2 | absent | absent | 14.8 | 22.3 | 50.7 |
| Comparative Example 5-3 | present | absent | 16.6 | 22.2 | 33.7 |

As shown in Table 25, the combination of the polymer P1 and OCT was confirmed to have an effect of suppressing a change in the battery resistance caused by trickle charging.

### [Example 5-3]

A coin-type battery was produced in the same manner as in Example 1-1, except that VC (added amount = 2 wt%) used as the additive (X) in the preparation of the non-aqueous electrolytic solution was changed to 1,3-dioxane (hereinafter, referred to as "13DOX") (added amount = 0.5 wt%).

The thus obtained coin-type battery was evaluated in the same manner as in "Evaluations at Voltage of 4.3 V" performed in Example 1-1.

The results thereof are shown in Table 26.

It is noted here that 13DOX is one example of the dioxane compound used as the additive (X).

### [Comparative Example 5-5]

The same operations as in Example 5-3 were performed, except that the polymer P1 solution was not used in the preparation of the positive electrode. The results thereof are shown in Table 26.

**[Table 26]**

| | Coin-type battery | | Evaluation results at voltage of 4.3 V | | |
|---|---|---|---|---|---|
| | Polymer P1 | 13DOX | Direct-current resistance [Ω] | | Rate of change in direct-current resistance (%) |
| | | | before trickle charging | after trickle charging | |
| Example 5-3 | present | present | 17.0 | 22.0 | 29.4 |
| Comparative Example 5-5 | absent | present | 17.0 | 31.0 | 82.4 |
| Comparative Example 5-2 | absent | absent | 14.8 | 22.3 | 50.7 |
| Comparative Example 5-3 | present | absent | 16.6 | 22.2 | 33.7 |

As shown in Table 26, the combination of the polymer P1 and 13DOX was confirmed to have an effect of suppressing a change in the battery resistance caused by trickle charging.

The disclosures of Japanese Patent Application No. 2014-215007, Japanese Patent Application No. 2014-234052, Japanese Patent Application No. 2014-234053, Japanese Patent Application No. 2014-234054, and Japanese Patent Application No. 2014-234055 are incorporated herein by reference in their entirety.

All publications, patent applications, and technical standards mentioned in this specification are herein incorporated by reference to the same extent as if each individual publication, patent application, or technical standard was specifically and individually indicated to be incorporated by reference.

## Claims

1. A lithium secondary battery comprising:
a positive electrode which contains a positive electrode active material capable of absorbing and desorbing lithium;
a negative electrode which contains a negative electrode active material capable of absorbing and desorbing lithium; and
a non-aqueous electrolytic solution,
wherein:
at least one of the positive electrode or the negative electrode contains a polymer that is a reaction product of at least one compound (A) and a compound (B), the at least one compound (A) being selected from the group consisting of an amine compound, an amide compound, an imide compound, a maleimide compound and an imine compound, and the compound (B) having two or more carbonyl groups in one molecule and being different from the compound (A), and
the non-aqueous electrolytic solution contains an additive (X), which is at least one compound selected from the group consisting of:
a carbonate compound having a carbon-carbon unsaturated bond,
a carbonate compound having a halogen atom and not having a carbon-carbon unsaturated bond,
an alkali metal salt,
a sulfonic acid ester compound,
a sulfuric acid ester compound,
a nitrile compound,
a dioxane compound, and
an aromatic hydrocarbon compound substituted with at least one substituent selected from the group consisting of a halogen atom, an alkyl group, a halogenated alkyl group, an alkoxy group, a halogenated alkoxy group, an aryl group and a halogenated aryl group.

2. The lithium secondary battery according to claim 1, wherein the polymer is a reaction product of the maleimide compound and the compound (B).

3. The lithium secondary battery according to claim 1 or 2, wherein the compound (B) is at least one compound selected from the group consisting of barbituric acid and derivatives

4. The lithium secondary battery according to any one of claims 1 to 3, wherein the polymer comprises a reactive double bond.

5. The lithium secondary battery according to any one of claims 1 to 4, wherein the maleimide compound is at least one compound selected from the group consisting of compounds each represented by any one of Formulae (1) to (4): 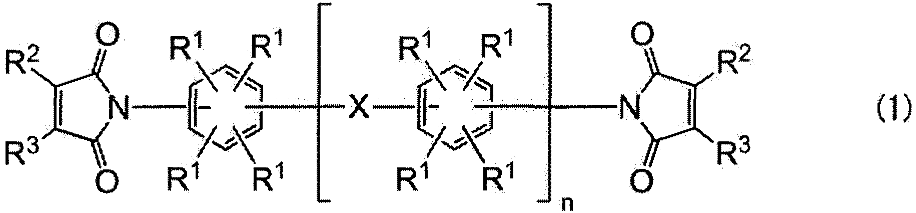 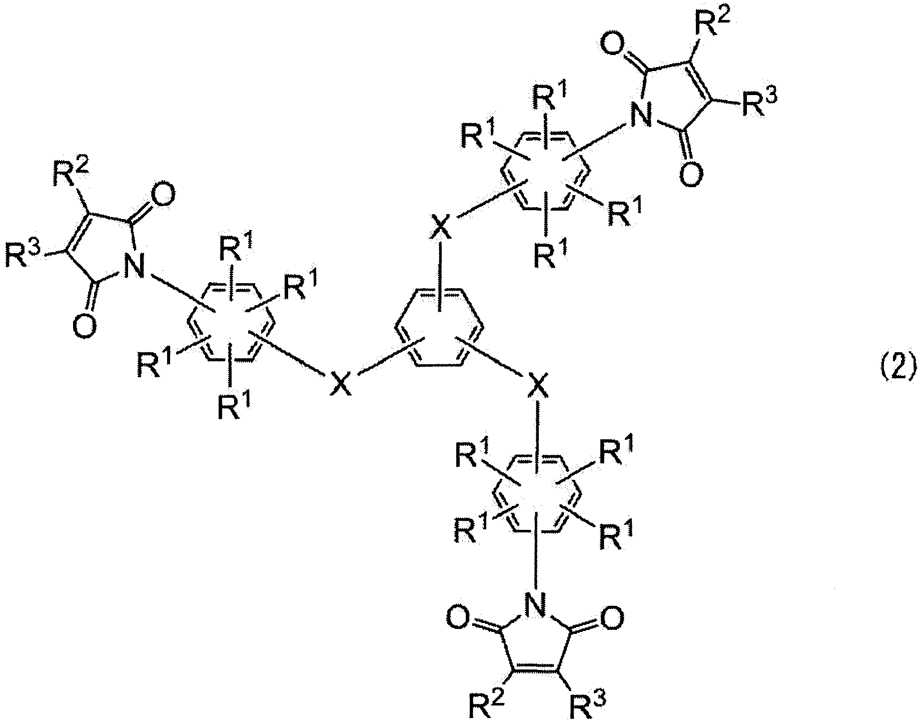 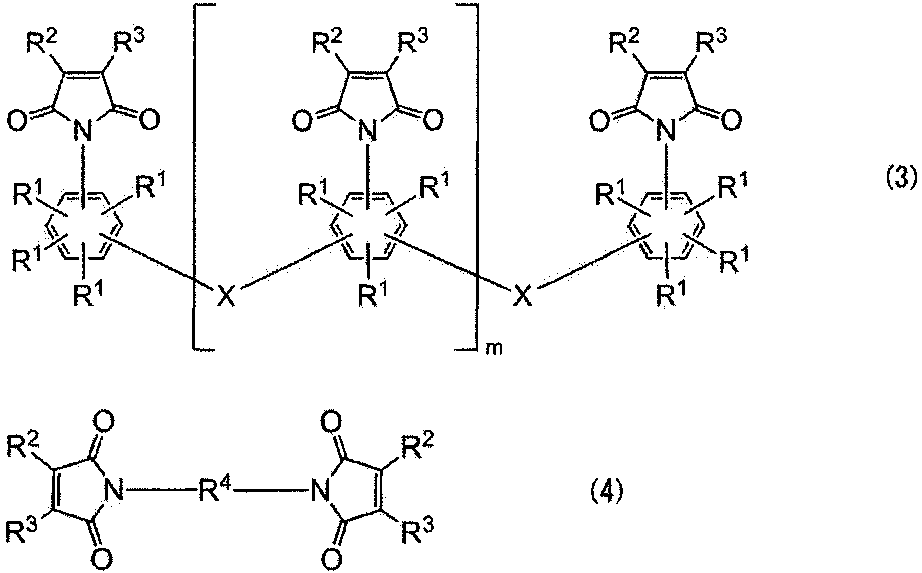 wherein, in Formula (1), n is an integer of 0 or larger;
in Formula (3), m represents a real number from 1 to 1,000;
in Formulae (1) to (3), X represents -O-, -SO₂-, -S-, -CO-, -CH₂-, -C(CH₃)₂-, -C(CF₃)₂-, -CR=CR-, or a single bond, wherein R is a hydrogen atom or an alkyl group, and when there are plural Xs in one molecule, the plural Xs may be the same as, or different from, each other;
in Formulae (1) to (3), R¹ represents a hydrogen atom, a halogen atom or a hydrocarbon group, plural R¹s existing in one molecule may be the same as, or different from, each other, and each of R² and R³ independently represents a hydrogen atom, a halogen atom, or an alkyl group having from 1 to 3 carbon atoms; and
in Formula (4), R⁴ represents an alkylene group having from 1 to 10 carbon atoms which optionally has a side chain, -NR³-, -C(O)CH₂-, -CH₂OCH₂-, -C(O)-, -O-, -O-O-, -S-, -S-S-, -S(O)-, -CH₂S(O)CH₂- or -SO₂-, and each of R² and R³ independently represents a hydrogen atom, a halogen atom, or an alkyl group having from 1 to 3 carbon atoms.

6. The lithium secondary battery according to claim 3, wherein the at least one compound selected from the group consisting of barbituric acid and derivatives thereof is a compound represented by Formula (5): 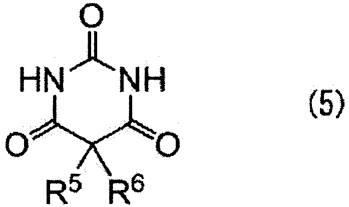 wherein each of R⁵ and R⁶ independently represents a hydrogen atom, a methyl group, an ethyl group, a phenyl group, an isopropyl group, an isobutyl group, an isopentyl group, or a 2-pentyl group.

7. The lithium secondary battery according to any one of claims 1 to 6, wherein at least one of the positive electrode or the negative electrode comprises a composite layer containing the polymer, and a content of the polymer in the composite layer is from 0.01% by mass to 5% by mass.

8. The lithium secondary battery according to any one of claims 1 to 7, wherein the carbonate compound having a carbon-carbon unsaturated bond is at least one selected from the group consisting of chain carbonate compounds each represented by Formula (X1), cyclic carbonate compounds each represented by Formula (X2), cyclic carbonate compounds each represented by Formula (X3) and cyclic carbonate compounds each represented by Formula (X4): 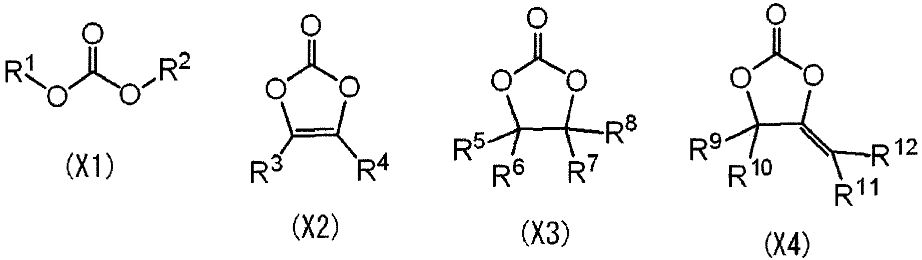 wherein, in Formula (X1), each of R¹ and R² independently represents a group having from 1 to 12 carbon atoms which optionally has a carbon-carbon unsaturated bond, an ether bond or a carbon-halogen bond, and at least one of R¹ or R² has a carbon-carbon unsaturated bond;
in Formula (X2), each of R³ and R⁴ independently represents a hydrogen atom, or a group having from 1 to 12 carbon atoms which optionally has a carbon-carbon unsaturated bond, an ether bond or a carbon-halogen bond;
in Formula (X3), each of R⁵ to R⁸ independently represents a hydrogen atom, or a group having from 1 to 12 carbon atoms which optionally has a carbon-carbon unsaturated bond, an ether bond or a carbon-halogen bond, at least one of R⁵ to R⁸ has a carbon-carbon unsaturated bond, and either R⁵ or R⁶, and either R⁷ or R⁸, are optionally combined to form, in combination with carbon atoms to which they are respectively bonded, a benzene ring structure or a cyclohexyl ring structure; and
in Formula (X4), each of R⁹ to R¹² independently represents a hydrogen atom, or a group having from 1 to 12 carbon atoms which optionally has a carbon-carbon unsaturated bond, an ether bond or a carbon-halogen bond.

9. The lithium secondary battery according to any one of claims 1 to 8, wherein the alkali metal salt is at least one selected from the group consisting of a monofluorophosphate salt, a difluorophosphate salt, an oxalato salt, a sulfonate salt, a carboxylate salt, an imide salt and a methide salt.

10. The lithium secondary battery according to claim 9, wherein the alkali metal salt is at least one selected from the group consisting of a monofluorophosphate salt, a difluorophosphate salt, an oxalato salt and a fluorosulfonate salt.

11. The lithium secondary battery according to any one of claims 1 to 10, wherein the sulfonic acid ester compound is at least one compound selected from the group consisting of chain sulfonic acid ester compounds each represented by Formula (X6), cyclic sulfonic acid ester compounds each represented by Formula (X7), cyclic sulfonic acid ester compounds each represented by Formula (X8) and disulfonic acid ester compounds each represented by Formula (X9): 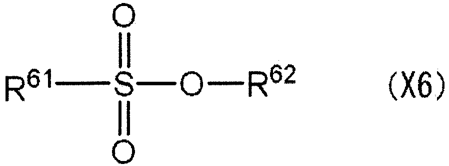 wherein each of R⁶¹ and R⁶² independently represents a linear or branched aliphatic hydrocarbon group having from 1 to 12 carbon atoms, an aryl group having from 6 to 12 carbon atoms, or a heterocyclic group having from 6 to 12 carbon atoms, and each of the groups is optionally substituted with a halogen atom; 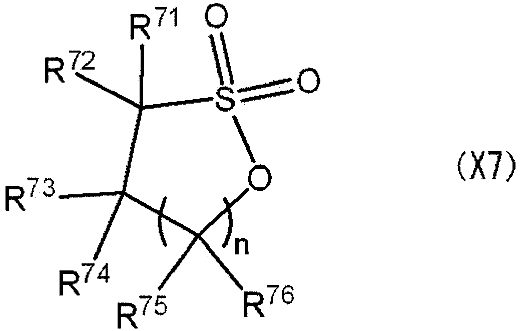 wherein each of R⁷¹ to R⁷⁶ independently represents a hydrogen atom, a halogen atom, or an alkyl group having from 1 to 6 carbon atoms; and n is an integer from 0 to 3; 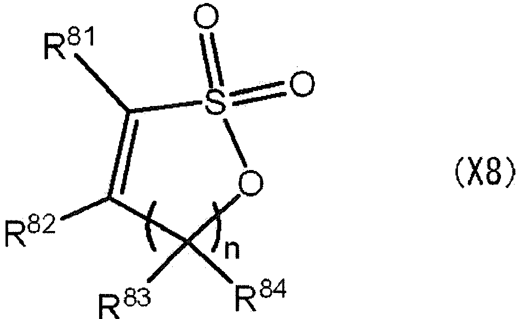 wherein each of R⁸¹ to R⁸⁴ independently represents a hydrogen atom, a halogen atom, or an alkyl group having from 1 to 6 carbon atoms; and n is an integer from 0 to 3; 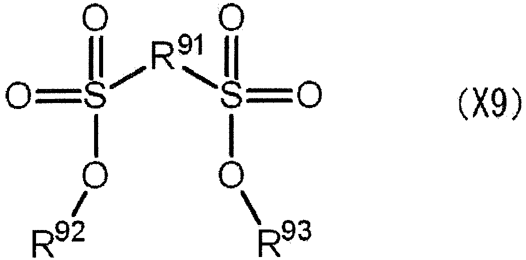
wherein R⁹¹ represents an aliphatic hydrocarbon group having from 1 to 10 carbon atoms, or a halogenated alkylene group having from 1 to 3 carbon atoms; and
R⁹² and R⁹³ each independently represent an alkyl group having from 1 to 6 carbon atoms or an aryl group, or
R⁹² and R⁹³ are combined to represent an alkylene group having from 1 to 10 carbon atoms, or a 1,2-phenylene group which is optionally substituted with a halogen atom, an alkyl group having from 1 to 12 carbon atoms or a cyano group.

12. The lithium secondary battery according to any one of claims 1 to 11, wherein the sulfuric acid ester compound is at least one compound selected from the group consisting of chain sulfuric acid ester compounds each represented by Formula (X10) and cyclic sulfuric acid ester compounds each represented by Formula (X11): 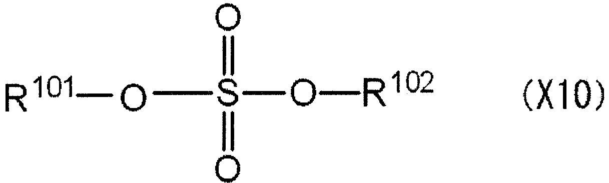 wherein each of R¹⁰ and R¹⁰² independently represents a linear or branched aliphatic hydrocarbon group having from 1 to 12 carbon atoms, an aryl group having from 6 to 12 carbon atoms, or a heterocyclic group having from 6 to 12 carbon atoms, and each of the groups is optionally substituted with a halogen atom; 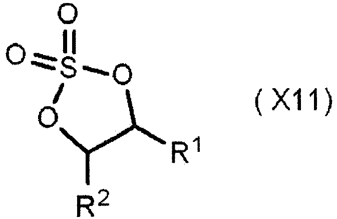 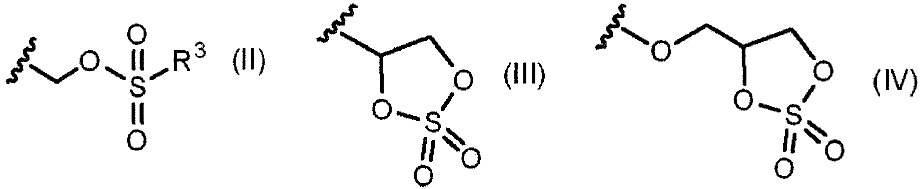 wherein, in Formula (X11), each of R¹ and R² independently represents a hydrogen atom, an alkyl group having from 1 to 6 carbon atoms, a phenyl group, a group represented by Formula (II) or a group represented by Formula (III), or R¹ and R² are combined to represent, in combination with carbon atoms to which R¹ and R² are respectively bonded, a group forming a benzene ring or a cyclohexyl ring;
in Formula (II), R³ represents a halogen atom, an alkyl group having from 1 to 6 carbon atoms, a halogenated alkyl group having from 1 to 6 carbon atoms, an alkoxy group having from 1 to 6 carbon atoms, or a group represented by Formula (IV), and wavy lines in Formulae (II), (III) and (IV) each represent a bonding position; and
when the cyclic sulfuric acid ester compound represented by Formula (X11) contains two groups each represented by Formula (II), the two groups each represented by Formula (II) may be the same as, or different from, each other.

13. The lithium secondary battery according to any one of claims 1 to 12, wherein the nitrile compound is a nitrile compound represented by Formula (X12): 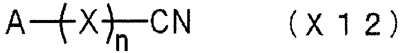 wherein, in Formula (X12):
A represents a hydrogen atom or a nitrile group;
X represents -CH₂-, -CFH-, -CF₂-, -CHR¹¹-, -CFR¹²-, -CR¹³R¹⁴-, -C(=O)-, -O-, -S-, -NH-, or -NR¹⁵-;
each of R¹¹ to R¹⁵ independently represents a nitrile group or a hydrocarbon group having from 1 to 5 carbon atoms, which optionally has a substituent;
n represents an integer greater than or equal to 1; and
when n is an integer greater than or equal to 2, plural Xs may be the same as, or different from, each other.

14. The lithium secondary battery according to any one of claims 1 to 13, wherein the aromatic hydrocarbon compound is an aromatic hydrocarbon compound which is substituted with at least one substituent selected from the group consisting of a fluorine atom, a chlorine atom, an alkyl group having from 1 to 6 carbon atoms, a halogenated alkyl group having from 1 to 6 carbon atoms, an alkoxy group having from 1 to 6 carbon atoms, a halogenated alkoxy group having from 1 to 6 carbon atoms, an aryl group having from 6 to 12 carbon atoms and a halogenated aryl group having from 6 to 12 carbon atoms.

15. The lithium secondary battery according to any one of claims 1 to 14, wherein a ratio of a battery resistance R1 at 150°C with respect to a battery resistance R0 at 30°C (R1/R0) is 3.8 or higher.

16. A lithium secondary battery comprising:
a positive electrode which contains a positive electrode active material capable of absorbing and desorbing lithium;
a negative electrode which contains a negative electrode active material capable of absorbing and desorbing lithium; and
a non-aqueous electrolytic solution,
wherein
a ratio of a battery resistance R1 at 150°C with respect to a battery resistance R0 at 30°C (R1/R0) is 3.8 or higher, and
the non-aqueous electrolytic solution contains an additive (X) which is at least one compound selected from the group consisting of:
a carbonate compound having a carbon-carbon unsaturated bond;
a carbonate compound having a halogen atom and not having a carbon-carbon unsaturated bond;
an alkali metal salt;
a sulfonic acid ester compound;
a sulfuric acid ester compound;
a nitrile compound;
a dioxane compound; and
an aromatic hydrocarbon compound substituted with at least one substituent selected from the group consisting of a halogen atom, an alkyl group, a halogenated alkyl group, an alkoxy group, a halogenated alkoxy group, an aryl group and a halogenated aryl group.

17. A lithium secondary battery obtained by charging and discharging the lithium secondary battery according to any one of claims 1 to 16.
